# EUROPÄISCHE PATENTSCHRIFT

(11) **EP 1 954 831 B1**
(45) Veröffentlichungstag und Bekanntmachung des Hinweises auf die Patenterteilung: **11.07.2012**
(21) Anmeldenummer: 06830160.5
(22) Anmeldetag: 28.11.2006
(51) Int. Cl.: C12Q 1/68

(54) **AHR-MEDIATOREN**
AHR MEDIATOR
MÉDIATEURS D'AHR

(30) Priorität: 28.11.2005 DE 102005056890; 03.05.2006 US 796854 P
(43) Veröffentlichungstag der Anmeldung: 13.08.2008
(73) Patentinhaber: Symrise AG, 37603 Holzminden (DE)
(72) Erfinder: KRUTMANN, Jean, 41844 Wegberg (DE); HERRMANN, Martina, 31789 Hameln (DE); VIELHABER, Gabriele, 37603 Holzminden (DE); LEY, Jakob, 37603 Holzminden (DE); KOCH, Oskar, 37079 Göttingen (DE)
(74) Vertreter: Eisenführ, Speiser & Partner
(86) Internationale Anmeldenummer: PCT/EP2006/069010
(87) Internationale Veröffentlichungsnummer: WO 2007/060256

(56) Entgegenhaltungen:
- EP-A- 1 382 329
- WO-A-2004/091558
- WO-A-2006/007503
- JP-A- 3 241 440
- JP-A- 6 016 531
- US-A- 5 959 738
- KRUTMANN ET AL: "The interaction of UVA and UVB wavebands with particular emphasis on signalling" PROGRESS IN BIOPHYSICS AND MOLECULAR BIOLOGY, PERGAMON PRESS, OXFORD, GB, Bd. 92, Nr. 1, September 2006 (2006-09), Seiten 105-107, XP005493608 ISSN: 0079-6107
- VILLANO ET AL: "2,3,7,8-tetrachlorodibenzo-p-dioxin (TCDD) induces matrix metalloproteinase (MMP) expression and invasion in A2058 melanoma cells" TOXICOLOGY AND APPLIED PHARMACOLOGY, ACADEMIC PRESS, Bd. 210, Nr. 3, 27. Juni 2005 (2005-06-27), Seiten 212-224, XP005252837 ISSN: 0041-008X
- HENLEY D V ET AL: "Aryl hydrocarbon receptor-mediated posttranscriptional regulation of IL-1beta" ARCHIVES OF BIOCHEMISTRY AND BIOPHYSICS, NEW YORK, US, US, Bd. 422, Nr. 1, 1. Februar 2004 (2004-02-01), Seiten 42-51, XP004483518 ISSN: 0003-9861
- PLISKOVA ET AL: "Effects of silymarin flavonolignans and synthetic silybin derivatives on estrogen and aryl hydrocarbon receptor activation" TOXICOLOGY, LIMERICK, IR, Bd. 215, Nr. 1-2, 5. November 2005 (2005-11-05), Seiten 80-89, XP005103760 ISSN: 0300-483X
- ZHANG S ET AL: "FLAVONOIDS AS ARYL HYDROCARBON RECEPTOR AGONISTS/ANTAGONISTS: EFFECTS OF STRUCTURE AND CELL CONTEXT" JOURNAL OF PARENTERAL SCIENCE AND TECHNOLOGY, XX, XX, Bd. 111, Nr. 16, Dezember 2003 (2003-12), Seiten 1877-1882, XP009071035 ISSN: 0279-7976 in der Anmeldung erwähnt
- AMAKURA Y ET AL: "SCREENING OF THE INHIBITORY EFFECT OF VEGETABLE CONSTITUENTS ON THE ARL HYDROCARBON RECEPTOR-MEDIATED ACTIVITY INDUCED BY 2,3,7,8-TETRACHLORODIBENZO-P-DIOXIN" BIOLOGICAL & PHARMACEUTICAL BULLETIN (OF JAPAN), PHARMACEUTICAL SOCIETY OF JAPAN, TOKYO, JP, Bd. 26, Nr. 12, Dezember 2003 (2003-12), Seiten 1754-1760, XP009070851 ISSN: 0918-6158
- SEIDEL S D ET AL: "AH RECEPTOR-BASED CHEMICAL SCREENING BIOASSAYS: APPLICATION AND LIMITATIONS FOR THE DETECTION OF AH RECEPTOR AGONISTS" TOXICOLOGICAL SCIENCES, ACADEMIC PRESS, SAN DIEGO, FL,, US, Bd. 55, Nr. 1, Mai 2000 (2000-05), Seiten 107-115, XP009010645 ISSN: 1096-6080
- HENRY E C ET AL: "Flavone antagonists bind competitively with 2,3,7, 8-tetrachlorodibenzo-p-dioxin (TCDD) to the aryl hydrocarbon receptor but inhibit nuclear uptake and transformation." MOLECULAR PHARMACOLOGY APR 1999, Bd. 55, Nr. 4, April 1999 (1999-04), Seiten 716-725, XP002447278 ISSN: 0026-895X
- ROBLIN S ET AL: "AH receptor antagonist inhibits constitutive CYP1A1 and CYP1B1 expression in rat BP8 cells" BIOCHEMICAL AND BIOPHYSICAL RESEARCH COMMUNICATIONS, ACADEMIC PRESS INC. ORLANDO, FL, US, Bd. 317, Nr. 1, 23. April 2004 (2004-04-23), Seiten 142-148, XP004496941 ISSN: 0006-291X
- SWANSON H I: "Cytochrome P450 expression in human keratinocytes: an aryl hydrocarbon receptor perspective" CHEMICO-BIOLOGICAL INTERACTIONS, ELSEVIER SCIENCE IRLAND, IR, Bd. 149, Nr. 2-3, 15. Oktober 2004 (2004-10-15), Seiten 69-79, XP004610276 ISSN: 0009-2797
- FRITSCHE E ET AL: "Ultraviolet B radiation mediates nuclear translocation of the aryl hydrocarbon receptor" JOURNAL OF INVESTIGATIVE DERMATOLOGY, NEW YORK, NY, US, Bd. 121, Nr. 1, 30. April 2003 (2003-04-30), Seite 1092, XP008082271 ISSN: 0022-202X
- BERGANDER L ET AL: "Metabolic fate of the Ah receptor ligand 6-formylindolo[3,2-b]carbazole" CHEMICO-BIOLOGICAL INTERACTIONS, ELSEVIER SCIENCE IRLAND, IR, Bd. 149, Nr. 2-3, 15. Oktober 2004 (2004-10-15), Seiten 151-164, XP004610284 ISSN: 0009-2797

## Beschreibung

Die Erfindung betrifft Verfahren zum Finden und Beurteilen von Agonisten Antagonisten des Arylkohlenwasserstoffrezeptors (aryl hydrocarbon receptor; Ah-Rezeptor; AhR), die Agonisten und Antagonisten selbst und deren Verwendungen.

Die Haut ist das größte Organ des menschlichen Körpers. Ihre wichtigste Funktion ist der Schutz des Körpers gegen das unkontrollierte Austreten von Wasser einerseits sowie gegen das Eindringen von schädlichen Chemikalien oder Bakterien sowie von Sonnenstrahlung andererseits.

Wird die menschliche Haut einer lang andauernden Sonnenbestrahlung ausgesetzt, kann dies zum Auftreten einer Vielzahl von Schäden führen. Beispielhaft seien hier Sonnenbrand, lichtinduzierte Hautalterung und Hautkrebs genannt. Diese schädigende Wirkung des Sonnenlichts wird u. a. auf die in dem Sonnenlichtspektrum enthaltene UVB-Strahlung (280 - 320 nm) zurückgeführt. Besonders im Hinblick auf die in der letzten Zeit durch die fortschreitende Zerstörung der Ozonschicht stark zunehmende Intensität des UVB-Anteils im Spektrum des Sonnenlichtes ist ein möglichst umfassender Schutz der Haut gegen UVB-Strahlung erforderlich.

Zum Schutz vor UV-Strahlung enthalten übliche Sonnenschutzmittel zur Ausbildung einer Schutzschicht auf der Haut Substanzen, welche die Strahlung im Bereich von 280-400 nm absorbieren und/oder reflektieren (UV-Filter). Solche photoprotektiven Substanzen sind beispielsweise anorganische Oxide wie Zinkoxid oder organische UV-Absorber wie beispielsweise Zimtsäure- oder Dibenzoylmethan-Derivate. Nachteil dieser Verbindungen ist jedoch, dass die durch sie ausgebildete Schutzschicht durch mechanischen Abrieb, Wasser oder Detergenzien leicht zerstört werden kann. Es ist daher wünschenswert, neben den erwähnten UV-Filtern auch auf Substanzen zugreifen zu können, die eine Schutzwirkung innerhalb der Haut entfalten.

Zur Lösung dieser Aufgabe ist die Kenntnis der molekularen Mechanismen, durch die UVB-Strahlung gesundheitsschädliche Wirkungen an der menschlichen Haut hervorrufen kann, von grundlegender Bedeutung. Entsprechende Untersuchungen haben gezeigt, dass biologische Wirkungen der UVB-Strahlung zum einen darauf zurückgeführt werden können, dass durch UVB-Strahlung strukturelle Veränderungen an den DNS-Molekülen ihm Zellkern der Hautzellen hervorgerufen werden. Entsprechend werden DNS-Reparaturenzyme zur Photoprotektion eingesetzt (Stege et al. (2000) PNAS 97:1790).

Zum anderen konnte nachgewiesen werden, dass UVB-Strahlung in der Lage ist, Veränderungen auf Ebene der Zellmembran auszulösen, die zu einer Aktivierung von Wachstumsrezeptoren wie dem Epidermal Growth Factor-Rezeptor (EGF-R) und nachfolgend zur Tumorbildung beitragen (Ashida et al. (2003) Exp Dermatol 12:445; Lirvall et al. (1996) Biosci Rep 16:227). Diese EGF-R Aktivierung kann durch antioxidative Enzyme gehemmt werden (Lirvall et al. (1996) Biosci Rep 16:227).

Auch wird die Expression von Cyclooxygenase-2 und Matrixmetalloproteinasen durch UVB- und UVA-Licht induziert (Pentland et al. (1999) Carcinogenesis 20(10):1939-44). Cyclooxygenasen gehören zu den Schlüsselenzymen der inflammatorischen Reaktion. Sie katalysieren den ersten Schritt der Synthese einer Reihe von Entzündungsmediatoren (Prostaglandine, Prostacycline, Thromboxane) aus Arachidonsäure. Es gibt 2 Formen: Cyclooxygenase-1 (COX-1) ist die konstitutive, dauerhaft expremierte Form, während die Expression von COX-2 nur nach Stimulation durch zelluläre Signale z.B. infolge einer Gewebsschädigung oder -entzündung erfolgt.

Matrixmetalloproteinasen (MMPs) sind Enzyme, die in der Lage sind, die Makromoleküle der extrazellulären Matrix (ECM) proteolytisch abzubauen. MMPs besitzen eine breite, oftmals überlappende Substratspezifität, und in Kombination sind sie fähig, sämtliche Proteinkomponenten der extrazellulären Matrix zu zerlegen. Bisher wurden etwa 20 MMPs identifiziert. In der menschlichen Haut spielen v. a. MMP-1 (Collagenase-1), MMP-2 (Gelatinase A), MMP-9 (Gelatinase B) und MMP-3 eine wichtige Rolle. MMP-1 spaltet neben Kollagen-1 und -3 auch Pro-MMP-2 und Pro-MMP-9 und bewirkt dadurch deren Aktivierung. MMP-2 und MMP-9 gehören zu den Elastin abbauenden Proteasen (A. Thibodeau, Cosmetics & Toiletries 2000, 115 (11), 75-82).

Es wurde festgestellt, dass in alter Haut gegenüber junger Haut der Gehalt an MMPs deutlich erhöht ist (J. H. Chung et al., J. Invest. Dermatol, 2001, 117, 1218-1224). Auch bei der durch exogene Faktoren bedingten vorzeitigen Hautalterung spielen MMPs eine entscheidende Rolle. So konnte in lichtgealterter Haut gegenüber lichtgeschützt gealterter Haut ein nochmals erhöhter Spiegel an MMPs detektiert werden (J. H. Chung et al., J. Invest. Dermatol, 2001, 117, 1218-1224). Sowohl für UVA- und UVB- als auch für Infrarot-Strahlung wurde die Induktion von Matrixmetalloproteinasen nachgewiesen. Diese Induktion konnte sowohl *in vitro* an kultivierten humanen dermalen Fibroblasten als auch *in vivo* an UV-bestrahlter menschlicher Haut beobachtet werden. Auch die Stimulation mit Tabakrauch führte in humanen dermalen Fibroblasten zu einer Aufregulation der MMP-Expression.

Ferner ist häufig, insbesondere aus kosmetischen Gründen, eine Beeinflussung der Hautbräune erwünscht, nach Möglichkeit ohne dass im Zuge der Beeinflussung schädigende Wirkungen auf Hautzellen auftreten. Insbesondere soll eine Hautbräunung auch ohne Einwirkenlassen von UVB-Strahfung auf zu bräunende Haut erreicht werden; ebenso soll eine Hautaufhellung ermöglicht werden.

Versuche, eine solche Bräunung lediglich auf kosmetischem Wege, insbesondere durch Verabreichen topischer Bräunungsmittel bzw. Hautaufhellungsmittel wie Cremes, Emulsionen oder Lotionen zu erreichen, sind bislang wenig erfolgreich geblieben. Zum einen werden herkömmlicherweise als Bräunungsmittel pigmenthaltige Zubereitungen angeboten, wie sie beispielsweise seit langem in Schminkzubereitungen verwendet werden. Als problematisch wird insbesondere empfunden, dass die so hergestellte Bräune durch mechanische Einwirkungen beispielsweise beim Waschen leicht entfernt werden kann, und dass ein natürlicher Braunton einer gesunden gebräunten Haut nur schwer erreichbar ist. In sogenannten "Selbstbräunern" wiederum wird mehrheitlich ein chemisches Oxidationsmittel wie z.B. Dihydroxyaceton als Wirkstoff eingesetzt; dieser Wirkstoff reagiert lediglich mit den Proteinen der Hornschicht der menschlichen Haut und bewirkt durch Oxidation von Histidin und Trytophan eine orange-braune Verfärbung dieser Hornschicht. Der durch diese Oxidation erzeugte Farbton ist zwar unempfindlicher gegenüber mechanischen Einwirkungen als eine Schminke, wird jedoch häufig als unnatürlich und einer Bräunung einer gesunden gebräunten Haut unähnlich empfunden.

Ferner vermittelt die Anwendung herkömmlicher Bräunungsmittel, insbesondere der beschriebenen Pigmetzubereitungen und Selbstbräuner, dem Anwender das unberechtigte Gefühl, durch die rasch und einfach erzielte Hautverfärbung ähnlich geschützt zu sein wie nach natürlicher Hautbräunung, die sich bei Sonnenbestrahlung entwickelt. Diese Fehleinschätzung führt quasi automatisch zu einer Überschreitung der für die Haut akzeptablen Bestrahlungszeit und verursacht damit besonders schlimme Hautschädigungen.

Es ist deshalb versucht worden, die Melaninsynthese der Haut zu stimulieren, ohne dazu Hautschädigungen wie bei Bestrahlung mit UVB-Licht hervorzurufen. Beispielsweise wurde versucht, die von den bekannten Schädigungen der Haut durch UV-Strahlung bewirkte und die Melaninbildung induzierende "SOS-Reaktion" von Hautzellen physiologisch dadurch nachzuahmen, dass man Melanozyten mit bestimmten pTpT-Oligonukleotiden simuliert. Man hoffte, dadurch eine Erhöhung der Melaninkonzentration in der Haut und damit nicht nur eine natürliche Bräunung sondern gleichzeitig auch die damit verbundene und erwünschte Verbesserung des Sonnenschutzes ohne jede UV-Einwirkung zu erreichen. Diese Untersuchungen haben aber nicht zu praktisch brauchbaren Ergebnissen geführt (vgl. Eller et al. Nature 1994, Vol. 372, Seite 414). Neben den bisher unzureichenden Versuchen mit Oligonukleotiden wie pTpT sind bisher keine weiteren Substanzen aufgefunden worden, die das Problem einer besseren Lösung zugeführt hätten. Der Mechanismus, auf welchem Weg die Melaninneubildung in der Haut erfolgt oder besser erfolgen könnte, ohne dass die Zellen bestrahlt und damit zwangsläufig geschädigt werden müssen, sind bisher nicht bekannt geworden. Über ggf. von Melanocyten exprimierte Arylhydrocarbon-Rezeptoren (AhR) ist bisher nur im Zusammenhang mit der Bildung und Ausbreitung von Tumorzellen unter dem Einfluss des extrem toxischen Dioxinderivats 2,3,7,8-Tetrachlorodibenzo-p-dioxin (TCDD) berichtet worden (vgl. Toxicol. Appl. Pharmacol v. 24.06 2005, vgl. unten). Hieraus lassen sich aber für den Fachmann aber keine Schlüsse ziehen, die zur Herstellung eines Mittels zur gefahrlosen Hautbräunung führen könnten. Demzufolge besteht nach wie vor ein sehr großes Bedürfnis nach Bräunungsmitteln, die ohne UVB-Bestrahlung und damit ohne Strahlenschäden und ohne denaturierende Substanzen, welche lediglich eine Verfärbung von Hautproteinen bewirken, durch einfaches Auftragen auf die Haut zu einer Vermehrung des Melanins in den Melanozyten führen, und dadurch ohne weitere Maßnahmen erhöhten Sonnenschutz und gleichzeitig eine von natürlicher Sonnenbräune möglichst nicht zu unterscheidende Hautfarbe erzielen.

Andererseits besteht für viele Menschen das Bedürfnis, ihre natürlicherweise dunkle Hautfarbe aufzuhellen oder der Hautpigmentierung vorzubeugen. Dafür sind sehr sichere und wirkungsvolle Haut- und Haaraufhellungsmittel notwendig.

Hautaufhellende Wirkstoffe greifen in irgendeiner Form in den Melaninmetabolismus bzw. -katabolismus ein. Die in der Regel braun bis schwarz gefärbten Melanin-Pigmente werden in den Melanozyten der Haut gebildet, in die Keratinozyten übertragen und verursachen die Färbung der Haut oder der Haare. Die braun-schwarzen Eumelanine werden in Säugetieren hauptsächlich aus hydroxysubstituierten aromatischen Aminosäuren wie L-Tyrosin und L-DOPA, die gelben bis roten Phäomelanine zusätzlich aus schwefelhaltigen Molekülen gebildet (Cosmetics & Toiletries 1996, 111 (5), 43-51). Ausgehend von L-Tyrosin wird durch das kupferhaltige Schlüsselenzym Tyrosinase L-3,4-Dihydroxyphenylalanin (L-DOPA) gebildet, welches wiederum durch Tyrosinase zu Dopachrom umgewandelt wird. Letzteres wird über mehrere, durch verschiedene Enzyme katalysierte Schritte zu Melanin oxidiert.

Viele Haut- und Haaraufhellungsmittel enthalten mehr oder minder starke Tyrosinase-Inhibitoren. Damit wird jedoch nur ein möglicher Weg der Haut- und Haaraufhellung beschritten.

Darüber hinaus werden auch UV-absorbierende Substanzen zum Schutz gegen die durch UV-Licht induzierte Zunahme der Hautpigmentierung eingesetzt. Dies ist jedoch ein rein physikalisch bedingter Effekt und muss unterschieden werden von der biologischen Wirkung hautaufhellender Mittel auf die zelluläre MelaninBildung, die auch in Abwesenheit von UV-Licht nachweisbar ist. UV-Absorber bewirken zudem keine echte Hautaufhellung, sondern verhindern lediglich die durch UV-Licht induzierte Zunahme der Hautpigmentierung.

In handelsüblichen kosmetischen oder therapeutischen Haut- und Haaraufhellungsformulierungen werden insbesondere Hydrochinon, Hydrochinonderivate wie z.B. Arbutin, Vitamin C, Derivate der Ascorbinsäure wie z.B. Ascorbylpalmitat, Kojisäure und Derivate der Kojisäure wie z.B. Kojisäuredipalmitat verwendet.

Einer der am häufigsten verwendeten Haut- und Haaraufheller ist Hydrochinon. Diese Verbindung hat jedoch einen zytotoxischen Effekt auf Melanozyten und wirkt irritierend auf die Haut. Daher sind solche Präparate z.B. in Europa, Japan und Südafrika für kosmetische Anwendungen nicht mehr zulässig. Zudem ist Hydrochinon sehr oxidationsempfindlich und nur schwierig in kosmetischen Formulierungen zu stabilisieren.

Arbutin ist ein Hydrochinon-Glucosid, das in situ zu Hydrochinon hydrolysiert und daher toxikologisch ebenso bedenklich ist wie Hydrochinon.

Vitamin C und Ascorbinsäurederivate haben nur eine unzureichende Wirkung auf der Haut. Sie wirken zudem nicht direkt als Tyrosinase-Hemmstoffe, sondern reduzieren die farbigen Zwischenstufen der Melaninbiosynthese.

Kojisäure (5-Hydroxy-2-hydroxymethyl-4-pyranon) ist ein Tyrosinaseinhibitor, der über eine Chelatisierung der Kupferatome des Enzyms dessen katalytische Wirkung hemmt; sie wird in kommerziellen Haut- und Haaraufheilungsmitteln eingesetzt, besitzt jedoch ein hohes sensibilisierendes Potential und verursacht Kontaktallergien.

Es war eine Aufgabe der vorliegenden Erfindung, den Nachteilen des Standes der Technik abzuhelfen und insbesondere hochwirksame Hautbräuner und Hautaufheller anzugeben, die eine möglichst natürliche Hautbräunung bzw. eine möglichst wirksame Verhinderung einer Hautbräunung bzw. eine Hautaufhellung, jeweils nach Möglichkeit ohne Schädigung von Hautzellen, bewirken sollen.

Es wurde nun überraschend gefunden, dass durch Beeinflussen des Arylkohlenwasserstoff-Rezeptors (AhR) sowohl eine Hautbräunung als auch eine Hautaufhellung erzielbar ist. Der Arylkohlenwasserstoff-Rezeptor (AhR) (NCBI-Gen-Akzessionsnummer BC0700800) war bisher lediglich als zentrales Element bei der Detoxifikation von exogenen Schadstoffen bekannt. Der Rezeptor vermittelt die biologische Antwort auf polyzyklische aromatische Kohlenwasserstoffe (PAK) wie Benz[a]pyren und halogenierte PAK wie 2,3,7,8-Tetrachlorodibenzo-p-dioxin (TCDD). Der AhR ist ein Liganden-aktivierter Transkriptionsfaktor, der nach Bindung eines Liganden in den Zellkern transloziert. Dort bildet er ein Dimer mit einem weiteren Transkriptionsfaktor, dem Aryl Hydrocarbon Receptor Nuclear Translocator (ARNT), bindet an regulatorische Gensequenzen und induziert die Transkription von verschiedenen Genen wie z.B. CYP1A1 und CYP1B1. Folgen der AhR-Aktivierung ist die Entwicklung von Hauttumoren (Shimizu et al. (2000) 97:779), Hautirritationen und -entzündungen (Inflammation), die Entwicklung von Allergien, atopischer Dermatitis sowie von Juckreiz und eine Störung der Hautintegrität (Tauchi et al. (2005) Mol. Cell Biol. 25: 9360-8; Henley et al., Arch. Biochem. Biophys. (2004) 422: 42-51) sowie die Induktion der MMP-1 (Kollagenase-1) (Murphy et al. (2004) J. Biol. Chem. 279, 25284-2593).

UVB-Licht induziert die CYP1A1-Expression in humanen Keratinozyten und Lymphozyten sowie in der Maus-Hepatoma-Zeillinie Hepa-1 (Wei et al., Chem Biol Interact (1999) 118: 127-40). Jedoch wurde nur für Maus-Hepa-1-Zellen nachgewiesen, dass die CYP1A1-Induktion AhR-abhängig ist. Die AhR-Aktivierung ist jedoch, wie weiter unten ausgeführt wird, abhängig vom Zelltyp, so dass nicht von der Wirkung an Maus-Hepatoma-Zellen auf die Wirkung auf humane Hautzellen extrapoliert werden kann. Des weiteren kann CYP1A1 auch durch AhR-unabhängige Wege induziert werden (Guigal et al. (2001) Life Sci. ;68(18):2141-50; Tijet et al. (2006), Mol Pharmacol 69(1): 140-153). Daher ist kein zwingender Zusammenhang zwischen UVB - AhR - CYP1A1 in Melanozyten und Keratinozyten nicht gegeben.

Aus der WO 99/56737 sind Stilbene als Liganden des Ah-Rezeptors bekannt. Einige der Stilbene sollen zwar an den Ah-Rezeptor binden, jedoch keine CYP1A1-Induktion bewirken. Zu diesen Stilbenen gehören 3,4,3',5-Tetrahydroxystilben oder Piceatannol, 2,3',4,5'-Tetrahydroxystilben oder Oxyresveratrol und 3,5,4'-Trihydoxystilben oder Resveratrol, insbesondere trans-Resveratrol. Eine photoprotektive Wirkung, insbesondere gegen UVB-Strahlung, ist nicht beschrieben. Nachteilig an den Stilbenen ist, dass diese photolabil sind und häufig endokrine Wirkungen hervorrufen. Beispielsweise ist Resveratrol ein Anti-Androgen (Mitchell et al. (1999) Cancer Res. 59: 5892-5895).

Henry et al (Mol Pharmacol 55 (1999):716-25) beschreiben 3-methoxylierte Flavone, die in 4-Stellung einen elektronenziehenden Substituenten tragen, als effektive AhR-Antagonisten in Leberzellen. Joiakim et al. (Drug Metab Dispos 31 (2003):1279-82) zeigten, dass der Jun N-terminal Kinase-Inhibitor Anthra[1,9-cd]pyrazol-6(2H)-on die Wirkung des potenten AhR-Agonisten 2,3,7,8-Tetrachlorodibenzo-p-dioxin (TCDD) in humanen Brustepitheizellen hemmen kann.

Die Bindung an den AhR ist zudem abhängig vom Zelltyp. Zhang et al (Environ. Health Perspec. 111 (2003): 1877-1882) haben gefunden, dass z.B. Quercetin die Wirkung von AhR in der humanen Brustkrebszelllinie MCF-7 verhindert, jedoch keinen Effekt auf die humane Leberkrebszelllinie HepG2 hat. Ein gegenteiliger Effekt wurde für Luteolin gefunden, welches keinen Effekt auf MCF-7-Zellen hat, aber als AhR-Inhibitor in HepG2-Zellen wirkt. Auch wurden Unterschiede in der Liganden-Affinität des AhR zwischen humanen Zellen und Nager-Zellen festgestellt (Ema et al. (1994) J. Biol. Chem. 269: 27337-43; Zhang et al (2003), Environ. Health Perspec. 111: 1877-1882).

Verbindungen, die als AhR-Antagonisten in humanen Hautzellen fungieren, sind kaum bekannt. Curcumin hemmt zwar die AhR-Aktivierung durch das Tabak-Karzinogen Benz[a]pyren-7R-trans-7,8-dihydrodiol in oralen humanen Keratinozyten-Krebszellen und in *ex vivo* Mundschleimhaut. Jedoch aktiviert Curcumin die AhR-Translokation in Abwesenheit des Tabak-Karzinogens (Rinaldi et al. (2002) Cancer Res. 62, 5451-5456) und ist damit kein AhR-Antagonist im Sinne der Erfindung.

Ferner ist bekannt, dass All-trans-Retinsäure die TCDD-induzierte AhR-Aktivierung in normalen humanen Keratinozyten hemmt, ohne die AhR-Aktivität in Abwesenheit von TCDD zu beeinflussen. All-trans-Retinsäure hat zudem den erheblichen Nachteil der Verstärkung der TCDD-induzierten Expression der MMP-1 (Murphy et al. (2004) J. Biol. Chem. 279, 25284-25293) und ist photolabil. Villano et al. (Toxicology and Applied Pharmacology 2005, 212-224) beschreiben, dass TCDD die Expression von Matrix-Metalloproteinase induziert. Das Dokument offenbart jedoch ebenso wenig wie die EP 1 382 329 A1 ein Verfahren, bei dem für jede Probe das Behandeln von Zellen mit UVB-Strahlung durchgeführt wird.

Hochdurchsatz-Screening-Verfahren sind beispielsweise bekannt aus US-A-5 959 738, EP-A-1 382 329, Pliskova et al. (Toxicology 2005, 80-89), JP 06-016531, Zhang et al. (Journal of Parenteral Science and Technology 2003, 1877-1882) und Amakura et al, (Biological and Pharmaceutical Bulletin of Japan 2003, 1754-1760). Die Verfahren umfassen jedoch nicht alle Schritte des Anspruchs 2, und sie offenbaren auch nicht eine Verbindung gemäß Anspruch 6 oder 9.

Erfindungsgemäß werden deshalb Ah-Rezeptor-Modulatoren (AhR-Agonisten und AhR-Antagonisten) sowie Verfahren zum Beurteilen der Wirksamkeit einer zu untersuchenden Substanz als AhR-Antagonist bzw. AhR-Agonist bereitgestellt. Dabei ist ein Ah-Rezeptor-Antagonist (AhR-Antagonist) im Sinne dieser Erfindung eine Substanz, die
1. in Hautzellen, insbesondere in vorzugsweise humanen Melanozyten oder Keratinozyten,
2. die AhR-vermittelte Induktion eines AhR-induzierbaren Gens, vorzugsweise von CYP1A1, verringert oder verhindert, und dabei
3. in Abwesenheit von UVB-Strahlung und/oder eines AhR-Induktors bzw - Agonisten, vorzugsweise eines polycyclischen aromatischen Kohlenwasserstoffs und besonders bevorzugt Benzy[a]pyren oder 3-Methylcholanthren, nicht selbst eine Induktion eines AhR-induzierbaren Gens, vorzugsweise von CYP1A1, auslöst und/oder die Translokation von AhR aus dem Zytoplasma in den Zellkern induziert, und ferner
4. vorzugsweise photostabil ist.

Ein Ah-Rezeptor-Agonist (AhR-Agonist) im Sinne dieser Erfindung ist eine Substanz, die
1. in Hautzellen, insbesondere in vorzugsweise humanen Melanozyten oder Keratinozyten,
2. die Expression eines AhR-induzierbaren Gens, vorzugsweise von CYP1 A1, induziert, und ferner
3. vorzugsweise photostabil ist.

Photostabil im Sinne dieser Erfindung ist eine Substanz, die bei 2stündiger Bestrahlung mit UVB-Strahlung (40 W/m²) zu nicht mehr als 10 mol% In eine oder mehrere andere Substanzen umgewandelt wird. Dementsprechend ist beispielsweise All-trans-Retinsäure kein bevorzugter AhR-Antagonist, da diese Substanz photolabil ist.

Im Sinne dieser Erfindung wird ein Gen als induziert bezeichnet, wenn die Konzentration der zugehörigen mRNA in Anwesenheit des zugeordneten Induktors bzw. Agonisten signifikant (p < 0.05, Student's t-test) zumindest 10 % höher Ist als in Abwesenheit des Induktors bzw. Agonisten.

Eine Ah-Rezeptor-Sequenz ist beispielsweise hinterlegt unter der NCBI-Nummer BC070080.

Erfindungsgemäß wird nunmehr ein Verfahren bereitgestellt zum Beurteilen der Wirksamkeit eines AhR-Modulators mit den Schritten gemäß Anspruch 1. Diese umfassen:
i) Beaufschlagen einer Zelle einer ex-vivo- oder in vitro- Melanozyten- und/oder Keratinozyten-Zellkultur oder eines in vitro-Hautmodells, mit einem möglichen AhR-Agonisten bzw AhR-Antagonisten,
ii) wenn die Zelle in Schritt i) mit einem möglichen AhR-Antagonisten beaufschlagt wurde Behandeln der beaufschlagten Zelle mit UVB-Strahlung oder einem AhR-Agonisten, und
iii) Bestimmen der Induktion eines AhR-induzierbaren Gens, vorzugsweise von CYP1A1,
sowie die weiter unten beschriebenen Schritte iv) bis vii).

Das erfindungsgemäße Beurteilungsverfahren ermöglicht nunmehr erstmals, Substanzen auf ihre Wirksamkeit als AhR-Modulator, also als Ah-Rezeptor-Agonist bzw. -Antagonist zu testen, ohne diesen Test am Körper eines lebenden Tieres oder einer lebenden Versuchsperson vornehmen zu müssen, sondern in einem ex vivo bzw. in vitro-Ansatz eine repräsentative Beurteilung der in vivo-Agonisten- bzw. Antagonistenwirksamkeit zu erreichen. Die Erfindung öffnet das Gebiet der Ah-Rezeptor-Agonisten und -Antagonisten somit erstmals einer systematischen Erforschung unter geringem Einsatz an Material, wobei die Ergebnisse der Beurteilung erstmals rasch gewonnen werden können und nicht, wie bei einer Behandlung am Körper eines Tieres oder eines Probanden, erst nach Entnahme weiterer Proben oder langfristiger Beobachtung behandelter Hautpartien erst sehr spät oder gar nicht gewonnen werden können. Das erfindungsgemäße Verfahren ermöglicht daher auch erstmals die systematische Suche nach Substanzen zum Verringern oder Verhindern einer Translokation von AhR in einen Zellkern, zum Verringern oder Verhindern einer UVB-induzierten oder -induzierbaren Genexpression, zum Verringern oder Verhindern einer durch polyzyklische aromatische Kohlenwasserstoffe wie insbesondere TCDD-induzierten oder -induzierbaren Genexpression, und/oder zum Verringern oder Verhindern von UVB-induzierten oder -induzierbaren Hautschäden, insbesondere Hautkrebs, Hautalterung, Hautentzündungen und Sonnenbrand. Ebenfalls ermöglicht das erfindungsgemäße Verfahren, systematisch potentielle AhR-Agonisten zu erproben und ihre Wirkung auf die Melaninbildung in einer Zellkultur reproduzierbar zu bestimmen. Das erfindungsgemäße Beurteilungsverfahren ermöglicht zudem, Substanzen zu erproben, deren Toxizität vor Durchführen des Beurteilungsverfahrens nicht oder nicht vollständig überprüft wurde. Damit erschließt das erfindungsgemäße Beurteilungsverfahren weitere Substanzklassen, die einer Beurteilung an Testpersonen zuvor nicht zugänglich waren. Das Verfahren unterstützt zudem die Auswahl geeigneter Agonisten und Antagonisten, indem das Verfahren erlaubt, potentielle Agonisten und Antagonisten anhand einer vorgewählten Steigerung bzw. Verringerung der Induktion des AhR-induzierbaren Gens, vorzugsweise von CYP1A1, standardisiert und reproduzierbar auszuwählen.

Als Zellkultur wird vorzugsweise eine Kultur bestehend aus oder enthaltend Melanozyten und/oder Keratinozyten verwendet. Ebenfalls bevorzugt ist es, eine Zellkultur eines ex vivo- oder in vitro-Hautmodells zu verwenden. Zweckmäßigerweise wird die gesamte Zellkultur mit dem potentiellen AhR-Agonisten bzw. -Antagonisten behandelt. Die Zelkultur umfasst in bevorzugten Ausführungsformen der Erfindung zumindest 20 Zellen, stärker bevorzugt zumindest 100 Zellen und besonders bevorzugt zumindest 200 Zellen.

Ein besonders bevorzugtes Verfahren zum Beurteilen der Wirksamkeit eines AhR-Modulators umfasst die Schritte:
i) Bereitstellen von vier Zellgruppen einer in vitro- Melanozyten- und/oder Keratinozyten-Zellkultur oder eines ex-vivo- oder in vitro-Hautmodells enthaltend Keratinozyten sowie optional zusätzlich Melanozyten und weitere Hautzelltypen,
ii) Behandeln der Zellgruppen nach folgendem Schema:

| Schritt | Zellgruppe 1 | Zellgruppe 2 | Zellgruppe 3 | Zellgruppe 4 |
|---|---|---|---|---|
| 1. | Behandeln mit einem zu untersuchenden möglichen AhR-Modulator, | kein Behandeln mit einem zu untersuchenden möglichen AhR-Modulator, | Behandeln mit einem zu untersuchenden möglichen AhR-modulator, | kein Behandeln mit einem zu untersuchenden möglichen AhR-Modulator, |
| 2. | kein Behandeln mit einem vorgewählten AhR-Agonisten oder UVB-Strahlung, | Behandeln mit einem vorgewählten AhR-Agonisten oder UVB-Strahlung, | Behandeln mit einem vorgewählten AhR-Agonisten oder UVB-Strahlung, | kein Behandeln mit einem vorgewählten AhR-Agonisten oder UVB-Strahlung, |
| 3. | Bestimmen der Induktion eines AhR-induzierbaren Gens, vorzugsweise von CYP1A1. | Bestimmen der Induktion eines AhR-induzierbaren Gens, vorzugsweise von CYP1A1. | Bestimmen der Induktion eines AhR-induzierbaren Gens, vorzugsweise von CYP1A1. | Bestimmen der Induktion eines AhR-induzierbaren Gens, vorzugsweise von CYP1A1. |

Ein AhR-Modulator wird dann daran erkannt, dass in Zellgruppe 1 das AhR-induzierbare Gen induziert wird (AhR-Agonist) oder nicht induziert wird (AhR-Antagonist), während in Zellgruppe 2 das AhR-induzierbare Gen induziert wird (Positivkontrolle). Ein AhR-Modulator wird ebenfalls daran erkannt, dass in Zellgruppe 3 das AhR-induzierbare Gen induziert wird (AhR-Antagonist) oder nicht induziert wird (AhR-Agonist), während in Zellgruppe 4 das AhR-induzierbare Gen nicht induziert wird (Negativkontrolle). Der Fachmann versteht, dass das Verfahren auch lediglich mit den Zellgruppen 1 und 2 bzw. mit den Zellgruppen 3 und 4 durchgeführt werden kann.

Soweit im folgenden nichts anderes gesagt ist, wird als Keratinozyten-Zellkultur eine Zellkultur menschlicher Keratinozyten verwendet. Bevorzugte Keratinozyten sind HaCaT-Zellen und NCTC2544-Keratinozyten. Bevorzugte Melanozyten sind murine Melanozyten, insbesondere B16-Zellen.

Das erfindungsgemäße Verfahren zum Beurteilen der Wirksamkeit eines AhR-Antagonisten umfasst ferner die Schritte:
iv) Beaufschlagen einer unbehandelten Zelle (vorzugsweise einer vorzugsweise murinen Melanozyten- oder Keratinozyten-Zelle und insbesondere vorzugsweise einer B16-, NCTC2544- oder HaCaT-Zelle) aus Schritt i) mit einer vorgewählten Verbindung wie unten beschrieben, vorzugsweise der Formel (II) und insbesondere vorzugsweise der Formel (X),
v) Behandeln der in Schritt iv) beaufschlagen Zelle mit UVB-Strahlung und/oder einem AhR-Agonisten wie in Schritt ii),
vi) Bestimmen der Induktion des in Schritt iii) bestimmten AhR-induzierbaren Gens, vorzugsweise von CYP9A1, und
vii) Vergleichen der in Schritt iii) und vi) bestimmten Geninduktion.

Das erfindungsgemäße Bestimmungsverfahren ist auf diese Weise besonders leicht standardisierbar und unterstützt es auf vorteilhaft einfache Weise, AhR-Antagonisten mit einer vorgewählen Mindest-antagonistischen Wirkung zu erzielen.

Besonders bevorzugt werden in Schritt ii) und gegebenenfalls in Schritt v) die beaufschlagte(n) Zellen(n) mit einem polyzyklischen aromatischen Kohlenwasserstoff und vorzugsweise mit TCDD behandelt. Ein derartiges erfindungsgemäßes Beurteilungsverfahren ermöglicht auch eine Analyse dazu, ob der beurteilte AhR-Antagonist zusätzlich zur Detoxifizierung, also zum Verhindern oder Unterdrücken schädlicher Auswirkungen polyzyklische aromatischer Kohlenwasserstoffe und insbesondere von TCDD geeignet ist. Der sichere und systematische Nachweis einer solchen detoxifizierenden Wirkung war zuvor, ohne das erfindungsgemäße Beurteilungsverfahren, praktisch nicht möglich, da eine absichtliche, kontrollierte Exposition von Testpersonen mit polyzyklischen aromatischen Kohlenwasserstoffen wegen des mit der Verwendung dieser Substanzen verbundenen Gsundheitsrisikos vermieden werden musste.

Erfindungsgemäß wird zudem ein Screening-Verfahren angegeben zum Screenen einer Substanzbibliothek auf AhR-Agonisten und AhR-Antagonisten, umfassend die Schritte:
1) Bereitstellen einer Probe jeder der Substanzen der Substanzbibliothek, und
2) für jede der in 1) bereitgestellten Proben Durchführen eines Verfahrens nach einer der zuvor beschriebenen Arten,
3) Auswählen derjenigen Substanzen als AhR-Antagonisten, bei deren Proben die Induktion eines AhR-induzierbaren Gens um ein vorgewähltes Maß verringert wurde; bzw. Auswählen derjenigen Substanzen als AhR-Agonisten, bei deren Proben die Expression eines AhR-induzierbaren Gens um ein vorgewähltes Maß erhöht wurde.

Das Screening-Verfahren verwirklicht die mit dem erfindungsgemäßen Beurteilungsverfahren verbundenen Vorteile. Es ist insbesondere als Hochdurchsatz-Verfahren mit zumindest 96 Proben durchführbar, wobei eine oder mehrere Proben Kontrollproben sein können, insbesondere Positiv- und/oder Negativkontrollen. Die Proben sind dabei vorzugsweise zusammen auf einem Träger, beispielsweise einer Mikrotiterplatte, angeordnet.

Eine Vorrichtung zum Durchführen eines erfindungsgemäßen Hochdurchsatz-Screeningverfahrens umfasst:
- eine Probenaufnahme für zumindest 96 Proben,
- Leuchtmittel zum Beaufschlagen der Proben mit UVB-Strahlung,
- Mittel zum Bestimmen der Induktion eines AhR-induzierbaren Gens in einer der 96 Proben, und
- Auswertemittel zum Anzeigen der Induktion des AhR-induzierbaren Gens.

Die Hochdurchsatz- (high throughput, HTS)-Screeningvorrichtung verringert auf vorteilhafte Weise den mit der Durchführung einer systematischen AhR-Agonisten und -Antagonisten-Suche verbundenen Aufwand. Durch ihre vorzugsweise selbsttätige Arbeit gestattet sie eine Standardisierung der Versuchsbedingungen, fördert die Vergleichbarkeit der Auswertungsergebnisse und beschleunigt so die Agonisten- und Antagonisten-Suche.

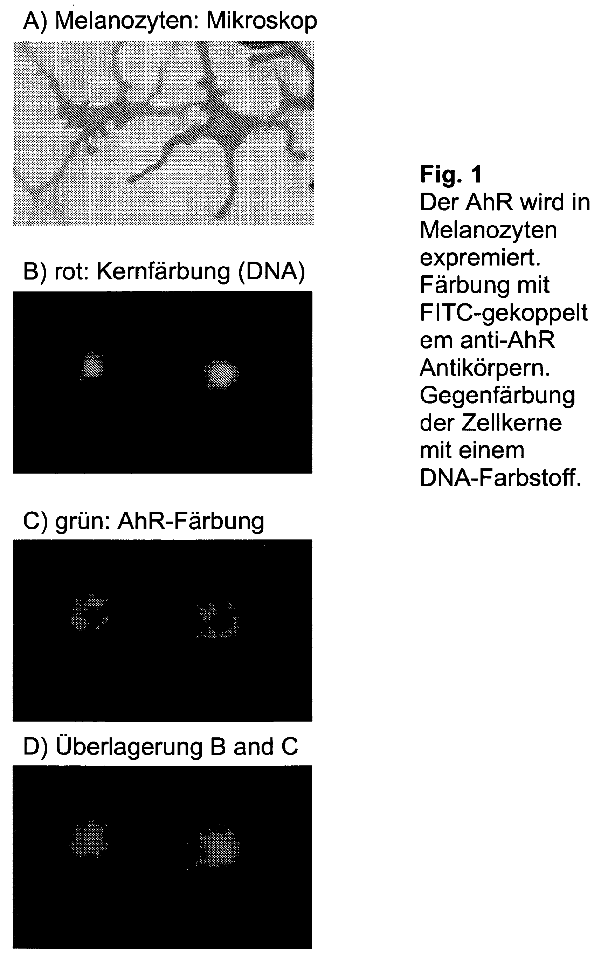

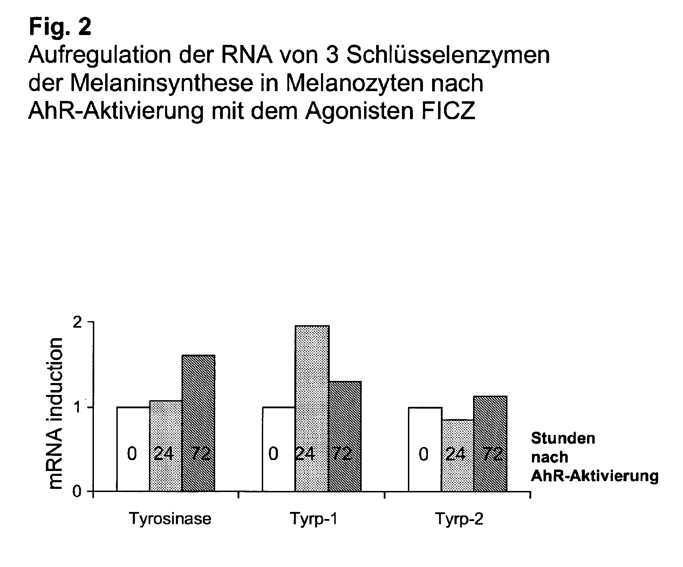

Es wurde nunmehr gefunden, dass primäre Hautmelanozyten diesen Rezeptor exprimieren (Figur 1). Weiter wurde gefunden, dass die Aktivierung des Rezeptors mit Hilfe eines AhR-Modulators die Expression der Tyrosinase, d.h, des Schlüsselenzyms der Melaninsynthese, überraschenderweise um den Faktor 2-3 zu induzieren vermag (Figur 2), und dass es in Folge zu einer vermehrten Melaninneubildung kommt (Figur 3). Dagegen haben Inhibitoren des AhR-Signalweges eine Melanin-reduzierende Wirkung (Figur 4).

Die Suche nach AhR-Agonisten und -Antagonisten ist daher insbesondere hingeordnet auf das Ziel, Mittel zum Schützen oder Behandeln tierischer und vorzugsweise menschlicher Haut anzugeben. Erfindungsgemäß wird dementsprechend auch ein Verfahren gemäß Anspruch 3 angegeben zum Herstellen einer Hautschutzzubereitung, umfassend das Mischen eines AhR-Antagonisten - vorzugsweise eines mit einem der vorgenannten erfindungsgemäßen Verfahren gefundenen AhR-Antagonisten - mit einem kosmetisch und/oder pharmazeutisch akzeptablen Träger, so dass die Konzentration des AhR-Antagonisten in der Mischung zumindest das Doppelte der zum Verringern der Induktion eines AhR-induzierten Gens erforderlichen Mindestkonzentration beträgt, wobei die Mindestkonzentration bestimmt wird oder bestimmbar ist mit einem Verfahren wie in Anspruch 3 angegeben.

Ebenfalls wird ein Verfahren angegeben zum Herstellen einer Hautbäunungszubereitung, umfassend das Mischen eines AhR-Agonisten - vorzugsweise eines mit einem der vorgenannten erfindungsgemäßen Verfahren gefundenen AhR-Agonisten - mit einem kosmetisch und/oder pharmazeutisch akzeptablen Träger, so dass die Konzentration des AhR-Agonisten in der Mischung zumindest das Doppelte der zum Erhöhen der Expression eines AhR-induzierbaren Gens erforderlichen Mindestkonzentration beträgt, wobei die Mindestkonzentration bestimmt wird oder bestimmbar ist mit einem Verfahren nach einem der vorgenannten erfindungsgemäßen Verfahren.

In den erfindungsgemäß hergestellten Hautschutzzubereitungen und den Hautbräunungsmitteln beträgt die Konzentration an AhR-Antagonisten bzw. AhR-Agonisten vorzugsweise nicht mehr als das 20fache der oben beschriebenen Mindestkonzentration, besonders bevorzugt nicht mehr als das 10flache.

Die erfindungsgemäßen Verfahren zum Beurteilen der Wirksamkeit eines AhR-Agonsten bzw. -Antagonisten ermöglichen die Bestimmung einer Mindestkonzentration, indem das jeweilige Verfahren mehrfach mit unterschiedlichen Konzentrationen des jeweiligen Agonisten bzw. Antagonisten durchgeführt wird. Die Verfahren unterstützen somit das Herstellen einer Hautschutzzubereitung bzw. Hautbräunungszubereitung, indem sie durch Bestimmbarmachen der Mindestkonzentration einen für das Herstellen einer Hautschutzzubereitung bzw. Hautbräunungszubereitung essentiellen Wert mit hoher Genauigkeit und hoher Prognosesicherheit hinsichtlich der Anwendung am Menschen liefern.

Es hat sich nunmehr gezeigt, dass ein erheblicher Anteil der AhR-Antagonisten eine hautaufhellende Wirkung und ein erheblicher Anteil der AhR-Agonisten eine hautbräunende Wirkung besitzen. Erfindungsgemäß wird deshalb auch ein Verfahren zum Beurteilen der Wirksamkeit eines Hautaufhellers gemäß Anspruch 4 angegeben, umfassend die Schritte
i) Beaufschlagen einer Zelle mit einem möglichen Hautaufheller,
ii) Bestimmen des Maßes der Melaninbildung der in Schritt i) behandelten Zelle,
iii) Beaufschlagen einer unbehandelten Zelle aus Schritt i) mit einer vorgewählten Menge a) einer Verbindung der Formel (X), wenn die Wirksamkeit eines Hautaufhellers beurteilt werden soll, und b) mit Formylindolo(3,2b)carbazol, wenn die Wirksamkeit eines Hautbräuners beurteilt werden soll,
iv) Bestimmen des Maßes der Melaninbildung der in Schritt iii) behandelten Zelle,
v) Vergleichen der in Schritt ii) und iv) bestimmten Hautaufhellung.

Analog wird ein Verfahren zum Beurteilen der Wirksamkeit eines Hautbräuners angegeben, umfassend die Schritte
i) Beaufschlagen einer Zelle mit einem möglichen Hautbräuner,
ii) Bestimmen des Maßes der Melaninbildung der in Schritt i) behandelten Zelle,
iii) Beaufschlagen einer unbehandelten Zelle aus Schritt i) mit einer vorgewählten Menge einer Hautbräuner-Standardsubstanz, vorzugsweise Naringin oder Coffein,
iv) Bestimmen des Maßes der Melaninbildung der in Schritt iii) behandelten Zelle,
v) Vergleichen der in Schritt ii) und iv) bestimmten Hautbräunung.

Dabei sind die Schritte iii-v optional.

Die Verfahren ermöglichen es, die Vorteile eines erfindungsgemäßen Verfahrens zum Beurteilen der Wirksamkeit eines AhR-Antagonisten bzw. AhR-Agonisten entsprechend zu erzielen, insbesondere die Reproduzierbarkeit, die Möglichkeit, systematisch nach Hautaufhellern bzw. Hautbräunem zu suchen, die hohe Prognosesicherheit für die Anwendung am Menschen und die Möglichkeit, das Verfahren als Hochdurchsatz-Verfahren durchzuführen, beispielsweise mit Hilfe der oben beschriebenen erfindungsgemäßen Vorrichtung. Bevorzugt werden die in den obigen Verfahren untersuchten möglichen Hautaufheller zuvor in einem erfindungsgemäßen Verfahren auf ihre Wirkung als AhR-Antagonisten überprüft; ebenso werden bevorzugt die in den obigen Verfahren untersuchten möglichen Hautbräuner zuvor in einem erfindungsgemäßen Verfahren auf ihre Wirkung als AhR-Agonisten überprüft.

Als Zellen in Schritt i) werden vorzugsweise Melanozyten verwendet, besonders bevorzugt murine Melanozyten. Wie eingangs beschrieben werden zweckmäßigerweise zumindest 20 Zellen, stärker bevorzugt zumindest 100 Zellen und besonders bevorzugt zumindest 200 Zellen behandelt.

Entsprechend wird erfindungsgemäß ebenfalls ein Verfahren angegeben zum Herstellen einer Hautaufnellungszubereitung gemäß Anspruch 5, umfassend das Mischen eines Hautaufhellers mit einem kosmetisch und/oder pharmazeutisch akzeptablen Träger, so dass die Konzentration des Hautaufhellers in der Mischung zumindest das Doppelte der zum Hautaufhellen erforderlichen Mindestkonzentration beträgt, wobei die Mindestkonzentration bestimmt wird mit einem Verfahren der soeben beschriebenen Art.

Ein bevorzugter Wirkstoff zum Herstellen einer Hautbräunungszubereitung ist Formylindolo(3,2b)carbazol, sowie dessen pharmazeutisch verträglich Salze und Ester. Ein erfindungsgemäß bevorzugter Wirkstoff zum Herstellen einer hautaufhellenden Zubereitung ist 3'-Methoxy-4'-nitroflavon, sowie dessen pharmazeutisch verträgliche Salze und Ester.

Erfindungsgemäß wurden einige wertvolle AhR-Antagonisten gefunden, nämlich Verbindungen der Formel (II) 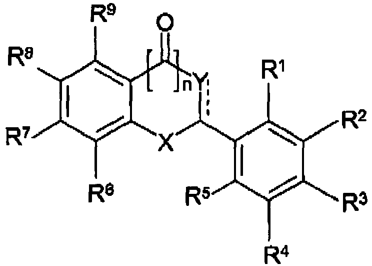 wobei
R¹ bis R⁹ unabhängig voneinander Wasserstoff, Hydroxy, C₁-C,₂-Alkoxy, C₁-C₁₂-Alkyl, C₂-C₁₂Alkenyl, und
n = 0 und Y = -CH- oder -C(CH₃)-, X = O und die gestrichelte Linie entsprechend entweder eine Doppelbindung oder zwei,
oder
n = 1, Y = O und X = Methylen und gestrichelte Linie zwei Wasserstoffe
bedeuten.

Die Verbindungen der Formel (II) haben sich überraschenderweise als sehr wirkungsvolle Ah-Rezeptor-Antagonisten erwiesen. Sie können in menschlichen Hautzellen die UVB-induzierte Translokation des AhR vom Zytoplasma in den Zellkern verhindern. Sie verringern stark die AhR-vermittelte Induktion von AhR-induzierbaren Genen in menschlichen Hautzellen, insbesondere die Induktion von CYP1A1. Sie sind photostabil und lösen in Abwesenheit einer AhRaktivierenden Substanz, insbesondere von polyzyklischen aromatischen Kohlenwasserstoffen wie TCDD, keine Induktion eines AhR-induzierbaren Gens aus und induzieren auch nicht die Translokation von AhR aus dem Zytoplasma in den Zellkern menschlicher Hautzellen, im Gegensatz beispielsweise zu Curcumin und All-trans-Retinsäure.

Die Verbindungen der Formel (II) sind deshalb besonders geeignet als Arzneimittel, insbesondere zum Behandeln von oder zur Vorbeugung gegen -insbesondere UVB-induzierte - Hautreizungen, gegen Hautschäden, Entzündungen der Haut, Juckreiz, atopischer Dermatitis, Hautalterung, Hautkrebs, und/oder zum Verringern des MMP-Gehaltes der Haut. Die Verbindungen sind ferner, als Arzneimittel oder in einer Nicht-Arzneimittel-Form, beispielsweise als Kosmetikum oder in kosmetischen Zubereitungen, geeignet zum Verringern oder Verhindern einer Translokation von AhR in einen Zellkern, zum Verringern oder Verhindern einer UVB-induzierten Genepression, und/oder zum Verringern oder Verhindern einer durch AhR-Agonisten wie polyzyklische aromatische Kohlenwasserstoffe und insbesondere TCDD induzierten Genexpression. Die Verbindungen der Formel (II) sind ferner geeignet als Sonnenschutzmittel und insbesondere als UVB-Schutzmittel.

Bevorzugte erfindungsgemäße Verbindungen sind solche, bei denen R¹ bis R⁹, unabhängig voneinander, Wasserstoff, Hydroxy, C₁-C₄-Alkoxy (verzweigt oder unverzweigt) oder C₂-C₄-Alkenyl bedeuten. Vorzugsweise sind nicht mehr als fünf und besonders bevorzugt nicht mehr als vier der Reste R¹ bis R⁹ nicht Wasserstoff. Ist einer der Reste Hydroxy oder C₁-C₄-Alkoxy, so können in bevorzugten Ausführungsformen die übrigen, also vorzugsweise maximal drei Reste gleich H oder C₁-C₄-Alkyl (verzweigt oder unverzweigt) sein. Derart substituierte Verbindungen haben sich als besonders wirksame AhR-Antagonisten erwiesen.

Insbesondere ist bevorzugt die Verbindung der Formel (X)

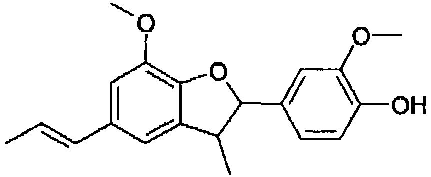

Diese Verbindung ermöglichen bereits in sehr niedrigen Einsatzkonzentrationen eine starke Inhibierung des Ah-Rezeptors und verhindern oder verringen bereits in niedrigen Konzentrationen die AhR-vermittelte Induktion AhR-induzierbarer Gene, insbesondere CYP1A1. Die Verbindung der Formel (X) wirkt zudem hautaufhellend. Darüber hinaus bewirkt die Verbindung der Formel (X) in Abwesenheit von UVB-Strahlung nicht selbst eine Induktion eines AhR-induzierbaren Gens, insbesondere von CYP1A1, und induzieren unter diesen Bedingungen auch nicht die Translokation von AhR aus dem Zytoplasma in den Zellkern. Die Verbindung der Formel (X) ist deshalb besonders geeignet für die oben beschriebenen Verwendungen von AhR-Antagonisten, und ist insbesondere bevorzugt als Arzneimittel oder Bestandteil pharmazeutischer oder nicht-pharmazuetischer und insbesondere kosmetischer Zubereitungen.

Erfindungsgemäß werden ferner Zubereitungen angegeben gemäß Anspruch 10, enthaltend eine oder mehrere Verbindungen der Formel (II), insbesondere der Formel (X). Zweckmäßigerweise liegen die Verbindungen der Formel (II) und insbesondere die Verbindung der Formel (X) in erfindungsgemäßen Zubereitungen vor in einer ausreichenden Menge (a) zum Verringern oder Verhindern einer Translokation von AhR in einen Zellkern, (b) zum Verringern oder Verhindern einer UVB-induzierten Genexpression, und/oder Verringern oder Verhindern einer durch polyzyklische aromatische Kohlenwasserstoffe, vorzugsweise TCDD, induzierten oder induzierbaren Genexpression, und/oder (d) Verringern oder Verhindern von UVB-induzierten oder -induzierbaren Hautschäden, insbesondere Hautkrebs, Hautalterung, Hautentzündungen und Sonnenbrand. Die Verbindung der Formel (X) liegt zudem vorzugsweise in einer zur Hautaufhellung ausreichenden Menge vor.

Die erfindungsgemäßen Zubereitungen enthalten die Verbindung(en) der Formel (II), insbesondere der Formel (X), bevorzugt in einem Anteil von zumindest 0,0001 Gew.-%, bezogen auf die gesamte Zubereitung. In diesen Konzentrationen ist insbesondere für die Verbindung der Formel (X) bereits eine Verringerung der Translokation des AhR-Rezeptors in den Zellkern von Hautzellen zu beobachten, und ferner wird die Induktion AhR-induzierbarer Gene insbesondere CYP1A1, beispielsweise durch polyzyklische aromatische Kohlenwasserstoffe wie TCDD bereits signifikant verringert und eine Hautaufhellung erzielt.

Vorzugsweise beträgt die Konzentration der Verbindung(en) der Formel (II), insbesondere der Formel (X), 0,0005 bis 15 Gew.-%, besonders bevorzugt 0,001 bis 10 Gew.-%, insbesondere 0,01 bis 5 Gew.-%, jeweils bezogen auf die gesamte Zusammensetzung. In diesen Konzentrationen entfalten die Verbindungen der Formel (II) bei Applikation auf die Haut eine starke AhR-Antagonisten-Wirkung, indem sie die Translokation von AhR in den Zellkern verhindern oder verringern und insbesondere eine UVB-induzierte Genexpression, namentlich von CYP1A1, verringern oder verhindern.

Die Zubereitungen können insbesondere kosmetische Zubereitungen sein, wobei Sonnenschutzzubereitungen und After-Sun-Zubereitungen besonders bevorzugt sind.

Die erfindungsgemäßen kosmetischen oder therapeutischen Formulierungen werden mit üblichen, an sich bekannten Verfahren hergestellt, dergestalt, dass die Verbindung(en) der Formel (II), insbesondere der Formel (X), in kosmetische oder dermatologische Formulierungen eingearbeitet werden, die wie üblich zusammengesetzt sind und neben der haut- und haaraufhellenden Wirkung auch zur Behandlung, der Pflege und der Reinigung der Haut oder der Haare dienen können.

Dementsprechend können die erfindungsgemäßen Verbindungen wie in Anspruch 5 beschrieben eingesetzt werden zum Herstellen eines Arzneimittels. Die erfindungsgemäßen Formulierungen liegen vorzugsweise vor als Emulsion, z.B. Emulsion des Typs W/O (Wasser in Öl), O/W (Öl in Wasser), W/O/W (Wasser in Öl in Wasser), O/W/O (Öl in Wasser in Öl), PIT-Emulsion, Pickering-Emulsion, Emulsion mit einem geringen Ölgehalt, Mikro- oder Nanoemulsion, als Lösung z.B. in Öl (fette Öle bzw. Fettsäureester, insbesondere C₆-C₃₂-Fettsäure-C₂-C₃₀-Ester) oder Silikonöl, Dispersion, Suspension, Creme. Lotion oder Milch, je nach Herstellungsverfahren und Inhaltsstoffen, als Gel (inklusive Hydro-, Hydrodispersions-, Oleogel), Spray (z.B. Pumpspray oder Spray mit Treibmittel) oder auch Schaum oder als Tränkungslösung für kosmetische Tücher, als Reinigungsmittel wie z.B. Seife, Syndet, flüssiges Wasch-, Dusch-, und Badepräparat, Badeartikel (Kapsel, Öl, Tablette, Salz, Badesalz, Seife, etc.), Brausezubereitung, als Hautpflegemittel wie z.B. Emulsion (wie oben beschrieben), Salbe, Paste, Gel (wie oben beschrieben), Öl, Toner, Balsam. Serum, Puder (z.B. Gesichtspuder, Körperpuder), als Maske, als Stift, Stick, Roll-On, Pumpe, Aerosol (schäumend, nicht schäumend oder nach-schäumend), als Deodorant und/oder Antitranspirant, Mundwasser und Munddusche, als Fusspflegemittel (inklusive Keratolytikum, Deodorant), als Insekten abwehrendes Mittel, als Sonnenschutzmittel, als Aftersun-Präparat, als Hauttöner, als Rasiermittel, Aftershave Balm, Pre- und Aftershave Lotion, als Haarentfernungsmittel, als Haarpflegemittel wie z.B. Shampoo (inklusive 2-in-1 Shampoo, Anti-Schuppen-Shampoo, Babyshampoo, Shampoo für trockene

Kopfhaut, Shampookonzentrat), Conditioner, Haarkur, Haarwasser, Haarspülung, Frisiercreme, Pomade, Dauerwell- und Fixierungsmittel, Haarfestiger (Spray), Styling-Aid (z.B. Gel oder Wachs), Haarglättungsmittel (Entkräuselungsmittel, Relaxer); als Blondiermittel, Haarfärbemittel wie z.B. temporäre, direktziehende Haarfärbemittel, semipermanente Haarfärbemittel, permanente Haarfärbemittel, Haartönung, Haaraufheller, Haarconditioner, Haarmousse, Augenpflegemittel, Make-Up, Make-Up-Entferner oder Babyartikel.

Es ist auch vorteilhaft, die Verbindung(en) der Formel (II), insbesondere der Formel (X), in verkapselter Form darzureichen, z.B. in Gelatine, Wachsmaterialien, Liposomen oder Cellulosekapseln.

Besonders bevorzugt liegen die erfindungsgemäßen Formulierungen in Form einer Emulsion, insbesondere in Form einer Emulsion des Typs W/O, O/W, W/O/W, O/W/O, PIT-Emulsion, Pickering-Emulsion, Emulsion mit einem geringen Ölgehalt, Mikro- oder Nanoemulsion, eines Gels (inklusive Hydro-, Hydrodispersions-, Oleogel), einer Lösung z.B. in Öl (fette Öle bzw. Fettsäureester, insbesondere C₆-C₃₂-Fettsäure-C₂-C₃₀-Ester)) oder Silikonöl, oder eines Sprays (z.B. Pumpspray oder Spray mit Treibmittel) vor.

Die erfindungsgemäßen (insbesondere topischen) kosmetischen oder therapeutischen Formulierungen, können bevorzugt kosmetische und/oder dermatologische Hilfs- und Zusatzstoffe enthalten, wie sie üblicherweise in solchen Formulierungen verwendet werden, z.B. Kühlwirkstoffe, Sonnenschutzwirkstoffe (insbesondere UV-Filter und/oder UV-filternde Pigmente), Farbstoffe, Pigmente, die eine färbende Wirkung haben, Antioxidantien, Konservierungsmittel, Antiirritantien, weichmachende, anfeuchtende (feuchtigkeitsspendende) und/oder feuchthaltende Substanzen (Feuchthalteregulatoren, z.B. Glycerin oder Harnstoff), Osmolyte, antimikrobielle Wirkstoffe (z.B. antibakterielle Wirkstoffe, Bakterizide, Fungizide), Viruzide, Deodorantien (z.B. schweißhemmende Mittel), oberflächenaktive Substanzen (Tenside), Emulgatoren, Insektrepellents (z.B. DEET, IR 3225, Dragorepel), Pflanzenextrakte, entzündungshemmende Wirkstoffe (entzündungshemmende Mittel), die Wundheilung beschleunigende Stoffe (z.B. Chitin oder Chitosan und dessen Derivate), gelbildende Mittel, filmbildende Substanzen (Filmbildner, z.B. Polyvinylpyrrolidone oder Chitosan oder dessen Derivate), Fixateure, hautglättende Wirkstoffe, faltenreduzierende Wirkstoffe wie beta-Glucan aus Hafer oder Brombeerblatt- oder Sojaextrakt, Vitamine (z.B. Vitamin C und Derivate, Tocopherole und Derivate, Vitamin A und Derivate), 2-Hydroxycarbonsäuren (z.B. Zitronensäure, Apfelsäure, L-, D- oder DL-Milchsäure), Hautfärbungsmittel (z.B. Walnussextrakte oder Dihydroxyaceton), hautpflegende und -reparierende Mittel (z.B. Cholesterol, Ceramide, Pseudoceramide, Creatin und Creatinester), hautberuhigende Mittel, rückfettende Mittel, optisch aufhellende Mittel, Gleitmittel, Glanzmittel, Fette, Öle, gesättigte Fettsäuren und deren Salze, ein- oder mehrfach ungesättigte Fettsäuren und deren Salze, alpha-Hydroxysäuren, Polyhydroxyfettsäuren oder deren Derivate (z.B. Linolsäure, alpha-Linolensäure, gamma-Linolensäure oder Arachidonsäure und deren jeweiligen natürlichen oder synthetischen Ester), Phospholipide, Wachse oder andere übliche Bestandteile einer kosmetischen oder dermatologischen Formulierung wie Alkohole, Alkandiole, Polyole, Polymere, Elektrolyte, organische Lösungsmittel, Silikone, Silikonderivate oder Chelatbildner (z.B. Ethylendiamintetraessigsäure und Derivate), Antischuppenwirkstoffe (Antischuppen-Mittel, z.B. Climbazol, Ketoconazol, Piroctonoleamin, Zink-Pyrithion), Haarpflegemittel, Haarverformungsmittel, Haarglättungsmittel, Enthaarungsmittel, Parfüme, ätherische Öle, Schaumbildner, Schaumstabilisatoren, Schaumbooster, Substanzen zum Verhindern des Schäumens, Verdickungsmittel, Binder, Pflanzenteile (z.B. Fasern) und Pflanzenextrakte (z. B. Arnika, Aloe, Bartflechte, Efeu, Brennnessel, Ginseng, Henna, Kamille, Ringelblume, Rosmarin, Salbei, Schachtelhalm oder Thymian), tierische Extrakte wie z. B. Geleé royale, Propolis, Proteine, Proteinhydrolysate, Hefeextrakte, Hopfen- und Weizenextrakte, Peptide oder Thymusextrakte, Abrasiva (abschleifende Mittel), Puffer, Enzyme.

Bestandsteile (Hilfs- und Zusatzstoffe), mit denen die Verbindung(en) der Formel (II), insbesondere der Formel (X), kombiniert werden können, sind besonders bevorzugt:
Abrasiva, Antischuppen-Mittel, entzündungshemmende Mittel, Antioxidantien, schweißhemmende Mittel, Binder, Puffer, Chelatbildner, Enthaarungsmittel, oberflächenaktive Substanzen, Emulgatoren, Enzyme, ätherische Öle, Pflanzenextrakte, Fasern, Filmbildner, Fixateure, Schaumbildner, Schaumstabilisatoren, Substanzen zum Verhindern des Schäumens, Schaumbooster, gelbildende Mittel, Haarpflegemittel, Haarverformungsmittel, Haarglättungsmittel, Wirkstoffe zur Haut- und Haaraufhellung, feuchtigkeitsspendende Mittel, feuchthaltende Substanzen, Insektrepellents, optisch aufhellende Mittel, Gleitmittel, Glanzmittel, Polymere, Proteine, rückfettende Mittel, hautberuhigende Mittel, hautglättende Wirkstoffe, faltenreduzierende Wirkstoffe, Sonnenschutzwirkstoffe, Vitamine, Öle, Wachse, Fette, Phospholipide, gesättigte Fettsäuren und deren Salze, ein- oder mehrfach ungesättigte Fettsäuren und deren Salze, alpha-Hydroxysäuren, Polyhydroxyfettsäuren, Polyole, Alkandiole, Silikone oder Silikonderivate.

Hilfs- und Zusatzstoffe können in Mengen von 5 bis 99 Gew.-%, vorzugsweise 10 bis 80 Gew.-%, bezogen auf das Gesamtgewicht der Formulierung, enthalten sein. Die jeweils einzusetzenden Mengen an kosmetischen oder dermatologischen Hilfs- und Zusatzstoffen und Parfüm können in Abhängigkeit von der Art des jeweiligen Produkts vom Fachmann durch einfaches Ausprobieren leicht ermittelt werden.

Ferner können die Formulierungen Wasser in einer Menge bis zu 99,99 Gew.-%, vorzugsweise 5 bis 80 Gew.-%, bezogen auf das Gesamtgewicht der Formulierung, aufweisen.

Erfindungsgemäße kosmetische oder therapeutische Formulierungen sind vorzugsweise solche Formulierungen,
die ausgewählt sind aus der Gruppe bestehend aus Emulsion, Lösung, Dispersion, Suspension, Creme, Lotion, Milch, Gel, Spray, Schaum, Tränkungslösung für kosmetische Tücher, Reinigungsmittel, Seife, Syndet, Waschpräparat, Duschpräparat, Badepräparat, Badeartikel, Brausezubereitung, Hautpflegemittel, Salbe, Paste, Öl, Toner, Balsam, Serum, Puder, Maske, Stift, Stick, Roll-On, Pumpe, Aerosol, Deodorant, Antitranspirant, Mundwasser, Munddusche, Fusspflegemittel, Insekten abwehrendes Mittel, Sonnenschutzmittel, Selbstbräunungsmittel, Aftersun-Präparat, Hauttöner, Rasiermittel, Aftershave Balm, Preshave Lotion, Aftershave Lotion, Haarentfernungsmittel, Haarpflegemittel, Shampoo, Conditioner, Haarkur, Haarwasser, Haarspülung, Frisiercreme, Pomade, Dauerwellmittel, Fixierungsmittel, Haarfestiger, Styling-Aid, Haarglättungsmittel, Blondiermittel, Haarfärbemittel, Haartönung, Haaraufheller, Haarconditioner, Haarmousse, Augenpflege, Make-Up, Make-Up-Entferner und Babyartikel, und/oder
die neben Verbindung(en) der Formel (II), insbesondere der Formel (X), einen oder mehrere Hilfs- und Zusatzstoffe ausgewählt aus der Gruppe bestehend aus
Abrasiva, Antischuppen-Mittel, entzündungshemmende Mittel, Antioxidantien, schweißhemmende Mittel, Binder, Puffer, Chelatbildner, Enthaarungsmittel, oberflächenaktive Substanzen, Emulgatoren, Enzyme, ätherische Öle, Pflanzenextrakte, Fasern, Filmbildner, Fixateure, Schaumbildner, Schaumstabilisatoren, Substanzen zum Verhindern des Schäumens, Schaumbooster, gelbildende Mittel, Haarpflegemittel, Haarverformungsmittel, Haarglättungsmittel, Wirkstoffe zur Haut- und Haaraufhellung, feuchtigkeitsspendende Mittel, feuchthaltende Substanzen, Insektrepelfents, optisch aufhellende Mittel, Gleitmittel, Glanzmittel, Polymere, Proteine, rückfettende Mittel, hautberuhigende Mittel, hautglättende Wirkstoffe, faltenreduzierende Wirkstoffe, Sonnenschutzwirkstoffe, Vitamine, Öle, Wachse, Fette, Phospholipide, gesättigte Fettsäuren und deren Salze, ein- oder mehrfach ungesättigte Fettsäuren und deren Salze, alpha-Hydroxysäuren, Polyhydroxyfettsäuren, Polyole, Alkandiole, Silikone und Silikonderivate enthalten,
und/oder
die vorgesehen sind für eine Applikation auf das Haar und/oder die Haut. Zur Anwendung werden die Verbindung(en) der Formel (II), insbesondere der Formel (X), enthaltenden Formulierungen in der Regel in der für Kosmetika und Dermatika üblichen Weise auf die Haut und/oder die Haare in ausreichender Menge aufgebracht. Besondere Vorteile bieten dabei solche kosmetischen, dermatologischen und/oder therapeutischen erfindungsgemäßen Formulierungen, die zusätzlich einen oder mehrere Sonnenschutzfilter (UV-Absorber, UV-Filter) umfassen.

In seltenen Fällen kann es zu Verfärbungen und/oder Instabilitäten bei erfindungsgemäße bzw. erfindungsgemäß einzusetzende Verbindungen der Formel (II) enthaltenden Formulierungen kommen, insbesondere wenn diese in wässrigalkoholischen oder rein alkoholischen Lösungen vorliegen. Überraschenderweise wurde nunmehr gefunden, dass UV-Filter die Stabilität der Verbindungen der Formel (II) in erfindungsgemäßen Formulierungen verbessern können. Insbesondere können UV-Filter eine durch Sonnenlicht oder anderes Licht hervorgerufene Verfärbung der Verbindungen der Formel (II) verhindern oder verlangsamen. Beides ist insbesondere in kosmetischen Formulierungen wichtig. Erfindungsgemäß werden UV-Filter daher verwendet zum Stabilisieren der Verbindungen der Formel (II), insbesondere indem ein oder mehrere UV-Filter in einer zum Stabilisieren der Verbindungen der Formel (II) ausreichenden Menge in einer erfindungsgemäßen Formulierung eingesetzt werden, vorzugsweise unter Verwendung der weiter unten (bevorzugt) genannten UV-Filter. In diesem Zusammenhang betrifft ein weiterer Aspekt der Erfindung die kosmetische oder therapeutische Verwendung einer oder mehrerer Verbindungen der Formel (II) zur Aufhellung von Haut und/oder Haar in Gegenwart einer die Verbindung oder die Verbindungen der Formel (II), insbesondere der Formel (X) stabilisierenden Menge eines oder mehrerer UV-Filter, wobei sämtliche weiter oben gemachten Angaben zur Auswahl des Substituenten natürlich auch insoweit zutreffen. Für die Zwecke der Stabilisierung liegt die Gesamtmenge an UV-Filtern vorzugsweise im Bereich von 0,1 bis 2 Gew.-%, insbesondere 0,2 bis 1 Gew.-%, bezogen auf das Gesamtgewicht der Formulierung.

Das Verhältnis des Gesamtgewichtsanteils an UV-Filtern zum Gesamtgewichtsanteil der erfindungsgemäßen bzw. erfindungsgemäß einzusetzenden Verbindungen der Formel (II) liegt vorzugsweise im Bereich von 100:1 bis 1:100, besonders bevorzugt im Bereich von 10:1 bis 1:10, ganz besonders bevorzugt im Bereich von 5:1 bis 1:5.

Erfindungsgemäße Formulierungen enthaltend einen oder mehrere UV-Filter (Sonnenschutzfiilter, UV-Absorber) weisen vorzugsweise einen Gesamtanteil an UV-Filtern auf im Bereich von 0,1 bis 30 Gew.-%, besonders bevorzugt im Bereich von 0,2 bis 20 Gew.-%, ganz besonders bevorzugt im Bereich von 0,5 bis 15 Gew.-%, bezogen auf das Gesamtgewicht der Formulierung. Besonders bevorzugt enthalten die erfindungsgemäßen Formulierungen einen oder mehre UVB-Filter, insbesondere der unten angegebenen Arten. Es hat sich gezeigt, dass die erfindungsgemäßen Substanzen der Formel (II) und insbesondere der Formeln (X) mit UVB-Filtern vorteilhaft zusammenwirken zum Verhindern von UVB-induzierten Hautschäden, Hautveränderungen und Hautkrebs.

Besonders bevorzugt werden die erfindungsgemäßen bzw. erfindungsgemäß einzusetzenden Verbindungen der Formel (II) und insbesondere der Formel (X) mit wasserlöslichen UV-Filtern kombiniert, in einer bevorzugten Ausgestaltung mit Phenylen-bis-benzimidazyl-tetrasulfonsäure-dinatriumsalz (Neo Heliopan®AP) und/oder 2-Phenylbenzimidazolsulfonsäure (Neo Heliopan®Hydro).

In einer weiteren bevorzugten Ausgestaltung enthält eine erfindungsgemäße Formulierung eine Gesamtmenge an Sonnenschutzwirkstoffen, d.h. insbesondere UV-Filtern und/oder anorganischen Pigmenten (UV-filternden Pigmenten), so dass die erfindungsgemäße Formulierung einen Lichtschutzfaktor von größer oder gleich 2 (bevorzugt größer oder gleich 5) aufweist. Solche erfindungsgemäßen Formulierungen sind besonders zum Schutz von Haut und Haar geeignet.

Erfindungsgemäße Formulierungen umfassend zusätzlich einen oder mehrere Sonnenschutzfilter (UV-Fllter, UV-Absorber) können dabei in verschiedenen Formen vorliegen, wie sie z.B. üblicherweise für Sonnenschutzformulierungen eingesetzt werden. So können sie z.B. in Form einer Emulsion vom Typ Öl-in-Wasser (O/W), eines Gels, einer Hydrodispersion, oder auch eines Aerosols vorliegen.

Vorteilhaft enthalten die erfindungsgemäßen Formulierungen mindestens einen UV-A-Filter und/oder mindestens einen UV-B-Filter und/oder einen Breitband-Filter und/oder mindestens ein anorganisches Pigment. Erfindungsgemäße Formulierungen enthalten bevorzugt mindestens einen UV-B-Filter oder einen Breitband-Filter, weiter besonders bevorzugt mindestens einen UV-A-Filter und mindestens einen UV-B-Filter.

Geeignete UV-Filter sind z.B. organische UV-Absorber aus der Klasse der 4-Aminobenzoesäure und Derivate, Salicylsäure-Derivate, Benzophenon-Derivate, Dibenzoylmethan-Derivate, Diphenylacrylate, 3-Imidazol-4-yl-acrylsäure und deren Ester, Benzofuran-Derivate, Benzylidenmalonat-Derivate, polymere UV-Absorber, enthaltend einen oder mehrere silizium-organische Reste, Zimtsäure-Derivate, Campher-Derivate, Trianilino-s-Triazin-Derivate, 2-Hydroxyphenylbenzotriazol-Derivate, Phenylbenzimidazolsulfonsäure Derivate und deren Salze, Anthranilsäurementhylester, Benzotriazol-Derivate, Indol-Derivate.

Die nachfolgend genannten UV-Filter, die im Sinne der vorliegenden Erfindung eingesetzt werden können, sind bevorzugt, jedoch selbstverständlich' nicht limitierend.

### Vorteilhafte UV-Filter sind

UV-B-Filter wie z.B.:
- p-Aminobenzoesäure
- p-Aminobenzoesäureethylester (25 Mol) ethoxyliert (INCI-Name: PEG-25 PABA)
- p-Dimethylaminobenzoesäure-2-ethylhexylester
- p-Aminobenzoesäureethylester (2 Mol) N-propoxyliert
- p-Aminobenzoesäureglycerinester
- Salicylsäure-homomenthylester (Homosalate) (Neo Heliopan^{®}HMS)
- Salicylsäure-2-ethylhexylester (Neo Heliopan^{®}OS)
- Triethanolaminsalicylat
- 4-Isopropylbenzylsalicylat
- Anthranilsäurementhylester (Neo Heliopan^{®}MA)
- Diisopropylzimtsäureethylester
- p-Methoxyzimtsäure-2-ethylhexylester (Neo Heliopan^{®}AV)
- Diisopropylzimtsäuremethylester
- p-Methoxyzimtsäureisoamylester (Neo. Hellopan^{®}E 1000)
- p-Methoxyzimtsäure-diethanolaminsalz
- p-Methoxyzimtsäure-isopropylester
- 2-Phenylbenzimidazolsulfonsäure und Salze (Neo Heliopan^{®}Hydro)
- 3-(4'-Trimethylammonium)-benzyliden-bornan-2-on-methylsulfat
- beta-Imidazol-4(5)-acrylsäure (Urocaninsäure)
- 3-(4'-Sulfo)benzyliden-bornan-2-on und Salze
- 3-(4'-Methylbenzyliden)-D,L-campher (Neo Heliopan^{®}MBC)
- 3-Benzyliden-D,L-campher
- N-[(2 und 4)-[2-(oxoborn-3-yliden)methyl]benzyl]-acrylamid-Polymer
- 4,4'-[(6-[4-(1,1-Dimethyl)-aminocarbonyl)-phenylamino]-1,3,5-triazin-2,4-diyl)diimino]-bis-(benzoesäure-2-ethylhexylester) (Uvasorb^{®}HEB)
- Benzylidenmalonat-Polysiloxan (Parsol^{®}SLX)
- Glyceryl-ethylhexanoat-dimethoxycinnamat
- Dipropylenglykolsalicylat
- Tris(2-etythexyl)-4,4*'*,4*''*-(1,3,5-triazin-2,4,6-triyltriimino)tribenzoat (= 2,4,6-Trianilino-(p-carbo-2'-ethylhexyl-1'-oxy)-1,3,5-triazin) (Uvinul^{®}T150)

Breitband-Filter wie z.B.
- 2-Ethylhexyl-2-cyano-3,3-diphenylacrylat (Neo Heliopan^{®}303)
- Ethyl-2-cyano-3,3'-diphenylacrylat
- 2-Hydroxy-4-methoxybenzophenon (Neo Heliopan^{®}BB)
- 2-Hydroxy-4-methoxybenzophenon-5-sulfonsäure
- Dihydroxy-4-methoxybenzophenon
- 2,4-Dihydroxybenzophenon
- Tetrahydroxybenzophenon
- 2,2'-Dihydroxy-4,4'-dimethoxybenzophenon
- 2-Hydroxy-4-n-octoxybenzophenon
- 2-Hydroxy-4-methoxy-4'-methylbenzophenon
- Natrium-hydroxymethoxybenzophenon-sulfonat
- Dinatrium-2,2'-dihydroxy-4,4'-dimethoxy-5,5'-disulfo-benzophenon
- Phenol, 2-(2H-benzotriazol-2-yl)-4-methyl-6-(2-methyl-3(1,3,3,3-tetramethyl-1-(trimethylsilyl)-oxy)-disiloxyanyl)-propyl), (Mexoryl®XL)
- 2,2'-Methylen-bis-(6-(2H-benztriazol-2-yl)-4-1,1,3,3-tetramethylbutyl)-phenol), (Tinosorb^{®}M)
- 2,4-bis-[4-(2-ethylhexyloxy)-2-hydroxyphenyl]-1,3,5-triazin
- 2,4-Bis-[{(4-(2-Ethyl-hexyloxy)-2-hydroxy}-phenyl]-6-(4-methoxyphenyl)-1,3,5-triazin, (Tinosorb^{®}S)
- 2,4-Bis-[{(4-(3-sulfonato)-2-hydroxy-propyloxy)-2-hydroxy}-phenyl]-6-(4-methoxyphenyl)-1,3,5-triazin-Natriumsalz
- 2,4-Bis-[{(3-(2-Propyloxy)-2-hydroxy-propyloxy)-2-hydroxy}-phenyl]-6-(4-methoxy-phenyl)-1,3,5-triazin
- 2,4-Bis-[{4-(2-Ethyl-hexyloxy)-2-hydroxy}-phenyl]-6-[4-(2-methoxyethyl-carbonyl)- phenylamino]-1,3,5-triazin
- 2,4-Bis-[{4-(3-(2-propyloxy)-2-hydroxy-propyloxy)-2-hydroxy}-phenyl]-6-[4-(2-ethylcarboxyl)-phenylamino]-1,3,5-triazin
- 2,4-Bis-[{4-(2-Ethyl-hexyloxy)-2-hydroxy}-phenyl]-6-(1-methyl-pyrrol-2-yl-)-1,3,5-triazin
- 2,4-Bis-[{4-tris-(trimethylsiloxy-silylpropyloxy)-2-hydroxy}-phenyl]-6-(4-methoxyphenyl)-1,3,5-triazin
- 2,4-Bis-[{4-2"-Methylpropenyloxy)-2-hydroxy}-phenyl]-6-(4-methoxyphenyl)-1,3,5-triazin
- 2,4-Bis-[{4-(1',1',1',3'5',5',5'-Heptamethylsiloxy-2"-methyl-propyloxy)-2-hydroxy}-phenyl]-6-(4-methoxyphenyl)-1,3,5-triazin

UV-A-Filter wie z.B.
- 4-Isopropyldibenzoylmethan
- Terephthalyliden-dibomansulfonsäure und Salze (Mexoryl^{®}SX)
- 4-t-Butyl-4'-methoxy-dibenzoylmethan (Avobenzon) / (Neo Heliopan^{®}357)
- Phenylen-bis-benzimidazyl-tetrasulfonsäure-dinatriumsalz (Neo Heliopan^{®}AP)
- 2,2'-(1,4-Phenylen)-bis-(1H-benzimidazol-4,6-disulfonsäure), Mononatriumsalz
- 2-(4-Diethylamino-2-hydroxybenzoyl)-benzoesäure-hexylester (Uvinul^{®} A Plus)
- Indanylidenverbindungen gemäß DE 100 55 940 (= WO 02/38537)

Besonders zur Kombination geeignete UV-Filter sind dabei
- p-Aminobenzoesäure
- 3-(4'-Trimethylammonium)-benzyliden-bornan-2-on-methylsulfat
- Salicylsäure-homomenthylester (Neo Heliopan^{®}HMS)
- 2-Hydroxy-4-methoxy-benzophenon (Neo Heliopan^{®}BB)
- 2-Phenylbenzimidazolsulfonsäure (Neo Heliopan^{®}Hydro)
- Terephthalyliden-dibornansulfonsäure und Salze (Mexoryl^{®}SX)
- 4-tert.-Butyl-4'-methoxydibenzoylmethan (Neo Heliopan^{®}357)
- 3-(4'-Sulfo)benzyliden-bornan-2-on und Salze
- 2-Ethylhexyl-2-cyano-3,3-diphenylacrylat (Neo Heliopan^{®}303)
- N-[(2 und 4)-[2-(oxoborn-3-yliden)methyl]benzyl]-acrylamid-Polymer
- p-Methoxyzimtsäure-2-ethylhexylester (Neo Heliopan^{®}AV)
- p-Aminobenzoesäureethylester (25 Mol) ethoxyliert (INCI-Name: PEG-25 PABA)
- p-Methoxyzimtsäure-isoamylester (Neo Heliopan^{®}E1000)
- 2,4,6-Trianilino-(p-carbo-2'-ethylhexyl-1'-oxy)-1,3,5-triazin (Uvinul^{®}T150)
- Phenol, 2-(2H-benzotriazol-2-yl)-4-methyl-6-(2-methyl-3(1,3,3,3-tetramethyl-1-(trimethylsilyl)-oxy)-disiloxyanyl)-propyl), (Mexoryl^{®}XL)
- 4,4'-[(6-[4-(1,1-Dimethyl)-aminocarbanyl)-phenylamino]-1,3,5-triazin-2,4-diyl)-diimino]-bis-(benzoesäure-2-ethylhexylester), (UvasorbHEB)
- 3-(4'-Methylbenzyliden)-D,L-campher (Neo Helipan^{®}MBC)
- 3-Benzylidencampher
- Salicylsäure-2-ethylhexylester (Neo Helipan^{®}OS)
- 4-Dimethylaminobenzoesäure-2-ethylhexylester (Padimate O)
- Hydroxy-4-methoxy-benzophenon-5-sulfonsäure und Na-Salz
- 2,2'-Methylen-bis-(6-(2H-benztriazol-2-yl)-4-1,1,3,3-tetramethylbutyl)-phenol), (Tinosorb^{®}M)
- Phenylen-bis-benzimidazyl-tetrasulfonsäure-dinatriumsalz (Neo Heliopan^{®}AP)
- 2,4-Bis-[{(4-(2-Ethyl-hexyloxy)-2-hydroxy}-phenyl]-6-(4-methoxyphenyl)-1,3,5-triazin, (Tinosorb^{®}S)
- Benzylidenmalonat-Polysiloxan (Parsol^{®}SLX)
- Menthylanthranilat (Neo Heliopan^{®}MA)
- 2-(4-Diethylamino-2-hydroxybenzoyl)-benzoesäure-hexylester (Uvinul^{®} A Plus)
- Indanylidenverbindungen gemäß DE 100 55 940 (= WO 02/38537)

Es können darüber hinaus partikuläre UV-Filter oder anorganische Pigmente eingesetzt werden, die gegebenenfalls hydrophobisiert sein können, wie die Oxide des Titans (TiO₂), Zinks (ZnO), Eisens (Fe₂O₃), Zirkoniums (ZrO₂), Siliziums (SiO₂), Mangans (z.B. MnO), Aluminiums (Al₂O₃), Cers (z.B. Ce₂O₃) und/oder deren Mischungen.

Erfindungsgemäße, insbesondere dermatologische Formulierungen können ferner vorteilhaft Farbstoffe und/oder Farbpigmente enthalten, insbesondere wenn sie im Bereich der dekorativen Kosmetik verwendet werden sollen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z.B. Fe₂O₃ Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett.

Einzelne zur Verwendung im Rahmen der vorliegenden Erfindung bevorzugte Kühlwirkstoffe sind nachfolgend aufgelistet. Der Fachmann kann die nachfolgende Liste um eine Vielzahl weiterer Kühlwirkstoffe ergänzen; die aufgelisteten Kühlwirkstoffe können auch in Kombination miteinander eingesetzt werden: L-Menthol, D-Menthol, racemisches Menthol, Menthonglycerinacetal (Handelsname: Frescolat^{®}MGA), Menthyllactat (Handelsname: Frescolat^{®}ML, vorzugsweise handelt es sich bei Menthyllactat um L-Menthyllactat, insbesondere L-Menthyl-L-lactat), substituierte Menthyl-3-carbonsäureamide (z.B. Menthyl-3-carbonsäure-N-ethylamid), 2-Isopropyl-N-2,3-trimethylbutanamid, substituierte Cyclohexancarbonsäureamide, 3-Menthoxypropan-1,2-diol, 2-Hydroxyethylmenthylcarbonat, 2-Hydroxypropylmenthylcarbonat, N-Acetyiglycinmenthylester, Isopulegol, Menthylhydroxycarbonsäureester (z.B. Menthyl-3-hydroxybutyrat), Monomenthylsuccinat, 2-Mercaptocyclodecanon, Menthyl-2-pyrrolidin-5-oncarboxylat, 2,3-Dihydroxy-p-menthan, 3,3,5-trimethylcyclohexanonglycerinketal, 3-Menthyl-3,6-di- und -trioxaalkanoate, 3-Menthyl methoxyacetat, Icilin.

Bevorzugte Kühlwirkstoffe sind: L-Menthol, D-Menthol, racemisches Menthol, Menthonglycerinacetal (Handelsname: Frescolat^{®}MGA), Menthyllactat. (vorzugweise L-Menthyllactat, insbesondere L-Menthyl-I-lactat, Handelsname: Frescolat^{®}ML), substituierte Menthyl-3-carbonsäureamide (z.B. Menthyl-3-carbonsäure-N-ethylamid), 2-Isopropyl-N-2,3-trimethylbutanamid, substituierte Cyclohexancarbonsäureamide, 3-Menthoxypropan-1,2-diol, 2-Hydroxyethylmenthylcarbonat, 2-Hydroxypropylmenthylcarbonat, Isopulegol.

Besonders bevorzugte Kühlwirkstoffe sind: L-Menthol, racemisches Menthol, Menthonglycerinacetal (Handelsname: Frescolat^{®}MGA), Menthyllactat (vorzugsweise L-Menthyllactat, insbesondere L-Menthyl-L-lactat, Handelsname: Frescolat^{®}ML), 3-Menthoxypropan-1,2-diol, 2-Hydroxyethylmenthylcarbonat, 2-Hydroxypropylmenthylcarbonat.

Ganz besonders bevorzugte Kühlwirkstoffe sind: L-Menthol, Menthonglycerinacetal (Handelsname: Frescolat^{®}MGA), Menthyllactat (vorzugsweise L-Menthyllactat, insbesondere L-Menthyl-L-lactat, Handelsname: Frescolat^{®}ML).

Die Einsatzkonzentration der einzusetzenden Kühlwirkstoffe liegt je nach Substanz vorzugsweise im Konzentrationsbereich von 0,01 bis 20 Gew.-% und besonders bevorzugt im Konzentrationsbereich von 0,1 bis 5 Gew.-%, bezogen auf die Gesamtmasse der fertigen (anwendungsbereiten), vorzugsweise topischen, kosmetischen oder therapeutischen (pharmazeutischen) Formulierung.

Bevorzugt können die erfindungsgemäßen Formulierungen für kosmetische (z.B. dermatologische) und/oder therapeutische Anwendungen geeignete weitere Wirkstoffe zur Haut- und Haaraufhellung enthalten. Vorteilhafte haut- und haaraufhellende Wirkstoffe sind insoweit Kojisäure (5-Hydroxy-2-hydroxymethyl-4-pyranon), Kojisäurederivate wie z.B. Kojisäuredipalmitat, Arbutin, Ascorbinsäure, Ascorbinsäurederivate, Hydrochinon, Hydrochinonderivate, Resorcin, schwefelhaltige Moleküle wie z.B. Glutathion oder Cystein, alpha-Hydroxysäuren (z.B. Zitronensäure, Milchsäure, Apfelsäure) und deren Derivate, N-Acetyl-Tyrosin und -Derivate, Undecenoylphenylalanin, Gluconsäure, 4-Alkylresorcine, 4-(1-Phenylethyl)1,3-dihydroxybenzol, Chromon-Derivate wie Aloesin, Flavonoide, Thymolderivate, 1-Aminoethylphosphinsäure, Thioharnstoffderivate, Ellagsäure, Nicotinamid (Niacinamid), Zinksalze wie z.B. Zinkchlorid oder -gluconat, Thujaplicin und -Derivate, Triterpene wie Maslinsäure, Sterole wie Ergosterol, Benzofuranone wie Senkyunolid, Vinyl- und Ethylgujacol, Dionsäuren wie Octodecendionsäure und Azelainsäure, Inhibitoren der Stickstoffoxid-Synthese wie z.B. L-Nitroarginin und dessen Derivate, 2,7-Dinitroindazol oder Thiocitrullin, Metallchelatoren (z.B. alpha-Hydroxyfettsäuren, Palmitinsäure, Phytinsäure, Lactoferrin, Huminsäure, Gallensäure, Gallenextrakte, Bilirubin, Biliverdin, EDTA, EGTA und deren Derivate), Retinoide, Sojamilch und -extrakt, Serin-Protease-Inhibitoren oder Liponsäure oder andere synthetische oder natürliche Wirkstoffe zur Haut- und Haaraufhellung, wobei letztere auch in Form eines Extrakts aus Pflanzen, wie z.B. Bearberry-Extrakt, Reis-Extrakt, Papaya-Extrakt, Süßholzwurzel-Extrakt (Licorice Extrakt) oder daraus angereicherte Bestandteile wie Glabridin oder Licochalkon A, Artocarpus-Extrakt, Extrakt von Rumex- und Ramulus-Arten, Extrakte aus Kieferarten (Pinus) und Extrakte aus Vitis-Arten oder daraus angereicherte Stilbenderivate, Extrakt aus Saxifraga, Maulbeer, Scutelleria oder/und Trauben verwendet werden.

Die Menge der vorgenannten beispielhaften weiteren Wirkstoffe zur Haut- und Haaraufhellung (eine oder mehrere Verbindungen) in den erfindungsgemäßen Formulierungen beträgt dann vorzugsweise 0,005 bis 30 Gew.-%, bevorzugt 0,01 bis 20 Gew.-%, besonders bevorzugt 0,01 bis 5 Gew.-%, bezogen auf das Gesamtgewicht der Formulierung.

Erfindungsgemäße Formulierungen (insbesondere, wenn die Anwendung im Gesichtsbereich vorgesehen ist), kann es vorteilhaft sein als Farbstoff eine oder mehrere Substanzen aus der folgenden Gruppe zu wählen: 2,4-Dihydroxyazobenzol, 1-(2'Chlor-4'-nitro-1'-phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (Colour Index Nummer (CIN): 77491 (rot) und 77499 (schwarz)), Eisenoxidhydrat (CIN: 77492), Manganammoniumdiphosphat und Titandioxid.

Ferner vorteilhaft sind öllösliche Naturfarbstoffe, wie z.B. Paprikaextrakte, ß-Carotin oder Cochenille.

Vorteilhaft im Sinne der vorliegenden Erfindung sind ferner dermatologische Formulierungen mit einem Gehalt an Perlglanzpigmenten. Bevorzugt sind insbesondere die im folgenden aufgelisteten Arten von Perlglanzpgimenten:
1. Natürliche Perlglanzpigmente, wie z.B.
   - "Fischsilber" (Guanin/Hypoxanthin-Mischkristalle aus Fischschuppen) und
   - "Perlmutt" (vermahlene Muschelschalen)
2. Monokristalline Perlglanzpigmente wie z.B. Bismuthoxychlorid (BiOCl)
3. Schicht-Substrat Pigmente: z.B. Glimmer / metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z.B. das unter der CIN 77163 aufgelistete Glanzpigment.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z.B. Silica und dergleichen mehr. Vorteilhaft sind z.B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z.B. unter dem Handelsnamen Sicopearl Kupfer 1000 bei der Firma BASF erhältlich. Besonders vorteilhaft sind ferner auch Effektpigmente, welche unter der Handelsbezeichnung Metasomes Standard / Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den CIN 19140, 77007, 77289,77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z.B. 0,1 Gew.-% bis 30 Gew.%, vorzugsweise von 0,5 bis 15 Gew.-%, insbesondere von 1,0 bis 10 Gew.-% gewählt, jeweils bezogen auf das Gesamtgewicht der (kosmetischen) Formulierungen.

Die erfindungsgemäßen Formulierungen können auch (zusätzliche) Antioxidantien oder Konservierungsmittel enthalten. Als Antioxidantien oder Konservierungsmittel können alle für kosmetische (z.B. dermatologische) und/oder therapeutische Anwendungen geeigneten oder gebräuchlichen Antioxidantien verwendet werden.

Antioxidantien im Sinne der Erfindung sind alle Substanzen, die die Menge an freien Radikalen in Zellen und Geweben senken. Vorteilhaft werden Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. alpha-Carotin, beta-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, gamma-Linoleyl-, Cholesteryl-, Glyceryl-, und Oligoglycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. alpha-Hydroxyfettsäuren, Palmitinsäure, Phytinsäure, Lactoferrin, alpha-Hydroxysäuren (z.B. Zitronensäure, Milchsäure, Apfelsäure), Huminsäure, Gallensäure, Gallenextrakte, Tannine, Bilirubin, Biliverdin, EDTA, EGTA und deren Derivate), ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat, Ascorbylglucosid), Tocopherole und Derivate (z.B. Vitamin E-acetat), Vitamin A und Derivate (Vitamin A-palmitat) sowie Koniferylbenzoat des Benzoeharzes, Rutinsäure und deren Derivate, Flavonoide und deren glykosylierte Vorstufen, insbesondere Quercetin und dessen Derivate z.B. alpha-Glucosylrutin, Rosmarinsäure, Carnosol, Carnosolsäure, Resveratrol, Kaffeesäure und deren Derivate, Sinapinsäure und deren Derivate, Ferulasäure und deren Derivate, Curcuminoide, Chlorogensäure und deren Derivate, Retinoide, Ursolsäure, Levulinsäure, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Superoxid-Dismutase, Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe oder antioxidativ wirksame Extrakte oder Fraktionen von Pflanzen wie z.B. Grüntee, Rooibos, Honeybush, Traube, Rosmarin, Salbei, Melisse, Thymian, Lavendel, Olive, Hafer, Kakao, Ginkgo, Ginseng, Süßholz, Geißblatt, Sophora, Pueraria, Pinus, Citrus, Phyllanthus emblica oder Johanniskraut, Traubenkernen, Weizenkeimen, Phyllantus emblica.

Weiterhin sind geeignet Coenzyme, wie z.B. Coenzym Q10, Plastochinon, Menachinon, Ubichinole 1-10, Ubichinone 1-10 oder Derivate dieser Stoffe.

Die Menge der Antioxidantien (eine oder mehrere Verbindungen) in den erfindungsgemäßen Formulierungen beträgt vorzugsweise 0,01 bis 20 Gew.-%, besonders bevorzugt 0,05 bis 10 Gew.-%, insbesondere 0,2 bis 5 Gel.-%, bezogen auf das Gesamtgewicht der Formulierung.

Sofern Vitamin E und/oder dessen Derivate das oder die Antioxidantien darstellen, ist es vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist es vorteilhaft, deren jeweilige Konzentrationen aus dem Bereich von 0,001 bis 10 Gew.-%, bezogen auf das Gesamtgewicht der Formulierung, zu wählen.

Erfindungsgemäße Formulierungen können auch Konservierungsmittel enthalten. Als Konservierungsmittel können eingesetzt werden: alle für kosmetische (z.B. dermatologische) und/oder therapeutische Anwendungen geeigneten oder gebräuchlichen Antioxidantien, klassische Konservierungsmittel (z.B. Formaldehyd, Glutardialdehyd, Parabene (z.B. Methyl-, Ethyl-, Propyl- und Butylparaben), Dibromdicyanobutan, Imidazolidinylharnstoffe ("Germall"), Isothiazolinone ("Kathon"), Methylchlorthiazolidin, Methylthiazolidin, organische Säuren (z.B. Benzoesäure, Sorbinsäure, Salicylsäure) sowie deren Salze und Ester, Propoinsäure und Ameisensäure und deren Salze, Glycole z.B. Propylenglycol. 1,2-Dihydroxyalkane), pflanzliche Konservierungshelfer wie z.B. Lantadin A, Caryophyllen, Hesperidin, Diosmin, Phellandren, Pigenin, Quercetin, Hypericin, Aucubin, Diosgenin, Plumbagin, Corlilagin etc.

Weiterhin kann der Einsatz von Antiirritantien in erfindungsgemäßen Formulierungen vorteilhaft sein. Antiirritantien können hierbei alle für kosmetische (z.B. dermatologische) und/oder therapeutische Anwendungen geeigneten oder gebräuchlichen entzündungshemmenden bzw. rötungs- und juckreizlindernden Wirkstoffe sein. Bevorzugt werden alle Substanzen, die die Menge an Zytokinen, Interleukinen, Prostaglandinen und/oder Leukotrienen in Zellen und Geweben senken.

Vorteilhaft werden als entzündungshemmende bzw. rötungs- und juckreizlindernde Wirkstoffe steroidale entzündungshemmende Substanzen vom Kortikosteroiden-Typ, wie z.B. Hydrocortison, Dexamethason, Dexamethasonphosphat, Methylprednisolon oder Cortison eingesetzt, wobei die Auflistung durch Zusatz weiterer steroidaler Entzündungshemmer erweiterbar ist. Auch nichtsteroidale Entzündungshemmer können eingesetzt werden. Beispielhaft erwähnt werden sollen hier Oxicame wie Piroxicam oder Tenoxicam; Salicylate wie Aspirin, Disalcid, Solprin oder Fendosal; Essigsäure-Derivate wie Diclofenac, Fenclofenac, Indomethacin, Sulindac, Tolmetin, oder Clindanac; Fenamate wie Mefenamic, Meclofenamic, Flufenamic oder Niflumic; Propionsäure-Derivate wie Ibuprofen, Naproxen, Benoxaprofen oder Pyrazole wie Phenylbutazon, Oxyphenylbutazon, Febrazon oder Azapropazon. Alternativ können natürliche entzündungshemmende bzw. rötungs- und juckreizlindernde Stoffe eingesetzt werden. Einsetzbar sind Pflanzenextrakte, spezielle hochwirksame Pflanzenextrakt-Fraktionen sowie aus Pflanzenextrakten isolierte hochreine Wirksubstanzen. Besonders bevorzugt sind Extrakte, Fraktionen und Wirksubstanzen aus Kamille, Aloe vera, Commiphora-Arten, Rubia-Arten, Echinacea-Arten, Weiden, Weidenröschen, Hafer, schwarzem und grünem Tee, Gingko, Kaffee, Pfeffer, Johannisbeere, Tomate, Vanille, Mandeln, sowie Reinsubstanzen wie u.a. Bisabolol, Apigenin-7-glucosid, Boswelliasäure, Phytosterole, Glycyrrhizinsäure, Glabridin oder Licochalkon A.

Die Menge der Antiirritantien (eine oder mehrere Verbindungen) in erfindungsgemäßen Formulierungen beträgt vorzugsweise 0,01 bis 20 Gew.-%, besonders bevorzugt 0,03 bis 10 Gew.-%, insbesondere 0,05 bis 5 Gew.-%, bezogen auf das Gesamtgewicht der Formulierung.

Die erfindungsgemäßen Formulierungen (insbesondere topischen kosmetischen Formulierungen) können desweiteren Feuchthalteregulatoren und Osmolyte enthalten. Als Feuchthalteregulatoren ("moisturizer") finden z. B. folgende Stoffe Verwendung: Natriumlactat, Harnstoff, Alkohole (inbesondere 1,2-Pentandiol, 1,2-Hexandiol, 1,2-Octandiol, 1,2-Decandiol und deren Mischungen), Sorbit, Glycerin, Propylenglykol, Kollagen, Elastin oder Hyaluronsäure, Diacyladipate, Petrolatum, Ectoin, Urocaninsäure, Lecithin, Pantheol, Phytantriol, Lycopen, Algen-Extrakt, Ceramide, Cholesterol, Glykolipide, Chitosan, Chondroitinsulfat, Polyaminosäuren und -zucker, Lanolin, Lanolinester, Aminosäuren, alpha-Hydroxysäuren (z.B. Zitronensäure, Milchsäure, Äpfelsäure) und deren Derivate, Zucker (z.B. Inositol), alpha-Hydroxy-Fettsäuren, Phytosterole, Triterpensäuren wie Betulinsäure oder Ursolsäure, Algenextrakte. Als Osmolyte können z.B. eingesetzt werden: Zuckeralkohole (myo-Inositol, Mannitol, Sorbitol), quaternäre Amine wie Taurin, Cholin, Betain, Betain-Glycin, Ectoin, Diglycerolphosphat, Phosphorylcholin, Glycerophosphorylcholine, Aminosäuren wie Glutamin, Glycin, Alanin, Glutamat, Aspartat oder Prolin, Phosphatidylcholin, Phosphatidylinositol, anorganische Phosphate, sowie Polymere der genannten Verbindungen wie Proteine, Peptide, Polyaminosäuren und Polyole.

Die erfindungsgemäßen Formulierungen (z.B. topische kosmetische Formulierungen) enthalten weiterhin vorteilhaft antimikrobielle Wirkstoffe. Als Beispiele seien genannt:
Aryl- oder Aryloxy-substituierte, unverzweigte oder ein und mehrfach alkylverzweigte gesättigte oder ein bis fünffach ungesättigte (bis zu fünf Doppel- oder Dreifachbindungen, auch gemischte En-In-Verbindungen) Fettalkohole, - aldehyde und -säuren der Kettenlängen C₂ bis C₄₀.
Aryl- oder Aryloxy-substituierte unverzweigte oder ein und mehrfach alkylverzweigte gesättigte oder ein bis fünffach ungesättigte (bis zu fünf Doppel- oder Dreifachbindungen, auch gemischte En-In-Verbindungen) Alkandiole, Dialdehyde und Dicarbonsäuren der Kettenlängen C₂ bis C₄₀, besonders bevorzugt sind die Kettenlängen C₄ bis C₁₂.
Mono- und Oligoglyceride (bis 4 Glycerin-Einheiten) Aryl- oder Aryloxy-substituierter unverzweigter oder ein und mehrfach alkylverzweigter gesättigter oder ein bis fünffach ungesättigter (bis zu fünf Doppel- oder Dreifachbindungen, auch gemischte En-In-Verbindungen) Fettalkohole (Mono- und Oligoglycerinmonoalkylether), Fettsäuren (Mono- und Oligoglycerinmonoalkylester), Alkandiole (Mono- und Oligoglycerinmonoalkylether; Bis(mono-/oligoglyceryl)alkyldiether) und Dicarbonsäuren (Mono- und Oligoglycerinmonoalkylester; Bis(mono-/oligoglyceryl)-alkyldiester) der Kettenlängen C₂ bis C₄₀.

Fettsäureester unverzweigter oder ein und mehrfach alkylverzweigter gesättigter oder ein bis fünffach ungesättigter (bis zu fünf Doppel- oder Dreifachbindungen, auch gemischte En-In-Verbindungen), ggf. auch Aryl- oder Aryloxy-substituierter Carbonsäuren der Kettenlängen C₂ bis C₄₀ mit unverzweigten oder ein und mehrfach alkylverzweigten gesättigten oder ein bis fünffach ungesättigten (bis zu fünf Doppel- oder Dreifachbindungen, auch gemischte En-In-Verbindungen), ggf. auch Aryl- oder Aryloxy-substituierten ein- bis sechswertigen Fettalkoholen der Kettenlängen C₂ bis C₄₀.

Pflanzliche und tierische Fettsäureschnitte, enthaltend unverzweigte oder ein und mehrfach alkylverzweigte gesättigte oder ein bis fünffach ungesättigte (bis zu fünf Doppel- oder Dreifachbindungen, auch gemischte En-In-Verbindungen), Fettalkohole, -aldehyde und -säuren der Kettenlängen C₂ bis C₄₀ (z.B. Kokosfettsäuren, Palmkernfettsäuren, Wollwachssäuren).

Mono- und Oligoglyceride des Lanolins, von Lanolinalkoholen und Lanolinsäuren (z.B. Glyceryl Lanolat, Neocerit), Glycyrrhezitinsäure und Derivate (z.B. Glycyrrhetinyl Stearate), natürliche und synthetische Cardenolide (z.B. Digitoxin, Dogoxin, Digoxygenin, Gitoxygenin, Strophanthin und Strophanthidin), natürliche und synthetische Bufadienolide (z.B. Scillaren A, Scillarenin und Bufotalin), Sapogenine und Steroid-Sapogenine (z.B. Amyrine, Oleanolsäure, Digitonin, Gitogenin, Tigogenin und Diosgenin), Steroid-Alkaloide pflanzlichen und tierischen Ursprungs (z.B. Tomatidin, Solanin, Solanidin, Conessin, Batrachotoxin und Homobatrachotoxin).

Ein- und mehrfach halogenierte Nitrile, Dinitrile, Trinitrile oder Tetranitrile.

Mono- und Oligohydroxyfettsäuren der Kettenlängen C₂ bis C₂₄ (z.B. Milchsäure, 2- Hydroxypalmitinsäure), deren Oligo- und/oder Polymere sowie pflanzliche und tierische Rohstoffe dieselben enthaltend.

Acyclische Terpene: Terpenkohlenwasserstoffe (z.B. Ocimen, Myrcen), Terpenalkohole (z.B. Geraniol, Linalool, Citronellol), Terpenaldehyde und -ketone (z.B. Citral, Pseudoionon, beta-Ionon); Monocyclische Terpene: Terpenkohlenwasserstoffe (z.B. Terpinen, Terpinolen Limonen), Terpenalkohole (z.B. Terpineol, Thymol, Menthol), Terpenketone (z.B. Pulegon, Carvon); Bicyclische Terpene: Terpenkohlenwasserstoffe (z.B. Caran, Pinan, Bornan), Terpenalkohole (z.B. Borneol, Isoborneol), Terpenketone (z.B. Campher); Sesquiterpene: Acyclische Sesquiterpene (z.B. Farnesol, Nerolidol), Monocyclische Sesquiterpene (z.B. Bisabolol), Bicyclische Sesquiterpene (z.B. Cadinen, Selinen Vetivazulen, Guajazulen), Tricyclische Sesquiterpene (z.B. Santalen), Diterpene (z.B. Phytol), Tricyclische Diterpene (z.B. Abietinsäure), Triterpene (Squalenoide; z.B. Squalen), Tetraterpene.

Ethoxylierte, propoxylierte oder gemischt ethoxylierte/propoxylierte kosmetische Fettalkohole, Fettsäuren und Fettsäureester der Kettenlängen C₂ bis C₄₀ mit 1 bis 150 E/O- und/oder P/O-Einheiten.

Antimikrobielle Peptide und Proteine mit einer Aminosäurezahl von 4 bis 200, z.B. Skin Antimicrobial Peptides (SAPs), Lingual Antimicrobial Peptides (LAPs), humane beta-Defensine (insbes. h-BD1 und h-BD2), Lactoferrine und deren Hydrolysate sowie daraus gewonnene Peptide, Bactericidal/Permeability Increasing Proteins [BPls], Cationic Microbial Proteins [CAPs], Lysozym.

Gut geeignete Kohlenhydrate oder "Kohlenhydrat-Derivate", die sprachlich kurzgefasst auch unter die Bezeichnung "Kohlenhydrate" fallen sollen, sind Zucker und substituierte Zucker oder Zuckerreste enthaltende Verbindungen. Zu den Zuckern zählen insbesondere auch jeweils die Desoxy- und Didesoxy-Formen, N-Acetyl-Galactosamin-, N-Acetyl Glucosamin- und Sialinsäuresubstituierte Derivate sowie Zuckerester und -ether. Bevorzugt werden
a) Monosaccharide, darunter insbesondere Pentosen und Hexosen,
b) Disaccharide, darunter insbesondere Saccharose, Maltose, Lactobiose,
c) Oligosaccharide, darunter besonders die Tri- und Tetrasaccharide und
d) Polysaccharide, darunter besonders Stärke, Glykogen, Cellulose, Dextran, Tunicin, Inulin, Chitin, insbesondere Chitosane, Chitinhydrolysate, Alginsäure und Alginate, Pflanzengumme, Körperschleime, Pektine, Mannane, Galactane, Xylane, Araban, Polyosen, Chondroitinsulfate, Heparin, Hyaluronsäure und Glycosaminoglykane, Hemicellulosen, substituierte Cellulose und substituierte Stärke, insbesondere jeweils die hydroxyalkylsubstituierten Polysaccharide.

Besonders geeignet sind Amylose Amylopektin, Xanthan, alpha-, beta- und gamma-Dextrin. Die Polysaccharide können z.B. aus 4 bis 1.000.000, insbesondere 10 bis 100.000 Monosacchariden bestehen. Vorzugsweise werden jeweils solche Kettenlängen gewählt, die gewährleisten, dass der Wirkstoff in der jeweiligen Formulierung löslich oder in sie einzuarbeiten ist.

Sphingolipide wie Sphingosin; N-Monoalkylierte Sphingosine; N,N-Dialkylierte Sphingosine; Sphingosin-1-Phosphat; Sphingosin-1-Sulfat; Psychosin (Sphingosin-beta-D-Galactopyranosid); Sphingosylphosphorylcholin; Lysosulfatide (Sphingosylgalactosylsulfat; Lysocerebrosidsulfat); Lecithin; Sphingomyelin; Sphinganin.

Es können auch sogenannte "natürliche" antibakterielle Wirkstoffe eingesetzt werden, meist handelt es sich hierbei um ätherische Öle. Typische antibakteriell wirksame Öle sind beispielsweise Öle aus Anis, Zitrone, Orange, Rosmarin, Wintergrün, Nelke, Thymian, Lavendel, Hopfen, Citronella, Weizen, Zitronengras, Zedernholz, Zimt, Geranium, Sandelholz, Veilchen, Eukalyptus, Pfefferminz, Gum benzoin, Basilikum, Fenchel, Menthol sowie Ocmea origanum, Hydastis carradensis, Berberidaceae daceae, Ratanhiae oder Curcuma Ionga.

Wichtige antimikrobiell wirksame Substanzen, die in ätherischen Ölen gefunden werden können sind beispielsweise Anethol, Catechol, Camphen, Carvacrol, Eugenol, Eucalyptol, Ferulasäure, Farnesol, Hinokitiol, Tropolon, Limonen, Menthol, Methylsalicylat, Thymol, Terpineol, Verbenon, Berberin, Curcumin, Caryophyllenoxid, Nerolodol, Geraniol.

Es können auch Gemische der genannten Wirksysteme oder Wirkstoffe sowie Wirkstoffkombinationen, die diese Wirkstoffe enthalten, verwendet werden.

Vorzugsweise beträgt die Menge an antimikrobiellen Wirkstoffen in den Formulierungen 0,01 bis 20 Gew.-%, bezogen auf das Gesamtgewicht der Formulierungen, besonders bevorzugt 0,05 bis 10 Gew.-%.

Die erfindungsgemäßen (insbesondere kosmetischen, einschließlich der dermatologischen) Formulierungen können Deodorantien enthalten, d.h. Wirkstoffe mit deodorierender und schweißhemmender Wirkung. Hierzu zählen beispielsweise Geruchsüberdecker, wie die gängigen Parfümbestandteile, Antiperspirantien auf Basis von Aluminium-, Zirkonium- oder Zinksalze, Geruchsabsorber, beispielsweise die in der Patentoffenlegungsschrift DE-P 40 09 347 beschriebenen Schichtsilikate, von diesen insbesondere Montmorillonit, Kaolinit, Nontronit, Saponit, Hectorit, Bentonit, Smectit, ferner beispielsweise Zinksalze der Ricinolsäure. Dazu zählen ebenfalls bakterizide bzw. bakteriostatische deodoriende Substanzen, wie z. B. Hexachlorophen, 2,4,4'-Trichior-2'hydroxydiphenylether (Irgasan), 1,6-Di-(4-chlorphenylbiguanido)-hexan (Chlorhexidin), 3,4,4'-Trichlorcarbanilid, sowie die in den Patentoffenlegungsschriften DE-37 40 186, DE-39 38 140, DE-42 04 321, DE-42 29 707, DE-42 29 737, DE-42 37 081, DE-43 09 372, DE-43 24 219 beschriebenen wirksamen Agenzien, und kationaktive Substanzen enthalten, wie z. B. quaternäre Ammoniumsalze und Geruchsabsorber, wie z. B. Grillocin® (Kombination von Zinkrizinoleat und verschiedenen Zusätzen) oder Triethylcitrat, gegebenenfalls in Kombination mit Ionenaustauschharzen.

Vorzugsweise beträgt die Menge an desodorierenden und/oder antitranspiranten Wirkstoffen in den Formulierungen 0,01 bis 20 Gew.-%, bezogen auf das Gesamtgewicht der Formulierungen, besonders bevorzugt 0,05 bis 10 Gew.-%.

Die erfindungsgemässen (insbesondere kosmetischen) Formulierungen können, insbesondere wenn kristalline oder mikrokristalline Festkörper, beispielsweise anorganische Mikropigmente in die Formulierungen eingearbeitet werden sollen, auch anionische, kationische, nichtionische und/oder amphotere Tenside enthalten.

Anionische Tenside weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quaternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch. Typisch für nicht-ionische Tenside sind Polyether-Ketten. Nicht-ionische Tenside bilden in wässrigem Medium keine Ionen.

### A. Anionische Tenside

Vorteilhaft zu verwendende anionische Tenside sind Acylaminosäuren (und deren Salze), wie
- Acylglutamate, beispielsweise Natriumacylglutamat, Di-TEA-palmitoylaspartat und Natrium Capryl/ Capringlutamat,
- Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
- Sarcosinate, beispielsweise Myristoyl Sarcosin, TEA-lauroylsarcosinat, Natriumlauroylsarcosinat und Natriumcocoylsarkosinat,
- Taurate, beispielsweise Natriumlauroyltaurat und Natriummethylcocoyltaurat,
- Acyllactylate, Lauroyllactylat, Caproyllactylat
- Alaninate
Carbonsäuren und Derivate, wie
beispielsweise Laurinsäure, Aluminiumstearat, Magnesiumalkanolat und Zinkundecylenat,
- Ester-Carbonsäuren, beispielsweise Calciumstearoyllactylat, Laureth-6 Citrat und Natrium PEG-4 Lauramidcarboxylat,
- Ether-Carbonsäuren, beispielsweise Natriumlaureth-13 Carboxylat und Natrium PEG-6 Cocamide Carboxylat,
Phosphorsäureester und Salze, wie beispielsweise DEA-Oleth-10-Phosphat und Dilaureth-4 Phosphat,
Sulfonsäuren und Salze, wie
- Acyl-isothionate, z.B. Natrium-/ Ammoniumcocoyl-isethionat,
- Alkylarylsulfonate,
- Alkylsulfonate, beispielsweise Natriumcocosmonoglyceridsulfat, Natrium C₁₂. ₁₄ Olefin-sulfonat, Natriumlaurylsulfoacetat und Magnesium PEG-3 Cocamidsulfat,
- Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie
Schwefelsäureester, wie
- Alkylethersulfat, beispielsweise Natrium-, Ammonium-, Magnesium-, MIPA, TIPA-Laurethsulfat, Natriummyrethsulfat und Natrium C12-13 Parethsulfat,
- Alkylsulfate, beispielsweise Natrium-, Ammonium- und TEA- Laurylsulfat.

### B. Kationische Tenside

Vorteilhaft zu verwendende kationische Tenside sind
- Alkylamine,
- Alkylimidazole,
- Ethoxylierte Amine und
- Quaternäre Tenside.
   RNH₂CH₂CH₂COO⁻ (bei pH=7)
   RNHCH₂CH₂COO- B⁺ (bei pH=12) B⁺ = beliebiges Kation, z.B. Na⁺
- Esterquats

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind Alkylbetain, Alkylamidopropylbetain und Alkylamidopropylhydroxysulfain. Die verwendeten kationischen Tenside können ferner bevorzugt gewählt werden aus der Gruppe der quaternären Ammoniumverbindungen, insbesondere Benzyltrialkyl-ammoniumchloride oder - bromide, wie beispielsweise Benzyldimethylstearyl-ammoniumchlorid, ferner Alkyltrialkylammoniumsalze, beispielsweise Cetyltrimethylammoniumchlorid oder - bromid, Alkyldimethylhydroxy-ethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
- Acyl-/dialkylethylendiamin, beispielsweise Natriumacylamphoacetat, Dinatrium-acylamphodipropionat, Dinatriumalkylamphodiacetat, Natriumacylamphohydroxy-propylsulfonat, Dinatriumacylamphodiacetat und Natriumacylamphopropionat,
- N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
- Alkohole,
- Alkanolamide, wie Cocamide MEA/ DEA/ MIPA,
- Aminoxide, wie Cocoamidopropylaminoxid,
- Ester, die durch Veresterung von Carbonsäuren mit Ethylenoxid, Glycerin, Sorbitan oder anderen Alkoholen entstehen,
- Ether, beispielsweise ethoxylierte/propoxylierte Alkohole, ethoxylierte/ propoxylierte Ester, ethoxylierte/ propoxylierte Glycerinester, ethoxylierte/ propoxy-lierte Cholesterine, ethoxylierte/ propoxylierte Triglyceridester, ethoxyliertes prop-oxyliertes Lanolin, ethoxylierte/ propoxylierte Polysiloxane, propoxylierte POE-Ether und Alkylpolyglycoside wie Laurylglucosid, Decylglycosid undcocoglyco-sid.
- Sucrose (Saccharose)-ester, -ether
- Polyglycerinester, Diglycerinester, Monoglycerinester
- Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Die oberflächenaktive Substanz (Tensid) bzw. die Kombination der oberflächenaktiven Substanzen kann in einer Konzentration zwischen 1 und 98 Gew.-% in den erfindungsgemäßen Formulierungen vorliegen, bezogen auf das Gesamtgewicht der Formulierungen.

Erfindungsgemäße kosmetische (z.B. dermatologische) oder therapeutische Formulierungen, die die erfindungsgemäßen bzw. erfindungsgemäß einzusetzenden Verbindungen der Formel (I) enthalten, können auch als Emulsionen vorliegen.

Die Ölphase (Lipidphase) in den erfindungsgemäßen Formulierungen (insbesondere topischen kosmetischen Formulierungen) kann vorteilhaft gewählt werden aus folgender Substanzgruppe:
- Mineralöle (vorteilhaft Paraffinöl), Mineralwachse fette Öle, Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate (z.B. Gemische von n-Dodecyl-, n-Tridecyl-, n-Tetradecyl- oder n-Pentadecylbenzoat);
- cyclische oder lineare Silikonöle wie Dimethylpolysiloxane, Diethylpolysiloxane, Diphenylpolysiloxane sowie Mischformen daraus.

Vorteilhaft einsetzbar sind (natürliche oder synthetische) Ester, insbesondere (a) Ester aus gesättigten und/oder ungesättigten verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, (b) Ester aus aromatischen Carbonsäuren und gesättigten und/oder ungesättigten, verzweigten und/oder unverzweigten Alkoholen einer Kettenlänge von 3 bis 30 C-Atomen. Bevorzugte Esteröle sind Isopropylmyristat, Isopropylpalmitat, Isopropylstearat, Isopropyloleat, n-Butylstearat, n-Hexyllaurat, n-Decyllaurat, n-Decyloleat, Isooctylstearat, Isononylstearat, Isononylisononanoat, 3,5,5-Trimethylhexyl-3,5,5-trimethylhexanoat, 2-Ethylhexylisononanoat, 2-Ethylhexyl-3,5,5-trimethylhexanoat, 2-Ethylhexyl-2-ethylhexanoat, Cetearyl-2-ethylhexanoat, 2-Ethylhexylpalmitat, 2-Ethylhexyllaurat, 2-Hexyldecylstearat, 2-Octyldecylpalmitat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können vorteilhaft gewählt werden aus der Gruppe der synthetischen, halbsynthetischen und natürlichen Öle, z.B. Triglyceride der Caprin- oder Caprylsäure, Aprikosenkernöl, Avocadoöl, Baumwollsamenöl, Borretschsamenöl, Distelöl, Erdnussöl, Gamma-Oryzanol, Hagebuttenkernöl, Hanföl, Haselnussöl, Johannisbeersamenöl, Kokosöl, Kirschkernöl, Lachsöl, Leinöl, Maiskeimöl, Makadamianussöl, Mandelöl, Nachtkerzenöl, Nerzöl, Olivenöl, Palmöl, Palmkernöl, Pekannussöl, Pfirsichkernöl, Pistazienkernöl, Rapsöl, Reiskeimöl, Rizinusöl, Safloröl, Sesamöl, Sojaöl, Sonnenblumennöl, Teebaumöl, Traubenkernöl oder Weizenkeimöl, und dergleichen mehr. Auch beliebige Abmischungen solcher Öl- und Wachskomponenten sind vorteilhaft einsetzbar. In manchen Fällen ist es auch vorteilhaft, Wachse, beispielsweise Cetylpalmitat, als alleinige Lipidkomponente der Ölphase einzusetzen, vorteilhaft wird die Ölphase gewählt aus der Gruppe, die besteht aus 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid und Dicaprylylether. Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat. Auch die Kohlenwasserstoffe Paraffinöl, Squalan und Squalen lassen sich vorteilhaft verwenden. Vorteilhaft kann die Ölphase ferner einen Gehalt an cyclischen oder linearen Silikonölen aufweisen oder vollständig aus solchen Ölen bestehen, wobei es allerdings bevorzugt ist, außer dem Silikonöl oder den Silikonölen einen zusätzlichen Gehalt an anderen Ölphasenkomponenten zu verwenden. Cyclomethicon (z.B. Decamethylcyclopentasiloxan) kann vorteilhaft als Silikonöl eingesetzt werden. Aber auch andere Silikonöle sind vorteilhaft verwendbar, beispielsweise Undecamethylcyclotrisiloxan, Polydimethylsiloxan und Poly(methyl-phenylsitoxan). Besonders vorteilhaft sind ferner Mischungen aus Cyclomethicon und Isotridecylisononanoat sowie aus Cyclomethicon und 2-Ethylhexylisostearat.

Die wässrige Phase von als Emulsion vorliegenden erfindungsgemäsen Formulierungen (insbesondere topischen kosmetischen Formulierungen), kann vorteilhafterweise umfassen: Alkohole, Diole oder Polyole niedriger C-Zahl, sowie deren Ether, vorzugsweise Ethanol, Isopropanol, Propylenglykol, Glycerin, Ethylenglykol, Ethylenglykol-monoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate wie z.B. Bentonite, Polysaccharide bzw. deren Derivate, z.B. Hyaluronsäure, Guarkernmehl, , Xanthangummi, Hydroxypropyl-methylcellulose, oder Allulose-Derivate, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination, oder aus der Gruppe der Polyurethane, weiterhin alpha- oder beta-Hydroxysäuren, vorzugsweise Milchsäure, Zitronensäure oder Salicylsäure, daneben Emulgatoren, welche vorteilhaft ausgewählt werden können aus der Gruppe der ionischen, nichtionischen, polymeren, phosphathaltigen und zwitterionischen Emulgatoren,

Als Emulsion vorliegende erfindungsgemäße Formulierungen umfassen vorteilhaft einen oder mehrere Emulgatoren. O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel R-COO-(-CH₂-CH₂-O-)ₙ-H,
- der veretherten Fettsäureethoxylate der allgemeinen Formel

   R-COO-(-CH₂-CH₂-O-)ₙ-R',
- der veresterten Fettsäureethoxylate der allgemeinen Formel

   R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel R-COO-(-CH₂-CH₂-O-)ₙ-OOH, wobei n eine Zahl von 5 bis 30 darstellt,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel R-O-(-CH₂-CH(CH₃)-O-)ₙ-H
- der Polypropylenglycolether der allgemeinen Formel

   R-O-(-CH₂-CH(CH₃)-O-)ₙ-R'
- der propoxylierten Wollwachsalkohole,
- der veretherten Fettsäurepropoxylate R-COO-(-CH₂-CH(CH₃)-O-)ₙ-R'
- der veresterten Fettsäurepropoxylate der allgemeinen Formel

   R-COO-(-CH₂-CH(CH₃)-O-)ₙ-C(O)-R'
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)-O-)ₙ-CH₂-COOH,
- der Alkylethersulfate bzw. die diesen Sulfaten zugrundeliegenden Säuren der allgemeinen Formel R-O-(-CH₂-CH(CH₃)-O-)ₙ-SO₃-H,
- der Fettalkoholethoxylate/propoxylate der allgemeinen Formel R-O-Xₙ-Yₘ-H
- der Polypropylenglycolether der allgemeinen Formel R-O-Xₙ-Yₘ-R'
- der veretherten Fettsäurepropoxylate der allgemeinen Formel R-COO-Xₙ-Yₘ-R'
- der Fettsäureethoxylate/propoxylate der allgemeinen Formel R-COO-Xₙ-Yₘ-H.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 bis 18, ganz besonders vorteilhaft mit HLB-Werten von 14,5 bis 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(n)stearylether (Steareth-n) mit n = 13-20,
Polyethylenglycol(n)cetylether (Ceteth-n) mit n = 13-20,
Polyethylenglycol(n)isocetylether (Isoceteth-n) mit n = 13-20,
Polyethylenglycol(n)cetylstearylether (Ceteareth-n), mit n = 13-20,
Polyethylenglycol(m)isostearylether (Isosteareth-m) mit m = 12-20,
Polyethylenglycol(k)oleylether (Oleth-k) mit k = 12-15,
Polyethylenglycol(12)laurylether (Laureth-12),
Polyethylenglycol(12)isolaurylether (Isolaureth12).

Es ist ferner von Vorteil, die Fettsäureethoxylate aus folgender Gruppe zu wählen:
Polyethylenglycol(n)stearat, mit n = 20-25
Polyethylenglycol(m)isostearat mit m =12-25
Polyethylenglycol(k)oleat mit k = 12-20

Als ethoxylierte Alkylethercarbonsäure bzw. deren Salz kann vorteilhaft das Natriumlaureth-11-carboxylat verwendet werden. Als Alkylethersulfat kann Natrium Laureth 1-4 sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(n)glyceryllaurat mit n = 20-23 Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 bis 18 C-Atomen, Diglycerinester gesättigter und/oder ungesättigter, verzweigter und/oder unverzweigter Alkancarbonsäuren einer Kettenlänge von 8 bis 24, insbesondere 12 bis 18 C-Atomen, Monoglycerinether gesättigter und/oder ungesättigter, verzweigter und/oder unverzweigter Alkohole einer Kettenlänge von 8 bis 24, insbesondere 12 bis 18 C-Atomen, Diglycerinether gesättigter und/oder ungesättigter, verzweigter und/oder unverzweigter Alkohole einer Kettenlänge von 8 bis 24, insbesondere 12 bis 18 C-Atomen, Propylenglycolester gesättigter und/oder ungesättigter, verzweigter und/oder unverzweigter Alkancarbonsäuren einer Kettenlänge von 8 bis 24, insbesondere 12 bis 18 C-Atomen sowie Sorbitanester gesättigter und/oder ungesättigter, verzweigter und/oder unverzweigter Alkancarbonsäuren einer Kettenlänge von 8 bis 24, insbesondere 12 bis 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether(Steareth-2), Glycerylmonolaurat, Glycerylmonocaprinat, Glycerylmonocaprylat.

Es können auch Gemische der genannten Wirksysteme verwendet werden.

Vorzugsweise beträgt die Gesamtmenge an Verbindungen der Formel (II), an UV-Absorbern und an (weiteren) hautaufhellenden Mitteln in den erfindungsgemäßen Formulierungen 0,0001 bis 20 Gew.-%, bezogen auf das Gesamtgewicht der Formulierung, besonders bevorzugt 0,0005 bis 15 Gel.-%.

Zur Anwendung werden die erfindungsgemäßen topischen Formulierungen in der für Kosmetika üblichen Weise in ausreichender Menge auf die Haut und/oder die Haare in aufgebracht.

Die Erfindung wird nachfolgend anhand der Beispiele weiter beschrieben, ohne dass diese Beispiele den durch die Patentansprüche definierten Schutzbereich einschränken sollen. Sofern nicht anders angegeben beziehen sich alle Angaben auf das Gewicht.

### Beispiel 1: Herstellung der Verbindung der Formel (X)

1 g (6,15 mmol) Isoeugenol wurde in eine Mischung aus 1500 mL Wasser und 100 mL Aceton gegeben und bei Raumtemperatur (ca. 20°C) mit 11 mg Horseradish Peroxidase (Sigma Aldrich) versetzt. Anschließend wurde eine Mischung aus 10 mL Wasser und 0,21 mL 50%igem Wasserstoffperoxid (3,1 mmol) zugetropft. Nach einer Nachreaktionszeit von etwa 3 h bei Raumtemperatur (ca. 20°C) wurde die Reaktion beendet und die organische Phase mittels mehrfacher Extraktion mit einem organischen Lösungsmittel abgetrennt. Nach dem Einengen wurden 850 mg Rückstand erhalten, der zu etwa 80% die Verbindung der Formel (X) enthielt. Zu analytischen Zwecken wurde der Rückstand an Kieselgel chromatographiert (Hexan / Essigester: 2 / 1 (v/v)), wobei 350 mg der hochreinen Verbindung der Formel (X) erhalten wurden (Reinheit > 98%). Die Struktur der Verbindung der Formel (X) wurde mittels MS und NMR bestätigt.

### Beispiel 2: Nachweis der UVB-ausgelösten AhR-Induktion in humanen Keratinozyten sowie die Wirkung von AhR-Antagonisten

### Zellkultur und Bestrahlung

HaCaT - Keratinozyten wurden in DMEM Medium mit 10% fetalem Kälberserum kultiviert. Die Bestrahlung der Zellen mit UVB-Strahlung erfolgte in PBS (mit Phosphat gepufferte Kochsalzlösung). Zur Bestrahlung mit UVB benutzten wir die Lampe TL20W/12RS, welche vier parallele Röhren enthält (Philips, Eindhoven, Niederlande) und den Grossteil ihrer Energie im UVB Bereich (290-320 nm) emittiert. Der Emissionspeak der Lampe liegt bei 310 nm. Kontrollzellen wurden der gleichen Behandlung unterzogen, jedoch nicht bestrahlt. Um den AhR zu inhibieren, wurden die Zellen 1 h vor der Bestrahlung mit den Testsubstanzen behandelt.

### Transfektion von HaCaT Zellen mit dem pEGFP-AhR Plasmid

HaCaT Zellen wurden auf gekammerten Objektträgern mit einer Zelldichte von 5 x 10⁴ Zellen/Kammer ausplattiert. Einige wurden 1 h mit den Testsubstanzen vorbehandelt. Nach 24 h wurden diese mit dem Plasmid pEGFP-AhR mittels FuGene 6 Transfektionsreagens (Roche, Mannheim, Duetschland) laut Angabe des Herstellers transfiziert. Nach weiteren 24 h wurden die transfizierten Zellen mit 100 J/m² UVB bestrahlt. Nach 40 min wurden die Zellen 10 min lang mit 4% Paraformaldehyd fixiert und mit PBS gewaschen. Die Objektträger wurden getrocknet und mit Vectashield Mounting Medium (Vector Laboratories, Burlingame, CA, USA) eingedeckelt. Der an das GFP gekoppelte AhR wurde mittels eines Fluoreszenzmikroskops sichtbar gemacht (Olympus, Hamburg, Deutschland) und mit einer digitalen Kamera ColorViewXS (Olympus) photographiert.

### RNA Präparation, cDNA Synthese und real time RT-PCR

HaCaT-Zellen wurden mit 100 J/m² UVB bestrahlt. Einige Zellen wurden 1 h vor der Bestrahlung mit den Testsubstanzen vorbehandelt. Nach 4 h wurde die RNA mit dem RNeasy kit (Qiagen, Hilden, Deutschland) nach den Vorschriften des Herstellers präpariert. Die reverse Transkription wurde wie beschrieben durchgeführt (*Arch Toxicol* (2005) PMID 16205913). PCR-Fragmente wurden mittels real-time PCR im LightCycler (Roche, Mannheim, Deutschland) amplifiziert. Der PCR Mix setzte sich aus 1/10 Volumen des QuantiTect^{®} SYBR Green PCR Master Mixes (Qiagen, Hilden, Deutschland), 0.5 µM des jeweiligen Primers, 2 µl cDNA und DEPC (diethylpyrocarbonat) behandeltem H₂O in einem Endvolumen von 20 µl zusammen. Die PCR begann mit einer initialen 15 minütigen Erwärmung auf 95°C zur Aktivierung der DNA-Polymerase. Die PCR-Konditionen waren wie folgt: 40 Zyklen von 15 sec 94° C zur Denaturierung, 25 sec 60° C zur Primer Anlagerung, 30 sec 72° C zur Extension und 2 sec 72° C zur Fluoreszenzmessung. PCR-Primers für humanes CYP1A1 hatten die folgenden Sequenzen: 5'-TAGACACTGATCTGGCTGCAG für den forwärts und 5'-GGGAAGGCTCCATCAGCATC für den Rückwärts-Primer (Cancer Res. 1990, 50, 4315), welche nach Amplifikation ein 146 bp Fragment bildeten. Die Quantifizierung der PCR Produkte geschah über eine fragmentspezifische Standardkurve und wurde mit der LightCycler software 3 quantifiziert. Standardkurven wurden mit 10² to 10⁶ CYP1A1 cDNA Kopien/µl hergestellt und wie oben beschrieben amplifiziert.

### Ergebnisse

Der Standard 3-Methoxy-4-Nitroflavon hemmte die AhR-Translokation in den Zellkern in An- und Abwesenheit von UVB-Licht und zeigte damit die It. Literatur erwartete Wirkung.

### Quantifizierung von CYP1A1 mittels RNA Präparation, cDNA Synthese und real time RT-PCR

HaCaT-Zellen wurden mit 100 J/m² UVB bestrahlt. Einige Zellen wurden 1 h vor der Bestrahlung mit den Testsubstanzen vorbehandelt. Nach 4 h wurde die RNA mit dem RNeasy kit (Qiagen, Hilden, Deutschland) nach den Vorschriften des Herstellers präpariert. Die reverse Transkription wurde wie beschrieben durchgeführt (*Arch Toxicol* (2005) PMID 16205913). PCR-Fragmente wurden mittels real-time PCR im LightCycler (Roche, Mannheim, Deutschland) amplifiziert. Der PCR Mix setzte sich aus 1110 Volumen des QuantiTect^{®} SYBR Green PCR Master Mixes (Qiagen, Hilden, Deutschland), 0.5 µM des jeweiligen Primers, 2 µl cDNA und DEPC (diethylpyrocarbonat) behandeltem H₂O in einem Endvolumen von 20 µl zusammen. Die PCR begann mit einer initialen 15 minütigen Erwärmung auf 95°C zur Aktivierung der DNA-Polymerase. Die PCR-Konditionen waren wie folgt: 40 Zyklen von 15 sec 94° C zur Denaturierung, 25 sec 60° C zur Primer Anlagerung, 30 sec 72° C zur Extension und 2 sec 72° C zur Fluoreszenzmessung. PCR-Primers für humanes CYP1A1 hatten die folgenden Sequenzen: 5'-TAGACACTGATCTGGCTGCAG für den forwärts und 5'-GGGAAGGCTCCATCAGCATC für den Rückwärts-Primer (Cancer Res. 1990, 50, 4315), welche nach Amplifikation ein 146 bp Fragment bildeten. Die Quantifizierung der PCR Produkte geschah über eine fragmentspezifische Standardkurve und wurde mit der LightCycler software 3 quantifiziert. Standardkurven wurden mit 10² to 10⁶ CYP1A1 cDNA Kopien/µl hergestellt und wie oben beschrieben amplifiziert.

**Tabelle 1**

| Substanz | Konzentration | CYP1A1-Inhibition in Gegenwart von UVB-Licht* |
|---|---|---|
| Verbindung (X) | 0,0001 Gel.-% | 20% |

| | | |
|---|---|---|
| * relativ zur Kontrolle (PBS ohne Testsubstanzen mit 0,1 % DMSO, UVBbestrahlt) | | |

Die erfindungsgemäße Verbindung der Formel (X) hemmte die Induktion von CYP1A1 infolge der AhR-Aktivierung in Anwesenheit von UVB-Licht.

### Beispiel 3: Nachweis der Induktion von CYP1A1 durch AhR-Aktivierung in murinen Melanozyten

Melanozyten der Maus des Stammes C57BU6 wurden in Kultur expandiert (Medium: "Melanocyte growth medium" (Promocell), 37°C, 5% CO2). Fibroblastenwachstum wurde durch Zugabe von G418 (Tag10-13, 45µg/ml) inhibiert. Um den AhR zu modulieren, wurden die semikonfluenten Zellen bis zu 7 Tagen mit dem AhR-Agonisten FICZ behandelt. Unbehandelte Zellen wurden parallel mitgeführt. Anschließend wurde das Medium abgenommen, die Zellen lysiert, die RNA wie in Beispiel 2 beschrieben präpariert und der Gehalt von CYP450 1A1 RNA mit quantitativer RT-PCR photometrisch bestimmt.

Ergebnis: Die Menge an CYP450 1A1 mRNA in den Zellen wird durch dieses Vorgehen signifikant erhöht im Vergleich zu unbehandelten Zellen (p < 0.05, Student's t-test).

### Transfektion von Melanozyten mit dem pEGFP-AhR Plasmid

Primäre Maus-Melanozyten-Zellen wurden auf gekammerten Objektträgern ausplattiert. Einzelne Kammern wurden für 1 h mit den Testsubstanzen behandelt. Nach 24 h wurden die Zellen mit dem Plasmid pEGFP-AhR transfiziert. Nach weiteren 40 min wurden die Zellen fixiert. Die Objektträger wurden fluoreszenzmikroskopisch ausgewertet. Sichtbar wird die Translokation des an das GFP gekoppelten, daher grünfluoreszierenden AhR vom Zytoplasma in den Zellkern.

### Ergebnisse

3-Methoxy-4-Nitroflavon (MNF) hemmte die AhR-Translokation in den Zellkern in An- und Abwesenheit von Benz-[a]-pyren.

FICZ stimulierte die AhR-Translokation in den Zellkern in An- und Abwesenheit von Benz-[a]-pyren.

### Beispiel 4: Modulation der Melaninsynthese mit AhR-Modulatoren

### Verstärkung der Melaninsynthese mit FICZ

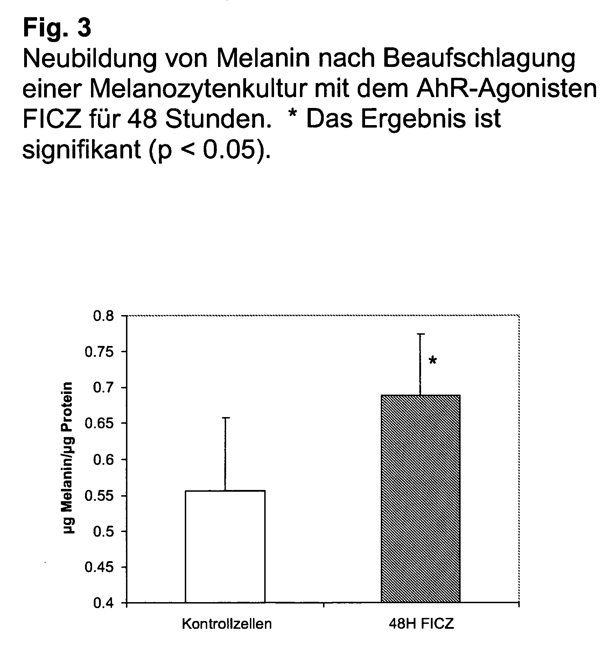

FICZ wurde durch eine 60-minütige Bestrahlung einer 50mM Tryptophanlösung mit einer UVB-Quelle generiert. Semikonfluente Kulturen primärer Maus-Melanozyten (Kulturbedingungen wie oben) wurden für 2 Tage mit dieser FICZhaltigen Lösung kultiviert. Die Zellen wurden abgenommen, mit 0,1 % Triton-X100 lysiert, zentrifugiert und der Melaningehalt aus dem Lysat-Pellet nach Zellaufschluss mit 1 M NH₄OH (4 h, 85°C) photometrisch in einem ELISA-Reader bei 405 nm bestimmt. Von dem Absorptionwert wurde der des Leerwerts, welcher nur NH₄OH enthielt, subtrahiert. Parallel wurde der Proteingehalt der Lysat-Überstände nach der Bradford-Methode durchgeführt. Die FICZ-behandelten Melanozyten enthielten signifikant mehr Melanin als die unbehandelten Zellen (p < 0.05, Student's t-test) (Fig. 3).

### Inhibition der Melaninsynthese mit MNF

Semikonfluente primäre Maus-Melanozyten (Kulturbedingungen wie oben) wurden für 7 Tage mit 10 µM MNF weiter kultiviert. Die Zellen wurden abgenommen, mit 0,1 % Triton-X100 lysiert, zentrifugiert und der Melaningehalt aus dem Lysat-Pellet nach Zellaufschluss mit 1 M NH4OH (4 h, 85°C) photometrisch in einem ELISA-Reader bei 405 nm bestimmt. Von dem Absorptionwert wurde der des Leerwerts, welcher nur NH4OH enthielt, subtrahiert.

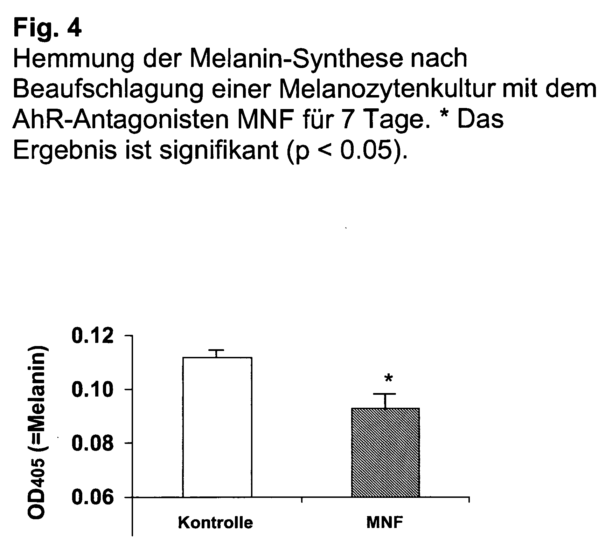

Ergebnis: Die MNF-behandelten Melanozyten enthielten signifikant weniger Melanin als die unbehandelten Zellen (p < 0.05, Student's t-test) (Fig. 4)

### Beispiel 5: Formulierungsbeispiele

**Formulierung 1: "Wasser in Öl"-Emulsion mit UVA/B-Breitbandschutz**
**Formulierung 2: "Öl in Wasser"-Emulsion mit UVA/B-Breitbandschutz**
**Formulierung 3: "Öl in Wasser"-Emutsion mit UVA/B-Breitbandschutz**
**Formulierung 4: Ölfreies Sonnenspray mit UVA/B-Breitbandschutz**
**Formulierung 5: Balm mit UVA/UVB-Schutz**
**Formulierung 6: Aerosol-Schaum mit UVB/UVA-Schutz**
**Formulierung 7: Nicht-Aerosol-Schaum**
**Formulierung 8: Shampoo mit UVB-Zellschutz**
**Formulierung 9: Haar-Conditioner mit UVB/UVA -Schutz**
**Formulierung 10: Tagescreme O/W mit UVB-Zellschutz**
**Formulierung 11: Nachtcreme W/O mit UVB-Zellschutz**

**Tabelle 2: Zusammensetzungen erfindungsgemäßer Formulierungen (Formulierungen 1-11)**

| **ROHMATERIAL-NAME (HERSTELLER)** | INCI | Gewichts-% | | | | | | | | | | |
|---|---|---|---|---|---|---|---|---|---|---|---|---|
| | | **1** | **2** | **3** | **4** | **5** | **6** | **7** | **8** | **9** | **10** | **11** |
| AhR-Antagonisten | | | | | | | | | | | | |
| Verbindung (X) | | 0.2 | 5.0 | 0.1 | 0.2 | 1.0 | 0.5 | 0.1 | 1.0 | 0.2 | 1.5 | 0.4 |
| Weitere Inhaltsstoffe | | | | | | | | | | | | |
| Abil 100® (Goldschmidt) | Dimethicone | | 1.0 | 0.3 | | | | | | | | |
| Abil 350 (Degussa-Gold-schmidt) | Dimethicone | | | | | | | | | | 0.5 | |
| -(Alpha-)-Bisabolol, natural (Symrise) | Bisabolol | | | | | | | | | | 0.3 | 0.2 |
| Aloe Vera Gel Concentrate 10/1 (Symrise) | Water (Aqua), Aloe Barbadensis Leaf Juice | | | | | | | | | | | |
| Alugel 34 TH (Baerlocher) | Aluminium Stearate | | | | | | | | | | | 1.0 |
| Arbutin (Sabinsa) | β-Arbutin | | | | | | | | | | 1.0 | |
| Arlypon F | Laureth-2 | | | | | | | | 2.0 | | | |
| Baysiloneöl M10 (GE Bayer) | Dimethicone | | | | | | | 1.0 | | | | |
| Baysilone Oil PK 20 (GE Bayer) | Phenyl Trimethicone | | | | | 5.0 | | | | | | |
| Bentone Gel M IO® (Rheox) | Mineral oil and Quatemium-18-hectorite and Propylencarbonate Glycerylstearate and Cetylalcohol | | 3.0 | | | | | | | | | |
| alpha-Bisabolol (Symrise) | Bisabolol | 0.1 | | 0.1 | | 0.2 | 0.1 | 0.1 | 0.1 | | | |
| 1,3-Butylengiykol | 1,3-Butylenglycol | | | 3.0 | | | | | | | | |
| Carbopol 2050® (B.F. Goodrich) | Carbomer | | | 0.2 | | | 0.1 | | | | | |
| Carbopol ETD 2001 (Noveon) | Carbomer | | | | | 0.5 | | | | | | |
| Ceramide 2 (Sederma) | Ceramide 2 | | | | | | | | | | 0.1 | |
| Ceramide SL (Sino Lion) | Hydroxyethyl Palmityl Oxyhydroxypropyl Palmitamide | | | | | | | | | | | 0.1 |
| Cetiol SN® (Cognis) | Cetyl- und Stearyl-isononanoat | 5.0 | 4.0 | 5.0 | | | | | | | | |
| Cetiol OE (Cognis) | Dicaprylyl Ether | | | | | | | 3.0 | | | | |
| Citric Acid | Citric Acid | | | | | | | | | 0.1 | | |
| Copherol 1250® (Cognis) | Tocopherol acetate | 1.0 | | 0.5 | | 0.5 | 0.5 | 0.5 | | | | |
| Corapan TQ® (Symrise) | Diethylhexyl-1,6-Naphtalate | | | 3.0 | | | | | | | | |
| Crinipan® AD (Symrise) | Climbazol | | | | | | | | 0.5 | | | |
| Crotein Q (Croda) | Hydroxypropyl trimonium Hydrolyzed | | | | | | | | | 1.0 | | |
| Cutina CBS® (Cognis) | Glycerylstearat and Cetylalkohol and Stearylalkohol and Cetylpalmitat and Cocosglyceride | | 2.0 | | | | | | | | | |
| Dehymuls PGPH® (Cognis) | Polyglycerol-2 Dipolyhydroxystearat | 3.0 | | | | | | | | | | |
| Dehyquart SP | Quaternium-52 | | | | | | | | | 0.5 | | |
| Dehyton K | Cocamidopropyl Betaine | | | | | | | | 12.0 | | | |
| Dow Corning® 193 (Dow corning) | Dimethicon-Polyol | | | | 1.0 | | | | | | | |
| Dow Corning 200 Fluid (Dow Corning) | Dimethicone | | | | | | | | | | | |
| D-Panthenol (BASF) | Panthenol | | | | 0.5 | | | 0.5 | 0.4 | | | |
| Dracorin 100 s.e.® (Symrise) | Glycerylstearat (and) PEG-100-Stearat | | 3.0 | | | | | | | | | |
| Dracorin CE (Symrise) | Glyceryl Stearate Citrate | | | | | | | | | | 5.0 | |
| Dragocid Liquid (Symrise) | Phenoxyethanol (and) Methylparaben (and) Ethylparaben (and) Butylparaben (and) Propylparaben (and) Isobutylparaben | 0.3 | 0.3 | 0.3 | | 0.3 | 0.3 | 0.3 | | 0.5 | | 0.8 |
| Drago-Beta-Glucan (Symrise) | Water (Aqua), Butylene Glycol, Glycerin, Avena Sativa (Oat), Kernel Extract | | | | | | | | | | 0.3' | |
| Dragoderm (Symrise) | Glycerin, Triticum Vulgare (Wheat) Gluten, Water (Aqua) | | | | | | | | 2.0 | | | |
| Dragophos S (Symrise) | Sodium Dihydroxycetyl Phosphate | | | | | | | | | | | |
| Dragorin GMS (Symrise) | Glyceryl Stearate | | | 2.0 | | | 2.0 | | | | | |
| Dragosan W/O Liquid (Symrise) | Polyglyceryl-3-Polyricinoleate, Sorbitan Isostearate | | | | | | | | | | | 1.0 |
| Dragosan W/O P (Symrise) | Sorbitan Isostearate, Hydrogenated Castor Oil, Ceresin, Beeswax (Cera Alba) | | | | | | | | | | | 6.0 |
| Dragoxat EH (Symrise) | Ethylhexyl Ethylhexanoate | | | | | | | | | | 3.0 | |
| Edeta BD® (BASF) | Dinatrium-EDTA | 0.1 | 0.1 | 0.1 | | 0.1 | 0.1 | 0.1 | | | | |
| Emulgin B2® (Cognis) | Ceteareth-20 | | 1.0 | | | | | | | 0.7 | | |
| Emulsiphos (Symrise) | Cetylphosphate, Hydrogented Palm glycerides | | | 1.5 | | | 1.5 | | | | | |
| Ethanol (96 %) | Ethyl alcohol | | | | 13.0 | 5.0 | | | | | | |
| Euxyl K 100® (Schülke & Mayr) | Methylchlorisothiazoli-none, Methyisothiazolinone | | | | 0.1 | | | | | | | |
| Extrapon Aloe Vera (Symrise) | Aqua, Aloe Barbadensis, Propylene Glycol, Alcohol | | | | 1.0 | | | | | | | |
| Extrapon Kamille (Symrise) | Glycerin, Water (Aqua), Chamomilla Recutita (Matricaria) Flower Extract | | | | 1.0 | | | | | | | |
| Extrapon Hamamelis (Symrise) | Propylene Glycol, Hamamelis Virginiana (Witch Hazel) Water, Water (Aqua), Alcohol, Hamamelis Virginiana (Witch Hazel) Bark/Leaf/Twig Extract | | | | 1.0 | | | | | | | |
| Glycerin 85 % | Glycerin | | | | | | | | | | 3.0 | 2.0 |
| Glycerin 99 % | Glycerin | 4.0 | 3.0 | | 4.5 | | 3.0 | 4.0 | | | | |
| Hydrolite-5 (Symrise) | 1,2-Pentanediol | | | | | 4,0 | | 5,0 | | | | |
| Isodragol (Symrise) | Trisononanoin | | | | | | | | | | | |
| Isopropylmyristat (Symrise) | Isopropyl Myristate | | | | | | | | | | | |
| Isopropylpalmitat (Symrise) | Isopropyl Palmitate | | | | | | | | | | 4.0 | |
| Karion F (Merck) | Sorbitol | | | | | | | | | | | 2.0 |
| Keltrol RD (CP-Kelco) | Xanthan Gum | | | | | | | | | | 0.2 | |
| Keltrol T® (Calgon) | Xanthan Gum | | | 0.2 | 0.2 | 0.3 | | | | | | |
| Kojic Acid (Cosmetochem) | Kojic Acid | | | | | | | | | | 1.0 | |
| Lanette E® (Cognis) | Natriumcetearylsulfat | | | 0.7 | | | | | | | | |
| Lanette O® (Cognis) | Cetyl- und Stearylalkohol | | 1.1 | | | | | | | 2.5 | | |
| Lanette 16® (Cognis) | Cetylalkohol | | | 1.2 | | | 0.5 | | | | 1.0 | |
| Lanette 18 (Care Chemicals) | Stearyl Alcohol | | | | | | | | | | 4.5 | |
| Lara Care A-200 (Rahn) | Galactoarabinan | | | | | | | 0.2 | | | | |
| Mg Ascorbyl-phosphate | Magnesium Ascorbyl-phosphate | | | | | | | | | | 3.0 | |
| Magnesium Chloride (Merck) | Magnesium Chloride | | | | | | | | | | | 0.7 |
| Monomuls 90-O 18® (Cognis) | Glyceryloleate | 1.0 | | | | | | | | | | |
| Myritol 318® (Cognis) | Capryl-/Caprinsäuretri-glyceride | 6.0 | 5.0 | | | | | | | | | |
| NaOH 10 % wässr. Lösung | Sodium hydroxide | | | 2.8 | | 2.2 | 2.9 | 0.6 | | | | |
| Natrium Ascorbyl Phosphate (EMD Chemicals) | Natrium Ascorbyl Phosphate | | | | | | | | | | 2.0 | |
| Natrosol 250 HHR (Aqualon) | Hydroxymethyl cellulose | | 0.3 | | | | | | | | | |
| Neo-Dragocid Pulver (Symrise) | Methylparaben, Sorbic Acid, Dehydroacetic Acid, Propylparaben | | | | | | | | | | | |
| Neo Heliopan® AP (Symrise), 15 % als Natriumsalz | Dinatrium-Phenyl-dibenzimidazoltetrasulfonat | 10.0 | | 22.0 | | | | | | | | |
| Neo Heliopan® AP (Symrise), 10 % wässr. Lösung neu-tralisiert mit NaOH) | Dinatrium-Phenyl-dibenzimidazoltetrasulfonat | | | 22.0 | 22.0 | | | | | | | |
| Neo Heliopan® AV (Symrise) | Ethylhexylmethoxy-cinnamat | 4.0 | | | | 5.0 | 6.0 | 2.0 | | | | |
| Neo Heliopan® BB (Symrise) | Benzophenone-3 | | 1.0 | | | | | | | | | |
| Neo Heliopan® 303 (Symrise) | Octocrylene | | 7.0 | | | | | | | | | |
| Neo Heliopan® 357 (Symrise) | Butyl Methoxydibenuzoylmethane | | | 2.0 | | 1.5 | 1.5 | 1.5 | 0.5 | 0.5 | | |
| Neo Heliopan® E 1000 (Symrise) | lsoamyl-p-methoxycinnamate | 4.0 | | | | 5.0 | | 6.0 | | 2.0 | | |
| Neo Heliopan® HMS (Symrise) | Homosatate | | | 5.0 | | | | | | | | |
| Neo Heliopan® Hydro (15 % wässr. Lösung neutralisiert mit NaOH) (Symrise) | Phenylbenzimidazol sulfonic acid | | | | 33.3 | 10.0 | 13.3 | | 3.3 | | | |
| Neo Heliopan® MA (Symrise) | Menthylanthranilate | | 3.0 | | | | | | | | | |
| Neo Heliopan® MBC (Symrise) | 4-Methylbenzylidencampher | 2.0 | | | | 2.0 | 4.0 | 3.0 | | | | |
| Neo Heliopan® OS (Symrise) | Ethylhexylsalicylate | 3.0 | | | | | | | | | | |
| Neutralöl (Symrise) | Capryl-/Caprinsäuretriglyceride | | | 5.0 | | | 2.0 | | | | 6.0 | |
| Octyltriazon | Ethylhexyltriazone | 1.0 | | | | | | | | | | |
| Oxynex 2004 (Merck) | BHT | | | | | | | | | | | 0.1 |
| Paraffinöl 5 Grad E (Parafluid) | Paraffinum Liquidum | | | | | | | | | | | |
| Parfümöl (Symrise Fragrance) | Parfum (Fragrance) | 0.3 | 0.3 | 0.3 | | 0.3 | 0.4 | 0.2 | 0.5 | 0.4 | 0.3 | 0.,4 |
| PCL liquid (Symrise) | Cetearyl Ethylhexanoate, Isopropyl Myristate | | | | | | | | | | | 12.0 |
| PCL Liquid 100 (Symrise) | Cetearyl Ethylhexoate | | | | | | | | | | 3.0 | |
| Pemulen TR 2 (Novion) | Acrylates/C10-30 Alkyl Acrylate Crosspolymer | | | | | | | 0.2 | | | | |
| Permulgin 2550® (Koster Keunen) | Bees Wax | 1.0 | | | | | | | | | | |
| Phenoxyethanol (Symrise) | Phenoxyethanol | 0.7 | | 0.7 | | 0.7 | 0.7 | 0.7 | | | | |
| Polymer JR 400 | Potyquatemium-10 | | | | | | | | 0.4 | | | |
| 1,2-Propylenglycol | Propylene Glycol | | | | | | | | | | | |
| Retinyl Palmitate in Oil (DSM Nutrional Products) | Retinyl Palmitate | | | | | | | | | | | 0.2 |
| Softigen 767 | PEG-6 Caprytic/Capric Glycerides | | | | | | | | 2.5 | | | |
| Solubilizer (Symrise) | PEG 40 Hydrogenated Cantor Oil, Trideceth-9, Propyleneglycol, Water | | | | | | | | 3.0 | | | |
| Sun Flower Oil (Wagner) | Helianthus Annuus (Sunflower) Seed Oil | | | | | | | | | | | 5.0 |
| Sweet Almond Oil (Wagner) | Prunus dulcis | | | | | | | | | | | 5.0 |
| SymCalmin | Butylene Glycol, Pentylene Glycol, Hydroxyphenyl Propamidobenzoic Acid | | | 0.5 | | | | | | | | |
| Symdiol 68 (Symrise) | 1,2 Hexanediol, Caprylylglycol | | | 0,5 | | | | | | | 0.5 | |
| SymMatrix (Symrise) | Maltodextrin, Rubus Fruticosus (Blackberry) Leaf Extract | | | | | | | | | | | 1.0 |
| SymWhite 377 | 4-(1-Phenylethyl)1,3-benzenediol | | | | | | | | | | 0.5 | |
| Tegosoft TN® (Goldschmidt) | C12-C15 Alkylbenzoate | 6.0 | | | | 4.0 | 2.0 | | | | | |
| Texapon N 70 (Cognis) | Sodium Laureth Sulfate | | | | | | 0.1 | 0.5 | | | | |
| Texapon NSO BZ (Cognis) | Sodum Laureth Sulfate | | | | | | | | 27.0 | | | |
| Titandioxid mikrofein | Titan dioxide | | 5.0 | | | | | | | | | |
| Tocopherol Acetate (DSM Nutritional Products) | Tocopheryl Acetate | | | | | | | | | | | 3.0 |
| Unimer U-151 (Induchem) | PVP/Hexadecene Copolymer | | | | | 0.5 | | | | | | |
| Veegum ultra® (Vanderbilt) | Magnesium Aluminium sulfate | | 1.0 | | | | | | | | | |
| Witch Hazel Distillate (Symrise) | Hamamelis Virginiana (Witch Hazel) | | | | | | | | 1.0 | | | |
| Zinkoxid neutral (Symrise) | Zinc oxide | 7.0 | | | | | | | | | | |
| Wasser, dest. | Aqua (Water) | ad 100 | ad 100 | ad 100 | ad 100 | ad 100 | ad 100 | ad 100 | ad 100 | ad 100 | ad 100 | ad 100 |

### Beispiel 6: Formulierungsbeispiele für Hautaufheller

In nachfolgender Tabelle 3 bezeichnen:
Formulierung 1: "Wasser in Öl"-Emulsion mit UVA/B-Breitbandschutz
Formulierung 2: "Öl in Wasser"-Emulsion mit UVA/B-Breitbandschutz
Formulierung 3: Hautaufhellende "Öl in Wasser"-Emulsion mit UVA/B-Breitbandschutz
Formulierung 4: Hautaufhellendes ölfreies Sonnenspray mit UVA/B-Breitbandschutz
Formulierung 5: Hautaufhellender Balm mit UVA/UVB-Schutz
Formulierung 6: Hautaufhellender Aerosol-Schaum mit UVB/UVA-Schutz
Formulierung 7: Hautaufhellender Nicht-Aerosol-Schaum
Formulierung 8: Shampoo mit haaraufhellenden Eigenschaften
Formulierung 9: Haaraufhellender Haar-Conditioner mit UVB/UVA -Schutz
Formulierung 10: Hautaufhellender Feuchtigkeitscreme O/W
Formulierung 11: Hautaufhellende Gesichtscreme O/W

**Tabelle 3: Zusammensetzungen erfindungsgemäßer Formulierungen (Formulierungen 1-11)**

| **ROHMATERIAL-NAME (HERSTELLER)** | INCI | Gewichts-% | | | | | | | | | | |
|---|---|---|---|---|---|---|---|---|---|---|---|---|
| | | **1** | **2** | **3** | **4** | **5** | **6** | **7** | **8** | **9** | **10** | **11** |
| Hautaufheller | | | | | | | | | | | | |
| 3-Methoxy-4-Nitroflavon | | 0.01 | 5.0 | 0.05 | 0.2 | 1.0 | 0.5 | 0.1 | 0.5 | 0.2 | 1.0 | 0.5 |
| SymWhite377 | Phenylethyl resorcinol | | 0.5 | | | | | 0.1 | | | | |
| beta-Arbutin | Arbutin | 1.0 | | | | 0.5 | | | | | 0.2 | |
| Nicotinamid | Niacinamide | | | | 0.5 | | | | | | 1.0 | |
| Kojisäure | Kojic Acid | | | 0.5 | | | | | | | | 1.0 |

| Weitere Inhaltsstoffe | | | | | | | | | | | | |
|---|---|---|---|---|---|---|---|---|---|---|---|---|
| Abil 100® (Goldschmidt) | Dimethicone | | 1.0 | 0.3 | | | | | | | | 0.3 |
| Dracorin 100 s.e.® (Symrise) | Glycerylstearat (and) PEG-100-Stearat | | 3.0 | | | | | | | | | |
| Arlypon F | Laureth-2 | | | | | | | | 2.0 | | | |
| Baysiloneöl M10 (GE Bayer) | Dimethicone | | | | | . | | 1.0 | | | | |
| Baysilone Oil PK 20 (GE Bayer) | Phenyl Trimethicone | | | | | 5.0 | | | | | | |
| Bentone Gel M IO® (Rheox) | Mineral oil and Quaternium-18-hectorite and Propylencarbonate Glycerylstearate and Cetylalcohol | | 3.0 | | | | | | | | | |
| alpha-Bisabolol (Symrise) | Bisabolol | 0.1 | | 0.1 | | 0.2 | 0.1 | 0.1 | 0.1 | | | |
| 1,3-Butylenglykol | 1,3-Butylenglycol | | | 3.0 | | | | | | | | |
| Carbopol 2050® (B.F. Goodrich) | Carbomer | | | 0.2 | | | 0.1 | | | | | |
| Carbopol ETD 2001 (Noveon) | Carbomer | | | | | 0.5 | | | | | | |
| Cetiol SN® (Cognis) | Cetyl- und Stearyl-isononanoat | 5.0 | 4.0 | 5.0 | | | | | | | | |
| Cetiol OE (Cognis) | Dicaprylyl Ether | | | | | | | 3.0 | | | | |
| Citric Acid | Citric Acid | | | | | | | | | 0.1 | 0.3 | |
| Copherol 1250® (Cognis) | Tocopherol acetate | 1.0 | | 0.5 | | 0.5 | 0.5 | 0.5 | | | | |
| Corapan TQ® (Symrise) | Diethylhexyl-1,6-Naphtalate | | | 3.0 | | | | | | | | |
| Crinipan® AD (Symrise) | Climbazol | | | | | | | | 0.5 | | | |
| Crotein Q (Croda) | Hydroxypropyl trimonium Hydrolyzed | | | | | | | | | 1.0 | | |
| Cutina CBS® (Cognis) | Glycerylstearat and Cetylalkohol and Stearylalkohol and Cetylpalmitat and Cocosglyceride | | 2.0 | | | | | | | | | |
| Dehymuls PGPH® (Cognis) | Polyglycerol-2 Dipolyhydroxystearat | 3.0 | | | | | | | | | | |
| Dehyquart SP | Quaternium-52 | | | | | | | | | 0.5 | | |
| Dehyton K | Cocamidopropyl Betaine | | | | | | | | 12.0 | | | |
| Dow Corning® 193 (Dow corning) | Dimethicon-Polyol | | | | 1.0 | | | | | | | |
| Dow Corning 200 Fluid (Dow Corning) | Dimethicone | | | | | | | | | | 2.0 | |
| D-Panthenol (BASF) | Panthenol | | | | 0.5 | | | 0.5 | 0.4 | | | |
| Dragocid Liquid (Symrise) | Phenoxyethanol (and) Methylparaben (and) Ethylparaben (and) Butylparaben (and) Propylparaben (and) Isobutylparaben | 0.3 | 0.3 | 0.3 | | 0.3 | 0.3 | 0.3 | | 0.5 | 0.8 | |
| Dragophos S (Symrise) | Sodium Dihydroxycetyl Phosphate | | | | | | | | | | 2.0 | |
| Dragorin GMS (Symrise) | Glyceryl Stearate | | | 2.0 . | 2.0 . | | 2.0 | | | | 2.0 | 3.0 |
| Dracorin 100 s.e. P (Symrise) | Glyceryl Stearate, PEG-100 Stearate | | | | | | | | | | | 8.0 |
| Edeta BD® (BASF) | Dinatrium-EDTA | 0.1 | 0.1 | 0.1 | | 0.1 | 0.1 | 0.1 | | | | |
| Emulgin B2® (Cognis) | Ceteareth-20 | | 1.0 | | | | | | | 0.7 | | |
| Emulsiphos (Symrise) | Cetylphosphate, .Hydrogented Palm glycerides | | | 1.5 | | | 1.5 | | | | | |
| Ethanol (96 %) | Ethyl alcohol | | | | 13.0 | 5.0 | | | | | | |
| Euxyl K 100® (Schülke & Mayr) | Methylchlorisothiazoli-none, Methyisothiazolinone | | | | 0.1 | | | | | | | |
| Extrapon Aloe Vera (Symrise) | Aqua, Aloe Barbadensis, Propylene Glycol, Alcohol | | | | 1.0 | | | | | | | |
| Extrapon Kamille (Symrise) | Glycerin, Water (Aqua), Chamomilla Recutita (Matricaria) Flower Extract | | | | 1.0 | | | | | | | |
| Extrapon Hamamelis (Symrise) | Propylene Glycol, Hamamelis Virginiana (Witch Hazel) Water, Water (Aqua), Alcohol, Hamamelis Virginiana (Witch Hazel) Bark/Leaf/Twig Extract | | | | 1.0 | | | | | | | |
| Glycerin 99 % | Glycerin | 4.0 | 3.0 | | 4.5 | | 3.0 | 4.0 | | | | |
| Hydrolite-5 (Symrise) | 1,2-Pentanediol | | | | | 4,0 | | 5,0 | | | 3,0 | |
| Isodragol (Symrise) | Triisononanoin | | | | | | | | | | 7.0 | |
| Isopropylmyristat (Symrise) | Isopropyl Myristate | | | | | | | | | | | 8.0 |
| Keltrol T® (Calgon) | Xanthan Gum | | | 0.2 | 0.2 | 0.3 | | | | | | |
| Lanette E® (Cognis) | Natriumcetearylsulfat | | | 0.7 | | | | | | | | |
| Lanette O® (Cognis) | Cetyl- und Stearylalkohol | | 1.1 | | | | | | | 2.5 | | |
| Lanette 16® (Cognis) | Cetylalkohol | | | 1.2 | | | 0.5 | | | | | 2.0 |
| Lanette 18 (Care Chemicals) | Stearyl Alcohol | | | | | | | | | | 4.5 | |
| Lara Care A-200 (Rahn) | Galactoarabinan | | | | | | | 0.2 | | | | |
| Mg Ascorbylphosphate | Magnesium Ascorbylphosphate | | | | | | | | | | 3.0 | |
| Monomuls 90-O 18® (Cognis) | Glyceryloleate | 1.0 | | | | | | | | | | |
| Myritol 318® (Cognis) | Capryl-/Caprinsäuretriglyceride | 6.0 | 5.0 | | | | | | | | | |
| NaOH 10 % wässr. Lösung | Sodium hydroxide | | | 2.8 | | 2.2 | 2.9 | 0.6 | | | | 0.2 |
| Natrosol 250 HHR (Aqualon) | Hydroxymethyl cellulose | | 0.3 | | | | | | | | | |
| Neo-Dragocid Pulver (Symrise) | Methylparaben, Sorbic Acid, Dehydroacetic Acid, Propylparaben | | | | | | | | | | | 0.8 |
| Neo Heliopan® AP (Symrise), 15 % als Natriumsalz | Dinatrium-Phenyldibenzimidazoltetrasulfonat | 10.0 | | 22.0 | | | | | | | | |
| Neo Heliopan® AP (Symrise), 10 % wässr. Lösung neu-tralisiert mit NaOH) | Dinatrium-Phenyldibenzimidazoltetrasulfonat | | | | 22.0 | | | | | | | |
| Neo Heliopan® AV (Symrise) | Ethylhexylmethoxycinnamat | 4.0 | | | | 5.0 | 6.0 | 2.0 | | | | |
| Neo Heliopan® BB (Symrise) | Benzophenone-3 | | 1.0 | | | | | | | | | |
| Neo Heliopan® 303 (Symrise) | Octocrylene | | 7.0 | | | | | | | | | |
| Neo Heliopan® 357 (Symrise) | Butyl Methoxydibenuzoylmethane | | | 2.0 | | 1.5 | 1.5 | 1.5 | 0.5 | 0.5 | | |
| Neo Heliopan® E 1000 (Symrise) | Isoamyl-p-methoxycinnamate | 4.0 | | | | 5.0 | | 6.0 | | 2.0 | | |
| Neo Heliopan® HMS (Symrise) | Homosalate | | | 5.0 | | | | | | | | |
| Neo Heliopan® Hydro (15% wässr. Lösung neutralisiert mit NaOH) (Symrise) | Phenylbenzimidazol sulfonic acid | | | | 33.3 | 10.0 | 13.3 | | 3.3 | | | |
| Neo Heliopan® MA (Symrise) | Menthylanthranilate | | 3.0 | | | | | | | | | |
| Neo Heliopan® MBC (Symrise) | 4-Methylbenzylidencampher | 2.0 | | | | 2.0 | 4.0 | 3.0 | | | | |
| Neo Heliopan® OS (Symrise) | Ethylhexylsalicylate | 3.0 | | | | | | | | | | |
| Neutralöl (Symrise) | Capryl-/Caprinsäuretriglyceride | | | 5.0 | | | 2.0 | | | | | |
| Octyltriazon | Ethylhexyltriazone | 1.0 | | | | | | | | | | |
| Paraffinöl 5 Grad E (Parafluid) | Paraffinum Liquidum | | | | | | | | | | | 4.0 |
| Parfümöl | Parfum (Fragrance) | 0.3 | 0.3 | 0.3 | | 0.3 | 0.4 | 0.2 | 0.5 | 0.4 | 0.3 | 0.3 |
| PCL liquid (Symrise) | Cetearyl Ethylhexanoate, Isopropylmyristate | | | | | | | | | | 3.0 | |
| Pemulen TR 2 (Novion) | Acrylates/C10-30 Alkyl Acrylate Crosspolymer | | | | | | | 0.2 | | | | |
| Permulgin 2550® (Koster Keunen) | Bees Wax | 1.0 | | | | | | | | | | |
| Phenoxyethanol (Symrise) | Phenoxyethanol | 0.7 | | 0.7 | | 0.7 | 0.7 | 0.7 | | | | |
| Polymer JR 400 | Polyquaternium-10 | | | | | | | | 0.4 | | | |
| 1,2-Propylenglycol | Propylene Glycol | | | | | | | | | | | 5.0 |
| Softigen 767 | PEG-6 Caprylic/Capric Glycerides | | | | | | | | 2.5 | | | |
| Solubilizer (Symrise) | PEG 40 Hydrogenated Castor Oil, Trideceth-9, Propyleneglycol, Water | | | | | | | | 3.0 | | | |
| Symdiol 68 (Symrise) | 1,2 Hexanediol, Caprylylglycol | | | 0,5 | | | | | | | | 1,0 |
| Tegosoft TN® (Goldschmidt) | C12-C15 Alkylbenzoate | 6.0 | | | | 4.0 | 2.0 | | | | | |
| Texapon N 70 (Cognis) | Sodium Laureth Sulfate | | | | | | 0.1 | 0.5 | | | | |
| Texapon NSO BZ (Cognis) | Sodum Laureth Sulfate | | | | | | | | 27.0 | | | |
| Titandioxid mikrofein | Titan dioxide | | 5.0 | | | | | | | | | |
| Unimer U-151 (Induchem) | PVP/Hexadecene Copolymer | | | | | 0.5 | | | | | | |
| Veegum ultra® (Vanderbilt) | Magnesium Aluminium sulfate | | 1.0 | | | | | | | | | |
| Witch Hazel Distillate (Symrise) | Hamamelis Virginiana (Witch Hazel) | | | | | | | | 1.0 | | | |
| Zinkoxid neutral (Symrise) | Zinc oxide | 7.0 | | | | | | | | | | |
| Wasser, dest. | Aqua (Water) | ad 100 | ad 100 | ad 100 | ad 100 | ad 100 | ad 100 | ad 100 | ad 100 | ad 100 | ad 100 | ad 100 |

### Beispiel 7: Formulierungsbeispiele für Hautbräuner

In nachfolgender Tabelle 4 bedeuten:
- Formulierung 1:: Hautbräunende "Wasser in Öl"-Emulsion mit UVA/B-Breitbandschutz
- Formulierung 2:: Intensiv-hautbräunende "Öl in Wasser"-Emulsion mit UVA/B-Breitbandschutz
- Formulierung 3:: Hautbräunende "Wasser in Öl"-Emulsion
- Formulierung 4:: Hautbräunende "Öl in Wasser"-Emulsion mit UVA/B-Breitbandschutz
- Formulierung 5:: Hautbräunende "Öl in Wasser"-Creme
- Formulierung 6:: Hautbräunender Aerosol-Schaum mit UVB/UVA-Schutz
- Formulierung 7:: Selbstbräunungscreme O/W
- Formulierung 8:: Shampoo mit haut- und haarbräunenden Eigenschaften
- Formulierung 9:: Haut- und haarbräunender Conditioner mit UVB/UVA -Schutz
- Formulierung 10:: Hautbräunende Feuchtigkeitscreme O/W
- Formulierung 11:: Hautbräunende Gesichtscreme O/W

| **ROHMATERIAL-NAME (HERSTELLER)** | **INCI** | **Gewichts%** | | | | | | | | | | |
|---|---|---|---|---|---|---|---|---|---|---|---|---|
| | | **1** | **2** | **3** | **4** | **5** | **6** | **7** | **8** | **9** | **10** | **11** |
| AhR-Agonist | | | | | | | | | | | | |
| FICZ | | 0.1 | 0.5 | 0.0 5 | 0.5 | 1.0 | 0.3 | 0.2 | 0.2 | 0.3 | 2.0 | 5.0 |
| Coffein | Caffeine | 0.5 | | 0.2 | | | 0.2 | 0.1 | | | | |
| Naringin | 4',5,7-Trihydroxyfiavon-7-O-neohesperidoside | 0.1 | | | | | | | | 0.1 | | |
| N-Acetyl-Tyrosin | | | 0.5 | | | | | | | | | 1.0 |
| Dihydroxyaceton | Dihydroxyacetone | | | | | | | 5.0 | | | | |

| Weitere Inhaltsstoffe | | | | | | | | | | | | |
|---|---|---|---|---|---|---|---|---|---|---|---|---|
| Abil 100® (Goldschmidt) | Dimethicone | | 1.0 | | | 2.0 | | 0.5 | | | | 0.3 |
| Alugel 34 TH (Baerlocher) | Aluminium Stearate | | | 1.0 | | | | | | | | |
| Arlacel 165® (ICI) | Glycerylstearat und Polyethylenglycol 100-Stearat | | 3.0 | | | | | | | | | |
| Arlypon F | Laureth-2 | | | | | | | | 2.0 | | | |
| Bentone Gel M.IO® (Rheox) | Mineral oil and Quaternium-18-hectorite and Propylencarbonate Glycerylstearate and Cetylalcohol | | 3.0 | | | | | | | | | |
| alpha-Bisabolol (Symrise) | Bisabolol | 0.1 | | 0.1 | | | 0.1 | | 0.1 | | | |
| Brij 78® | Steareth-20 | | 0,5 | | | | | | | | | |
| Carbopol 2050® (B.F. Goodrich) | Carbomer | | | | | 0.1 | 0.1 | | | | | |
| Cetiol SN® (Cognis) | Cetyl- und Stearylisononanoat | 5.0 | 4.0 | | | | | | | | | |
| Citric Acid | Citric Acid | | | | | | | | | 0.1 | 0.3 | |
| Copherol 1250® (Cognis) | Tocopherol acetate | 1.0 | | | 0.5 | | 0.5 | | | | | |
| Corapan TQ® (Symrise) | Diethylhexyl-1,6-Naphtalate | | | 3.0 | 3.0 | | | | | | | |
| Crinipan® AD (Symrise) | Climbazol | | | | | | | | 0.5 | | | |
| Crotein Q (Croda) | Hydroxypropyl trimonium Hydrolyzed | | | | | | | | | 1.0 | | |
| Cutina CBS® (Cognis) | Glycerylstearat and Cetylalkohol and Stearylalkohol and Cecylpalmitat and Cocosglyceride | | 2.0 | | | | | | | | | |
| Dehymuls PGPH® (Cognis) | Polyglycerol-2 Dipolyhydroxystearat | 3.0 | | | | | | | | | | |
| Dehyquart SP | Quaternium-52 | | | | | | | | | 0.5 | | |
| Dehyton K | Cocamidopropyl Betaine | | | | | | | | 12.0 | | | |
| Dow Corning 200 Fluid (Dow Corning) | Dimethicone | | | | | | | | | | 2.0 | |
| D-Panthenol (BASF) | Panthenol | | | | | | | | 0.4 | | | |
| Dragocid Liquid (Symrise) | Phenoxyethanol (and) Methylparaben (and) Ethylparaben (and) Butylparaben (and) Propylparaben (and) Isobutylparaben | 0.3 | 0.3 | 1.0 | 0.3 | 0.8 | 0.3 | | 0.7 | 0.5 | 0.8 | |
| Dragophos S (Symrise) | Sodium Dihydroxycetyl Phosphate | | | | | | | | | | 2.0 | |
| Dragosan W/O P (Symrise) | Sorbitan Isostearate, Hydrogenated Castor Oil, Ceresin, Beeswax (Cera Alba) | | | 7.0 | | | | | | | | |
| Dracorin CE | Glyceryl Stearate Citrate | | | | | | | 5.0 | | | | |
| Dracorin GMS (Symrise) | Glyceryl Stearate | | | | | 2.0 | 2.0 | | | | 2.0 | 3.0 |
| Dracorin 100 s.e. P (Symrise) | Glyceryl Stearate, PEG-100 Stearate | | | | | | | | | | | 8.0 |
| Dragoxat EH (Symrise) | Ethylhexyl Ethylhexanoate | | | | | 3.0 | | 3.0 | | | | |
| Edeta BD® (BASF) | Dinatrium-EDTA | 0.1 | 0.1 | | 0.1 | | 0.1 | 0.1 | | | | |
| Emulgade PL | Cetearyl Glucoside, Cetearyl Alcohol | | | | | 0,5 | | | | | | |
| Emulgin B2® (Cognis) | Ceteareth-20 | | 1.0 | | | | | | | 0.7 | | |
| Emulsiphos (Symrise) | Cetylphosphate, Hydrogenated Palm glycerides | | | | | 2.0 | 1.5 | | | | | |
| Ethanol (96 %) | Ethyl alcohol | | | | | | | 2.0 | | | | |
| Extrapon Aloe Vera (Symrise) | Glyerin, Water (Aqua), Aloe barbadensis Leaf Extract | | | 3.0 | | | | | | | | |
| Extrapon Grüntee (Symrise) | Glyerin, Water (Aqua), Cemallia Sinensis Leaf Extract | | | | | 0.2 | | | | | | |
| Extrapon Hamamelis (Symrise) | Propylene Glycol, Hamamelis Virginiana (Witch Hazel) Water, Water (Aqua), Hamamelis Virginiana (Witch Hazel) Extract | | | 1.0 | | | | | | | | |
| Extrapon Kamille (Symrise) | Glycerin, Water (Aqua), Chamomilla Recutica (Matricaria), Flower Extract | | | | | 0.5 | | | | | | |
| Extrapon Rosmarin (Symrise) | Glycerin, Water (Aqua), Rosmarinus Officinalis (Rosemary) Leaf Extract | | | | | 0.3 | | | | | | |
| Glycerin 99 % | Glycerin | 4.0 | 3.0 | 2.0 | 4.0 | 2.0 | 3.0 | 1.5 | | | | |
| Hostacerin DGMS ® (Clariant) | Polyglceryl-2-Stearat | | | | 3.0 | | | | | | | |
| Hydrolite-5 (Symrise) | 1,2-Pentyleneglycol | | | | | | | 3.5 | | | 3.0 | |
| Isodragol (Symrise) | Triisononanoin | | | | | 2.0 | | | | | 7.0 | |
| Isopropylmyristat (Symrise) | Isopropyl Myristate | | | | | | | 4.0 | | | | 8.0 |
| Karion F (Merck) | Sorbitol | | | 2.0 | | | | | | | | |
| Keltrol T® (Calgon) | Xanthan Gum | | | | 0.2 | 0.1 | 0.3 | 0.3 | | | | |
| Lanette E® (Cognis) | Natriumcetearylsulfat | | | | 0.7 5 | | | | | | | |
| Lanette O® (Cognis) | Cetyl- und Stearylalkohol | | 1.1 | | | 3.0 | | | | 2.5 | | |
| Lanette 16® (Cognis) | Cetylalkohol | | | | 2.0 | | 0.5 | 1.0 | | | | 2.0 |
| Lanette 18 (Care Chemicals) | Stearyl Alcohol | | | | | | | | | | 4.5 | |
| Mg Ascorbylphosphate | Magnesium Ascorbylphosphate | | | | | | | | | | 3.0 | |
| Magnesium Sulfat Hepathydrat (Merck) | Magnesium Sulfate | | | 0.7 | | | | | | | | |
| Monomuls 90-O 18® (Cognis) | Glyceryloleate | 1.0 | | | | | | | | | | |
| Myritol 318® (Cognis) | Capryl-/Caprinsäuretriglyceride | 6.0 | 5.0 | | | 0.2 5 | | | | | | |
| NaOH 10% wässr. Lösung | Sodium hydroxide | | | | 0.2 | | 2.9 | | | | | 0.2 |
| Natrosol 250 HHR (Aqualon) | Hydroxymethyl cellulose | | 0.3 | | | | | | | | | |
| Neo-Dragocid Pulver (Symrise) | Methylparaben, Sorbic Acid, Dehydroacetic Acid, Propylparaben | | | | | | | | | | | 0.8 |
| Neo Heliopan® AP (Symrise), 15 % als Natriumsalz | Dinatrium-Phenyldibenzimidazoltetrasulfonat | 10. 0 | | | 6.7 | | | | | | | |
| Neo Heliopan® AV (Symrise) | Ethylhexylmethoxycinnamat | 4.0 | | | | | 6.0 | | | | | |
| Neo Heliopan® BB (Symrise) | Benzophenone-3 | | 1.0 | | | | | | | | | |
| Neo Heliopan® 303 (Symrise) | Octocrylene | | 7.0 | | | | | | | | | |
| Neo Heliopan® 357 (Symrise) | Butyl Methoxydibenuzoylmethane | | | | 0.6 | | 1.5 | | 0.5 | 0.5 | | |
| Neo Heliopan® E 1000 (Symrise) | Isoamyl-p-methoxycinnamate | 4.0 | | | | | | | | 2.0 | | |
| Neo Heliopan® HMS (Symrise) | Homosalate | | | | 9.5 | | | | | | | |
| Neo Heliopan® Hydro (15 % wässr. Lösung neutralisiert mit NaOH) (Symrise) | Phenylbenzimidazol sulfonic acid | | | | 6.7 | | 13.3 . | | 3.3 | | | |
| Neo Heliopan® MA (Symrise) | Menthylanthranilate | | 3.0 | | | | | | | | | |
| Neo Heliopan® MBC (Symrise) | 4-Methylbenzylidencampher | 2.0 | | | | | 4.0 | | | | | |
| Neo Heliopan® OS (Symrise) | Ethylhexylsalicylate | 3.0 | | | | | | | | | | |
| Neutralöl (Symrise) | Capryl-/Caprinsäuretriglyceride | | | | 5.0 | | 2.0 | 6.0 | | | | |
| Octyltriazon | Ethylhexyltriazone | 1.0 | | | | | | | | | | |
| Paraffinöl 5 Grad E (Parafluid) | Paraffinum Liquidum | | | | | | | | | | | 4.0 |
| Parfümöl | Parfum (Fragrance) | 0.3 | 0.3 | 0.4 | 0.5 | 0.3 | 0.4 | 0.3 | 0.5 | 0.4 | 0.3 | 0.3 |
| PCL liquid (Symrise) | Cetearyl Ethylhexanoate, Isopropylmyristate | | | 12. 0 | | 5.0 | | 3.0 | | | 3.0 | |
| PCL solid (Symrise) | Stearyl Heptanoate, Stearyl Caprylate | | | | | 2.0 | | | | | | |
| Permulgin 2550® (Koster Keunen, NL) | Bee wax | 1.0 | | | | | | | | | | |
| Phenoxyethanol (Symrise) | Phenoxyethanol | 0.7 | | | 0.7 | | 0.7 | | | | | |
| Polymer JR 400 | Polyquaternium-10 | | | | | | | | 0.4 | | | |
| Prisorine 3505® (UniQema) | Isostearic acid | | | | 0.5 | | | | | | | |
| 1,2-Propylenglycol | Propylene Glycol | | | | | 5.0 | | | | | | 5.0 |
| Sepigel 305 | Polyacrylamide, C13-14 Isoparaffin, Laureth-7 | | | | | | | 1.0 | | | | |
| SF1214® (Bayer) | Cyclopentasiloxane, Dimethicone | | | | 1.0 | | | | | | | |
| Softigen 767 | PEG-6 Caprylic/Capric Glycerides | | | | | | | | 2.5 | | | |
| Solubilizer (Symrise) | PEG 40 Hydrogenated Castor Oil, Trideceth-9, Propyleneglycol, Water | | | | | | | | 3.0 | | | |
| Sun Flower Oil (H. Erhard Wagner) | Helianthus Annus (Sunflower) Seed Oil | | | 5.0 | | | | | | | | |
| Sweet Almon Oil (H. Erhard Wagner) | Prunus Dulcis | | | 5.0 | | | | | | | | |
| Symdiol 68 (Symrise) | 1,2-Hexanediol, Caprylalcohol | | | | 0.5 | | | | | | | |
| SymGlucan (Symrise) | Water, Glycerin, Beta-Glucan | | | | | 0.3 | | | | | | |
| Tegosoft TN® (Goldschmidt) | C12-C15 Alkylbenzoate | 6.0 | | | 2.0 | | 2.0 | | | | | |
| Texapon N 70 (Cognis) | Sodium Laureth Sulfate | | | | | | 0.1 | 0.5 | | | | |
| Texapon NSO BZ (Cognis) | Sodium Laureth Sulfate | | | | | | | | 27.0 | | | |
| Titandioxid mikrofein | Titan dioxide | | 5.0 | | | | | | | | | |
| Vitamin E Acetat (DSM Nutritional Products) | Tocopheryl Acetate | | | 3.0 | | | | | | | | |
| Vitamin A Palmitat in ÖL (1 Mio le/g) (DSM Nutritional Products) | Retinyl Palmitate | | | 0.2 | | | | | | | | |
| Veegum ultra® (Vanderbilt) | Magnesium Aluminium sulfate | | 1.0 | | | | | | | | | |
| Witch Hazel Distillate (Symrise) | Hamamelis Virginiana (Witch Hazel) | | | | | | | | 1.0 | | | |
| Zinkoxid neutral (Symrise) | Zinc oxide | 7.0 | | | | | | | | | | |
| Wasser, dest. | Aqua (Water) | ad 100 | ad 100 | ad 100 | ad 100 | ad 100 | ad 100 | ad 100 | ad 100 | ad 100 | ad 100 | ad 100 |

## Patentansprüche

1. Verfahren zum Beurteilen der Wirksamkeit eines AhR-Agonisten bzw. AhR-Antagonisten, umfassend die Schritte
i) Beaufschlagen einer Zelle einer ex-vivo- oder in vitro- Melanozyten- und/oder Keratinozyten-Zellkultur oder eines in vitro-Hautmodells, mit einem möglichen AhR-Agonisten bzw AhR-Antagonisten,
ii) wenn die Zelle in Schritt i) mit einem möglichen AhR-Antagonisten beaufschlagt wurde Behandeln der beaufschlagten Zelle mit UVB-Strahlung oder einem AhR-Agonisten,
iii) Bestimmen der Induktion eines AhR-induzierbaren Gens,
iv) Beaufschlagen einer unbehandelten Zelle aus Schritt i) mit einer vorgewählten Verbindung gemäß einem der Ansprüche 6 bis 9,
v) Behandeln der in Schritt iv) beaufschlagten Zelle mit UVB-Strahlung wie in Schritt ii),
vi) Bestimmen der Induktion des in Schritt iii) bestimmten AhR-induzierbaren Gens, und
vii) Vergleichen der in Schritt iii) und vi) bestimmten Geninduktion.

2. Verfahren zum Screenen einer Substanzbibliothek auf AhR-Agonisten bzw. Antagonisten, umfassend die Schritte:
1) Bereitstellen einer Probe jeder der Substanzen der Substanzbibliothek, und
2) für jede der in 1) bereitgestellten Proben Durchführen eines Verfahrens nach Anspruch 1,
3) Auswählen derjenigen Substanzen als AhR-Antagonisten, bei deren Proben die Induktion eines AhR-induzierbaren Gens um ein vorgewähltes Maß verringert wurde, bzw. Auswählen derjenigen Substanzen als AhR-Agonisten, bei deren Proben die Expression eines AhR-induzierbaren Gens um ein vorgewähltes Maß erhöht wurde.

3. Verfahren zum Herstellen einer Hautschutzzubereitung, umfassend das Mischen eines AhR-Agonisten und/oder -Antagonisten mit einem kosmetisch und/oder pharmazeutisch akzeptablen Träger, so dass die Konzentration des AhR-Agonisten bzw. -Antagonisten in der Mischung zumindest das Doppelte der zum Verringern der Induktion eines AhR-induzierten Gens erforderlichen Mindestkonzentration beträgt, wobei die Mindestkonzentration bestimmt wird mit einem Verfahren nach Anspruch 1 2oder einem Verfahren umfassend die Schritte
i) Beaufschlagen einer Zelle einer ex-vivo- oder in vitro- Melanozyten- und/oder Keratinozyten-Zellkultur oder eines in vitro-Hautmodells, mit einem möglichen AhR-Agonisten bzw AhR-Antagonisten,
ii) wenn die Zelle in Schritt i) mit einem möglichen AhR-Antagonisten beaufschlagt wurde Behandeln der beaufschlagten Zelle mit UVB-Strahlung oder einem AhR-Agonisten, und
iii) Bestimmen der Induktion eines AhR-induzierbaren Gens.

4. Verfahren zum Beurteilen der Wirksamkeit eines Hautaufhellers bzw. Hautbräuners, umfassend die Schritte
i) Beaufschlagen einer Zelle mit einem möglichen Hautaufheller bzw. Hautbräuner,
ii) Bestimmen des Maßes der Melaninbildung der in Schritt i) behandelten Zelle,
iii) Beaufschlagen einer unbehandelten Zelle aus Schritt i) mit einer vorgewählten Menge
a) einer Verbindung der Formel (X), wenn die Wirksamkeit eines Hautaufhellers beurteilt werden soll, und
b) mit Formylindolo(3,2b)carbazol, wenn die Wirksamkeit eines Hautbräuners beurteilt werden soll,
iv) Bestimmen des Maßes der Melaninbildung der in Schritt iii) behandelten Zelle,
v) Vergleichen der in Schritt ii) und iv) bestimmten Hautaufhellung.

5. Verfahren zum Herstellen einer Hautaufhellungszubereitung, umfassend das Mischen eines Hautaufhellers mit einem kosmetisch und/oder pharmazeutisch akzeptablen Träger, so dass die Konzentration des Hautaufhellers in der Mischung zumindest das Doppelte der zum Hautaufhellen erforderlichen Mindestkonzentration beträgt, wobei die Mindestkonzentration bestimmt wird mit einem Verfahren nach Anspruch 4.

6. Verbindung der Formel (II) 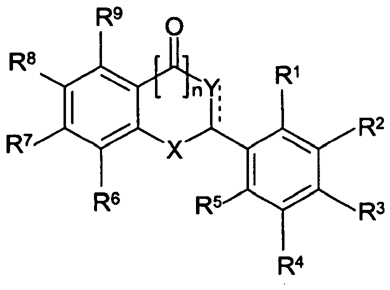 wobei
R¹ bis R⁹ unabhängig voneinander Wasserstoff, Hydroxy, C₁-C₁₂-Alkoxy, C₁-C₁₂-Alkyl, C₂-C₁₂-Alkenyl, und
n = 0 und Y = -CH- oder -C(CH₃)-, X = O und die gestrichelte Linie entsprechend entweder eine Doppelbindung oder zwei Wasserstoffe,
oder
n = 1, Y = O und X = Methylen und gestrichelte Linie zwei Wasserstoffe bedeuten.

7. Verbindung nach Anspruch 6, **dadurch gekennzeichnet, dass** R¹ bis R⁹, unabhängig voneinander, Wasserstoff, Hydroxy, C₁-C₄-Alkoxy oder C₂-C₄-Alkenyl bedeuten.

8. Verbindung nach einem der Ansprüche 6 bis 7, **dadurch gekennzeichnet, dass** zumindest 5 der Reste R¹ bis R⁹ Wasserstoff sind.

9. Verbindung nach einem der Ansprüche 6 bis 8 mit der Formel (X)

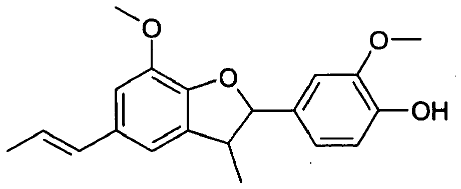

10. Zubereitung enthaltend, in einem pharmazeutisch und/oder kosmetisch akzeptablen Träger, eine Verbindung nach einem der Ansprüche 6 bis 9 oder einer nach einem der Verfahren gemäß einem der Ansprüche 1 bis 2 oder 4 ausgewählte Verbindung in einer ausreichenden Menge zum
(a) Verringern oder Verhindern einer Translokation von AhR in einen Zellkern,
(b) Verringern oder Verhindern einer UVB-induzierten oder -induzierbaren Genexpression,
(c) Verringern oder Verhindern einer durch polyzyklische aromatische Kohlenwasserstoffe induzierten oder induzierbaren Genexpression, und/oder
(d) Verringern oder Verhindern von UVB-induzierten oder -induzierbaren Hautschäden, insbesondere Hautkrebs, Hautalterung, Hautentzündungen und Sonnenbrand.

11. Zubereitung nach Anspruch 10, enthaltend die Verbindung der Formel (II) in einem Anteil von zumindest 0,0001 Gew.-%, bezogen auf die gesamte Zubereitung.

12. Zubereitung nach einem der Ansprüche 10 bis 11, ferner enthaltend einen UVA- und/oder einen UVB-Filter.

13. Zubereitung nach einem der Ansprüche 10 bis 12, **dadurch gekennzeichnet, dass** die Zubereitung eine kosmetische Zubereitung ist.

14. Arzneimittel, bestehend aus, bestehend im wesentlichen aus oder umfasend
- eine Verbindung nach einem der Ansprüche 6 bis 9 oder
- eine Zubereitung nach einem der Ansprüche 10 bis 13 oder
- eine nach einem der Verfahren gemäß einem der Ansprüche 1 bis 2 oder 4 ausgewählte Verbindung.

15. Verwendung einer Verbindung nach einem der Ansprüche 6 bis 9 zum Herstellen eines Arzneimittels.

## Claims

1. Method for assessing the effectiveness of an AhR agonist or AhR antagonist respectively, comprising the steps
i) treating a cell derived from an ex-vivo or in-vitro melanocyte and/or keratinocyte culture or from an in-vitro skin model with a potential AhR agonist or AhR antagonist,
ii) if the cell was treated with a potential AhR antagonist in step i), applicating UVB irradiation or an AhR agonist to the treated cell,
iii) determining gene induction of an AhR inducible gene,
iv) treating an untreated cell from step i) with a preselected compound according to one of claims 6 to 9,
v) applicating of UVB irradiation as in step ii) to the cell treated in step iv)
vi) determining gene induction of the AhR inducible gene analyzed in step iii) and
vii) comparing of the gene induction determined in step iii) and vi).

2. Method for screening a substance library for AhR agonists or AhR antagonists, comprising the steps
1) providing a sample of each substance in the library and
2) executing a method according to claim 1 for each sample provided in 1)
3) selecting those substances as AhR antagonists, in the samples of which the gene induction of an AhR inducible gene was reduced by a predetermined factor, and selecting those substances as AhR agonists respectively, in the samples of which the expression of an AhR inducible gene was increased by a predetermined factor.

3. Method for providing a skin protecting formulation comprising the blending of a AhR agonist and/or AhR antagonist with a cosmetically and/or pharmaceutically acceptable carrier so that the concentration of the AhR agonist or the AhR antagonist respectively in the mixture is at least double the minimal concentration necessary to reduce gene induction of the AhR inducible gene, where the minimal concentration is determined by a method according to claim 1 or 2 or a method comprising the steps
i) treating a cell derived from an ex-vivo or in-vitro melanocyte and/or keratinocyte culture or from an in-vitro skin model with a potential AhR agonist or AhR antagonist,
ii) if the cell was treated with a potential AhR antagonist in step i), applicating UVB irradiation or an AhR agonist to the treated cell,
iii) determining gene induction of an AhR inducible gene.

4. Method for assessing the effectiveness of a skin lightener or skin tanner, comprising the steps
i) treating a cell with a potential skin lightener or skin tanner respectively,
ii) determining the degree of melanin formation by the cell treated in step i),
iii) treating an untreated cell from step i) with a predetermined amount of
a) a compound with the formula (X) if the effectiveness of a skin lightener is to be determined and
b) with formylindolo(3,2b) carbazole if the effectiveness of a skin tanner is to be determined,
iv) determining the degree of melanin formation by the cell treated in step iii),
v) comparing the skin lightening determined in step ii) and iv).

5. Method for providing a skin lightening formulation comprising the blending of a skin lightener with a cosmetically and/or pharmaceutically acceptable carrier so that the concentration of the skin lightener in the mixture is at least double the minimal concentration necessary to lighten the skin, wherein the minimal concentration is determined by a method according to claim 4.

6. Compound with the formula (II), 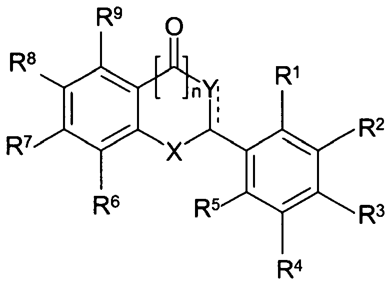 wherein R¹ to R⁹ independently mean hydrogen, hydroxy, C₁-C₁₂-alkoxy, C₁-C₁₂-alkyl, C₂-C₁₂-alkenyl and
n = 0 and Y = -CH- or -C(CH₃)-, X = O and the dotted line accordingly either a double bond or two hydrogens
or
n = 1, Y = O and X = methylene and the dotted line two hydrogens.

7. Compound according to claim 6, **characterized in that** R¹ to R⁹ independently mean hydrogen, hydroxy, C₁-C₄-alkoxy or C₂-C₄-alkenyl.

8. Compound according to one of claims 6 to 7, **characterized in that** at least 5 of the groups R¹ to R⁹ are hydrogens.

9. Compound according to one of the claims 6 to 8 with the formula (X)

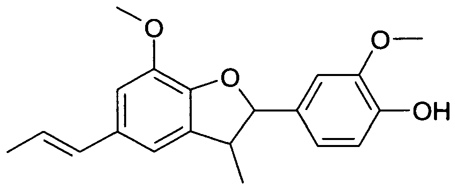

10. Formulation comprising, in a pharmaceutically and/or cosmetically acceptable carrier, a compound according to one of the claims 6 to 9 or a compound selected by one of the methods described in claims 1 to 2 or 4 in a sufficient amount to
a) reduce or inhibit translocation of AhR into a cell nucleus,
b) reduce or inhibit UVB induced or inducible gene expression,
c) reduce or inhibit a polycyclic aromatic hydrocarbon induced or inducible gene expression, and/or
d) reduce or inhibit UVB induced or inducible skin damage, particularly skin cancer, skin ageing, inflammation of skin and sunburn.

11. Formulation according to claim 10, comprising the compound with the formula (II) in a fraction of at least 0.0001 wt.% based on the total weight of the formulation.

12. Formulation according to one of the claims 10 to 11 additionally containing a UVA filter and/or a UVB filter.

13. Formulation according to one of the claims 10 to 12, **characterized in that** the formulation is a cosmetic formulation.

14. Medicament consisting of, consisting mainly of or comprising
- a compound according to one of the claims 6 to 9 or,
- a formulation according to one of the claims 10 to 13 or,
- a substance selected by a method according to one of the claims 1 to 2 or 4.

15. Use of a compound according to one of the claims 6 to 9 for producing a medicament.

## Revendications

1. Procédé servant à évaluer l'efficacité d'un agoniste d'AhR ou d'un antagoniste d'AhR, comprenant les étapes suivantes:
i) soumettre une cellule issue d'une culture cellulaire de mélanocytes et/ou de kératinocytes ex vivo ou in vitro ou issue d'un modèle de peau in vitro à l'action d'un éventuel agoniste d'AhR ou d'un antagoniste d'AhR,
ii) si la cellule a été soumise à l'étape i) à l'action d'un éventuel antagoniste d'AhR, traiter la cellule avec un rayonnement UVB ou avec un agoniste d'AhR,
iii) déterminer l'induction d'un gène pouvant être induit par AhR,
iv) soumettre une cellule non-traitée issue de l'étape i) à l'action d'un composé présélectionné selon l'une quelconque des revendications 6 à 9,
v) traiter la cellule soumise à l'étape iv) avec un rayonnement UVB comme à l'étape ii),
vi) déterminer l'induction du gène pouvant être induit par AhR déterminé à l'étape iii), et
vii) comparer l'induction de gènes déterminée à l'étape iii) et celle déterminée à l'étape vi).

2. Procédé servant à cribler une bibliothèque de substances d'agoniste d'AhR ou d'antagoniste d'AhR, comprenant les étapes consistant à :
1) préparer un échantillon de chacune des substances de la bibliothèque de substances, et
2) pour chacun des échantillons préparés à l'étape 1), mettre en oeuvre un procédé selon la revendication 1,
3) choisir précisément les substances comme antagonistes d'AhR, pour les échantillons dans lesquels l'induction d'un gène pouvant être induit par AhR est réduite d'une valeur présélectionnée, ou choisir précisément les substances comme agonistes d'AhR, pour les échantillons dans lesquels l'expression d'un gène pouvant être induit par AhR a été augmentée d'une valeur présélectionnée.

3. Procédé de production d'une préparation de protection cutanée, comprenant le mélange d'un agoniste et/ou d'un antagoniste d'AhR avec un vecteur acceptable d'un point de vue cosmétique et/ou pharmaceutique, de telle sorte que la concentration de l'agoniste ou de l'antagoniste d'AhR dans le mélange correspond au moins au double de la concentration minimale nécessaire à la réduction de l'induction d'un gène pouvant être induit par AhR, dans lequel la concentration minimale est déterminée à l'aide d'un procédé selon la revendication 1 à 2 ou à l'aide d'un procédé comportant les étapes consistant à :
i) soumettre une cellule d'une culture cellulaire de mélanocytes et/ou de kératinocytes ex vivo ou in vitro ou issue d'un modèle de peau in vitro à l'action d'un éventuel agoniste d'AhR ou d'un antagoniste d'AhR,
ii) si la cellule a été soumise à l'étape i) à l'action d'un éventuel antagoniste d'AhR, traiter la cellule avec un rayonnement UVB ou avec un agoniste d'AhR, et
iii) déterminer l'induction d'un gène pouvant être induit par AhR.

4. Procédé servant à évaluer l'efficacité d'un agent éclaircissant la peau ou d'un agent bronzant, comportant les étapes consistant à :
i) soumettre une cellule à l'action d'un éventuel agent éclaircissant la peau ou d'un agent bronzant,
ii) déterminer la proportion de formation de mélanine de la cellule traitée à l'étape i),
iii) soumettre une cellule non-traitée issue de l'étape i) à l'action d'une quantité présélectionnée
a) d'un composé de formule (X), lorsque l'efficacité d'un agent éclaircissant la peau doit être évaluée, et
b) de formylindolo(3,2b)carbazole, lorsque l'efficacité d'un agent bronzant doit être évaluée,
iv) déterminer la proportion de la formation de mélanine de la cellule traitée à l'étape iii),
v) comparer l'éclaircissement de la peau déterminé lors de l'étape ii) et celui déterminé lors de l'étape iv).

5. Procédé de production d'une préparation d'éclaircissement de la peau, comportant le mélange d'un agent éclaircissant la peau avec un vecteur acceptable d'un point de vue cosmétique et/ou pharmaceutique, de telle sorte que la concentration du produit éclaircissant la peau dans le mélange correspond au moins au double de la concentration minimale requise pour l'éclaircissement de la peau, dans lequel la concentration minimale est déterminée à l'aide d'un procédé selon la revendication 4.

6. Composé de formule (II) 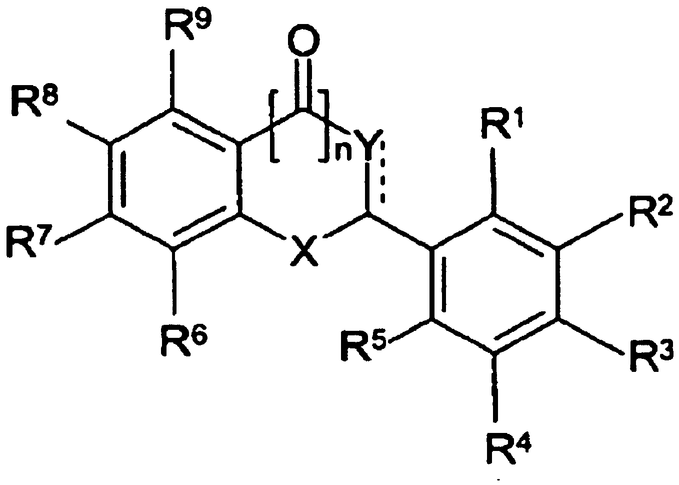 dans lequel
R¹ à R⁹ représentent indépendamment les uns des autres un atome d'hydrogène, un groupe hydroxy, un alcoxy en C₁-C₁₂, un alkyle en C₁-C₁₂, un alcényle en C₂-C₁₂, et
n = 0, Y = -CH- ou -C(CH₃) -, X = O et la ligne en pointillés représente de manière correspondante soit une double liaison soit deux hydrogènes,
ou
n = 1, Y = O et X = méthylène et la ligne en pointillés représente deux hydrogènes.

7. Composé selon la revendication 6, **caractérisé en ce que** R¹ à R⁹ représentent indépendamment les uns des autres un atome d'hydrogène, un groupe hydroxy, un alcoxy en C₁-C₄ ou un alcényle en C₂-C₄.

8. Composé selon l'une quelconque des revendications 6 à 7, **caractérisé en ce qu'**au moins 5 des radicaux R¹ à R⁹ sont de l'hydrogène.

9. Composé selon l'une quelconque des revendications 6 à 8 de la formule (X)

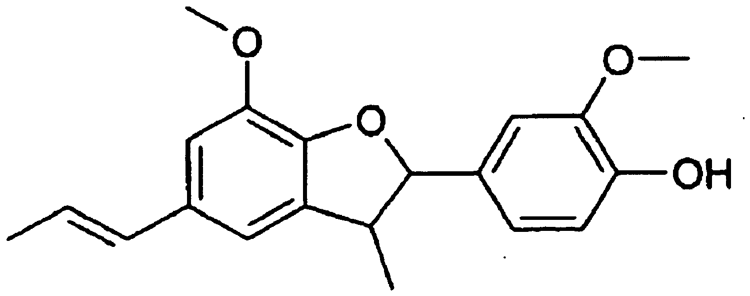

10. Préparation contenant, dans un vecteur acceptable d'un point de vue pharmaceutique et/ou cosmétique, un composé selon l'une quelconque des revendications 6 à 9 ou un composé choisi selon l'un des procédés selon l'une quelconque des revendications 1 à 2 ou 4 et présent en une quantité suffisante pour
(a) réduire ou empêcher une translocation d'AhR dans un noyau de cellule,
(b) réduire ou empêcher une expression génique induite ou pouvant être induite par les UVB,
(c) réduire ou empêcher une expression génique induite ou pouvant être induite par des hydrocarbures aromatiques polycycliques, et/ou
(d) réduire ou empêcher des lésions cutanées induites ou pouvant être induites par les UVB, en particulier le cancer de la peau, le vieillissement de la peau, les inflammations cutanées et les coups de soleil.

11. Préparation selon la revendication 10, contenant le composé de la formule (II) en une proportion d'au moins 0,0001 % en poids, par rapport au poids total de la préparation.

12. Préparation selon l'une quelconque des revendications 10 à 11, contenant en outre un filtre UVA et/ou UVB.

13. Préparation selon l'une quelconque des revendications 10 à 12, **caractérisée en ce que** la préparation est une préparation cosmétique.

14. Médicament, se composant de, se composant essentiellement de ou comprenant
- un composé selon l'une quelconque des revendications 6 à 9 ou
- une préparation selon l'une quelconque des revendications 10 à 13 ou
- un composé choisi selon l'un des procédés selon l'une quelconque des revendications 1 à 2 ou 4.

15. Utilisation d'un composé selon l'une quelconque des revendications 6 à 9 pour produire un médicament.
